(19) **Europäisches Patentamt / European Patent Office / Office européen des brevets**

(11) **EP 3 178 941 B1**

(12) # EUROPEAN PATENT SPECIFICATION

(45) Date of publication and mention of the grant of the patent:
**13.10.2021 Bulletin 2021/41**

(21) Application number: **15825462.3**

(22) Date of filing: **24.07.2015**

(51) Int Cl.:
*C12Q 1/68* (2018.01)

(86) International application number:
**PCT/CN2015/085109**

(87) International publication number:
**WO 2016/011982 (28.01.2016 Gazette 2016/04)**

(54) **METHOD FOR DETERMINING THE FRACTION OF CELL-FREE FETAL NUCLEIC ACIDS IN A PERIPHERAL BLOOD SAMPLE FROM A PREGNANT WOMAN AND USE THEREOF**

VERFAHREN ZUR BESTIMMUNG DES ANTEILS ZELLFREIER FOETALER NUKLEINSÄUREN IN EINER PROBE AUS PERIPHEREM BLUT VON EINER SCHWANGEREN FRAU UND VERWENDUNG DAVON

PROCÉDÉ DE DÉTERMINATION DE LA FRACTION D'ACIDES NUCLÉIQUES FETAUX SANS CELLULES DANS UN ÉCHANTILLON DE SANG PÉRIPHÉRIQUE D'UNE FEMME ENCEINTE ET UTILISATION DE CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.07.2014 CN 201410359726**

(43) Date of publication of application:
**14.06.2017 Bulletin 2017/24**

(73) Proprietor: **BGI Genomics Co., Limited Guangdong 518083 (CN)**

(72) Inventors:
- **JIANG, Fuman**
  **Shenzhen**
  **Guangdong 518083 (CN)**
- **YUAN, Yuying**
  **Shenzhen**
  **Guangdong 518083 (CN)**
- **WANG, Wei**
  **Shenzhen**
  **Guangdong 518083 (CN)**
- **YIN, Ye**
  **Shenzhen**
  **Guangdong 518083 (CN)**

(74) Representative: **HGF**
**1 City Walk**
**Leeds LS11 9DX (GB)**

(56) References cited:
EP-A1- 2 524 056    WO-A1-2013/132305
WO-A1-2014/068075    CN-A- 101 137 760
CN-A- 103 923 987

- S. C. Y. YU ET AL: "Size-based molecular diagnostics using plasma DNA for noninvasive prenatal testing", PROCEEDINGS NATIONAL ACADEMY OF SCIENCES PNAS, vol. 111, no. 23, 19 May 2014 (2014-05-19), pages 8583-8588, XP055297276, US ISSN: 0027-8424, DOI: 10.1073/pnas.1406103111
- H. C. FAN ET AL: "Analysis of the Size Distributions of Fetal and Maternal Cell-Free DNA by Paired-End Sequencing", CLINICAL CHEMISTRY, vol. 56, no. 8, 1 August 2010 (2010-08-01), pages 1279-1286, XP055026439, ISSN: 0009-9147, DOI: 10.1373/clinchem.2010.144188
- ZHAO, YUEHONG; ET AL.: 'Quantification of Fetal DNA by Using Methylation-Based Discrimination' JOURNAL OF XINXIANG MEDICAL UNIVERSITY vol. 29, no. 8, 31 August 2012, pages 589 - 591, XP008184613
- Bernhard Zimmermann ET AL: "Noninvasive prenatal aneuploidy testing of chromosomes 13, 18, 21, X, and Y, using targeted sequencing of polymorphic loci", Prenatal Diagnosis, vol. 32, no. 13, 30 October 2012 (2012-10-30), pages 1233-1241, XP055119823, ISSN: 0197-3851, DOI: 10.1002/pd.3993

EP 3 178 941 B1

**(Cont. next page)**

- **FUMAN JIANG ET AL: "Noninvasive Fetal Trisomy (NIFTY) test: an advanced noninvasive prenatal diagnosis methodology for fetal autosomal and sex chromosomal aneuploidies", BMC MEDICAL GENOMICS, BIOMED CENTRAL LTD, LONDON UK, vol. 5, no. 1, 1 December 2012 (2012-12-01), page 57, XP021137777, ISSN: 1755-8794, DOI: 10.1186/1755-8794-5-57**

**Description**

**FIELD OF THE INVENTION**

The present disclosure relates to determining the fraction of cell-free fetal nucleic acids in a peripheral blood sample from a pregnant woman and uses thereof.

**BACKGROUND**

Since 1977, researchers have successively found cancer-derived DNA in peripheral blood of patients with tumors; also confirmed the presence of cell-free fetal-DNA (cff-DNA) in plasma from pregnant women. Determination of the cell-free fetal DNA fraction in plasma from a pregnant woman is of great significance.

WO2013/132305 (The Chinese University of Hong Kong), WO2014/068075 (Genesupport SA) and Yu et al. (2014) Proc. Natl.Acad. Sci. 111, 8583-8588) exemplify disclosures which seek to make use of fragment size distribution of cell-free nucleic acids in blood samples of pregnant females in the context of non-invasive fetal testing for fetal genetic abnormality. However, the current methods for determining the fraction of cell-free fetal nucleic acids in such biological samples remain to be improved. An object of the present disclosure is to provide a method capable of accurately and efficiently determining the fraction of cell-free fetal nucleic acids in a maternal blood sample.

At present, there are two main directions for estimating the fraction of cell-free fetal DNA in peripheral blood: (1) taking advantage of different responses of maternal DNA fragments and cell-free fetal DNA fragments in mononuclear cells from maternal peripheral blood to methylation of specific markers; (2) selecting a plurality of representative SNPs sites based on differences among single nucleotide polymorphism (SNPs) sites. Both methods have a certain limitation: Method (1) requires a large amount of plasma, and method (2) requires probe capture and high sequencing depth, or requires obtaining of parental information. However, no report has so far been published of cell-free fetal DNA fraction estimation with whole genome sequencing at low coverage depth. Studies have shown that cell-free fetal DNA fragments in maternal blood circulation, the majority of which are shorter than 313 bp, are generally shorter than cell-free maternal DNA fragments. Under this inspiration, the inventors have invented a procedure for estimating cell-free fetal DNA fraction based on sequencing the plasma from the pregnant woman, and which method has wide applications.

**SUMMARY OF THE INVENTION**

The present invention provides a method for determining the fraction of cell-free fetal nucleic acids in a peripheral blood sample from a pregnant woman, comprising:

(i) performing sequencing on cell-free nucleic acids contained in the sample so as to obtain a sequencing result consisting of a plurality of sequencing data;
(ii) determining the number of the cell-free nucleic acids of a length falling into a predetermined range in the sample based on the sequencing result; and
(iii) determining the fraction of cell-free fetal nucleic acids in the sample based on the number of cell-free nucleic acids of a length falling into the predetermined range, wherein said predetermined range is determined prior to step (i) by the following steps:

(a) determining lengths of the cell-free nucleic acids in a plurality of control peripheral blood samples from pregnant women in each of which the fraction of cell-free fetal nucleic acids is known;
(b) setting a plurality of candidate length ranges and determining the percentage of cell-free nucleic acids obtained from each of the plurality of control samples present in each candidate length range;
(c) determining a correlation coefficient for each candidate length range based on the percentage of cell-free nucleic acids obtained from each of the plurality of control samples present in each candidate length range and the known fraction of cell-free fetal nucleic acids in each of the control samples; and
(d) determining at least one candidate length range or a combination of the candidate length ranges as the predetermined range based on the maximum correlation coefficient.

In embodiments of the present disclosure, the cell-free nucleic acids in the biological sample are sequenced by paired-end sequencing, single-end sequencing or single molecule sequencing. Thereby, lengths of the cell-free nucleic acids may be obtained easily, which is conducive to subsequent steps.

In embodiments of the present disclosure, the cell-free nucleic acids are DNA.

In embodiments of the present disclosure, determining the number of the cell-free nucleic acids of a length falling into the predetermined range in the biological sample based on the sequencing result further includes: aligning the sequencing result to a reference genome, so as to construct a dataset consisting of a plurality of uniquely mapped reads, where each read in the dataset can be maped to a single position of the reference genome; determining the length of the cell-free nucleic acid corresponding to each uniquely mapped read in the dataset; and determining the number of the cell-free nucleic acids of a length falling into the predetermined range. Thereby, the number of the cell-free nucleic acids of a length falling into the predetermined range in the biological sample can be determined easily, which gives rise to an accurate and reliable result and good reproducibility.

In embodiments of the present disclosure, determining the length of the cell-free nucleic acid corresponding to each uniquely mapped read in the dataset further includes: determining the length of each read uniquely mapped to the reference genome as the length of the cell-free nucleic acid corresponding to the read. Thereby, the length of the cell-free nucleic acid corresponding to each uniquely-aligned read in the dataset can be determined accurately.

In embodiments of the present disclosure, in the case that the cell-free nucleic acids in the biological sample are sequenced by paired-end sequencing, determining the length of the cell-free nucleic acid corresponding to each uniquely-mapped read in the dataset includes: determining the position corresponding to the reference genome of the 5'-end of the cell-free nucleic acid based on sequencing data for the 5' end of each uniquely-mapped read obtained in the paired-end sequencing; determining the position corresponding to the reference genome of the 3'-end of the cell-free nucleic acid based on sequencing data at the other end of the same uniquely-mapped read obtained in the paired-end sequencing; and determining the length of the cell-free nucleic acid based on the position of the 5'-end of the cell-free nucleic acid and the position of the 3'-end of the cell-free nucleic acid. Thereby, the length of the cell-free nucleic acid corresponding to each uniquely-mapped read in the dataset can be determined accurately.

The predetermined range is determined based on a plurality of control samples, in each of which the fraction of the cell-free fetal nucleic acids is known. Thereby, the predetermined range can be determined as an accurate and reliable result.

In embodiments of the present disclosure, the predetermined range is determined based on at least 20 control samples.

In embodiments of the present disclosure, the candidate length range is of a span of 5 bp to 20 bp.

In embodiments of the present disclosure, the candidate length range is of a span of 1 bp to 20 bp.

In embodiments of the present disclosure, the plurality of candidate length ranges is of a step size of 1 bp to 2 bp.

In embodiments of the present disclosure, determining the fraction of cell-free fetal nucleic acids in the sample based on the number of the cell-free nucleic acids of a length falling into the predetermined range further includes:

determining the percentage of cell-free nucleic acids present in the predetermined range based on the number of cell-free nucleic acids of a length falling into the predetermined range; and
determining the fraction of cell-free fetal nucleic acids in the sample, based on the percentage of the cell-free nucleic acids present in the predetermined range, according to a predetermined function, wherein the predetermined function is determined based on the plurality of control samples. Thereby, the fraction of cell-free fetal nucleic acids in the sample can be determined efficiently, which gives rise to an accurate and reliable result and good reproducibility.

In embodiments of the present disclosure, the predetermined function is obtained by following steps:

(i) determining the percentage of the cell-free nucleic acids obtained from each control sample present in the predetermined range; and
(ii) fitting the percentage of the cell-free nucleic acids obtained from each control sample present in the predetermined range with the known fraction of cell-free fetal nucleic acids to determine the predetermined function. Thereby, the predetermined function can be determined accurately and efficiently, which is conducive to subsequent steps.

In embodiments of the present disclosure, the percentage of the cell-free nucleic acids obtained from each control sample present in the predetermined range is fitted with the known fraction of cell-free fetal nucleic acids by a linear fitting.

By way of example, the predetermined range is 185 bp to 204 bp. By way of example, as illustrated by the exemplification, the predetermined function may be d=0.0334*p+1.6657, where d represents the fraction of cell-free fetal nucleic acids, and p represents the percentage of cell-free nucleic acid present in the predetermined range.

In embodiments of the present disclosure, the control sample is a peripheral blood sample obtained from a pregnant woman with a normal male fetus, in which the fraction of the cell-free fetal nucleic acids is known to be determined by chromosome Y. Thereby, the predetermined range is determined accurately.

In embodiments of the present disclosure, the fraction of cell-free fetal nucleic acids in the control sample is a cell-free fetal DNA fraction which is estimated by chromosome Y. Thereby, the predetermined range can be determined by efficiently utilizing the fraction of cell-free fetal nucleic acids of the control sample, and then the number of the cell-free nucleic acids of a length falling into the predetermined range and the cell-free fetal DNA fraction in a sample obtained from a pregnant woman under detection can be further determined.

It should be noted that, the method for determining the fraction of cell-free fetal nucleic acids in a maternal blood sample according to the present disclosure has at least the following advantages:

1) Universality: cell-free fetal (in particular female fetus) DNA fractions in all samples meeting the quality control can be estimated.
2) Accuracy of NIPT detection can be improved.
3) Operational simplicity: the cell-free fetal DNA fractions can be estimated directly merely using offline data.

In a further aspect, the present invention provides a method for determining the sex of twins. Such a method includes: performing sequencing on cell-free nucleic acids contained in a peripheral blood sample obtained from a pregnant woman with twins, so as to obtain a sequencing result consisting of a plurality of sequencing data; determining a first cell-free fetal DNA fraction based on the sequencing data by the method of the invention hereinbefore described; determining a second cell-free fetal DNA fraction based on sequencing data derived from chromosome Y in the sequencing result; and determining the sex of the twins based on the first cell-free fetal DNA fraction and the second cell-free fetal DNA fraction. The inventors have surprisingly found that the sex of twins in a pregnant woman can be accurately and efficiently determined by this method.

In embodiments of the present disclosure, the second cell-free fetal DNA fraction is determined according to the following formula:

$$fra.chry = (chry.ER\% - Female.chry.ER\%) / (Man.chry.ER\% - Female.chry.ER\%) * 100\%,$$

where *fra.chry* represents the second cell-free fetal DNA fraction, *chry.ER%* represents a percentage of the sequencing data derived from chromosome Y in the sequencing result to total sequencing data; *Female.chry.ER%* represents an average percentage of sequencing data of cell-free nucleic acids aligned to chromosome Y in a peripheral blood sample obtained from a pregnant woman predetermined to be with a normal female fetus to total sequencing data thereof; and *Man.chry.ER%* represents an average percentage of sequencing data of cell-free nucleic acids derived from chromosome Y in a peripheral blood sample obtained from a healthy man to total sequencing data thereof.

Thereby, the second cell-free fetal DNA fraction can be determined accurately.

In embodiments of the present disclosure, determining the sex of twins based on the first cell-free fetal DNA fraction and the second cell-free fetal DNA fraction further includes: (a) determining a ratio of the second cell-free fetal DNA fraction to the first cell-free fetal DNA fraction; and (b) determining the sex of the twins by comparing the ratio determined in (a) with a predetermined first threshold and a predetermined second threshold, wherein the first threshold has been determined based on a plurality of control samples obtained from pregnant women known to have female twins and the second threshold has been determined based on a plurality of control samples obtained from pregnant women known to have male twins.

Both fetuses of the twins are determined as female if the ratio of the second cell-free fetal DNA fraction to the first cell-free fetal DNA fraction is lower than the first threshold, both fetuses of the twins are determined as male if the ratio of the second cell-free fetal DNA fraction to the first cell-free fetal DNA fraction is greater than the second threshold, and the twins are determined as including a male fetus and a female fetus if the ratio of the second cell-free fetal DNA fraction to the first cell-free fetal DNA fraction is equal to the first threshold or the second threshold, or between the first threshold and the second threshold.

In embodiments of the present disclosure, the first threshold is 0.35 and the second threshold is 0.7.

In another aspect, the present invention provides a method for detecting a chromosome aneuploidy of twins. Such a method includes:

performing sequencing on cell-free nucleic acids contained in a peripheral blood sample obtained from a pregnant woman with twins so as to obtain a sequencing result consisting of a plurality of sequencing data;

determining a first cell-free fetal DNA fraction based on the sequencing data by the method of the invention hereinbefore described;

determining a third cell-free fetal DNA fraction based on sequencing data derived from a predetermined chromosome in the sequencing result; and

determining whether the twins under detection have aneuploidy with respect to the predetermined chromosome based on the first cell-free fetal DNA fraction and the third cell-free fetal DNA fraction.

Thereby, the chromosome aneuploidy of twins can be detected acurately and efficiently.

In embodiments of the present disclosure, the third cell-free fetal DNA fraction is determined according to the following formula:

*fra.chri* = 2*(*chri.ER%*/*adjust.chri.ER%*-1)*100%, where *fra.chri* represents the third cell-free fetal DNA fraction, i represents a serial number of the predetermined chromosome, and i is any integer in the range of 1 to 22; *chri.ER%* represents a percentage of the sequencing data derived from the predetermined chromosome in the sequencing result to total sequencing data; *adjust.chri.ER%* represents an average percentage of sequencing data of cell-free nucleic acids derived from the predetermined chromosome in a peripheral blood sample obtained from a pregnant woman predetermined to be with normal twins to total sequencing data thereof.

Thereby, the third cell-free fetal DNA fraction can be determined accurately.

In embodiments of the present disclosure, determining whether the twins under detection have aneuploidy with respect to the predetermined chromosome based on the first cell-free fetal DNA fraction and the third cell-free fetal DNA fraction further includes: (a) determining a ratio of the third cell-free fetal DNA fraction to the first cell-free fetal DNA fraction; and (b) determining whether the twins under detection have aneuploidy with respect to the predetermined chromosome by comparing the ratio determined in (a) with a predetermined third threshold and a predetermined fourth threshold and wherein the third threshold has been determined based on a plurality of control samples obtained from pregnant women with twins known not to have aneuploidy with respect to the predetermined chromosome, and the fourth threshold has been determined based on a plurality of control samples obtained from pregnant women with twins known to have aneuploidy with respect to the predetermined chromosome.

Both fetuses of the twins are determined to have no aneuploidy with respect to the predetermined chromosome if the ratio of the third cell-free fetal DNA fraction to the first cell-free fetal DNA fraction is lower than the third threshold, both fetuses of the twins are determined to have aneuploidy with respect to the predetermined chromosome if the ratio of the third cell-free fetal DNA fraction to the first cell-free fetal DNA fraction is greater than the fourth threshold, and one fetus of the twins is determined to have aneuploidy with respect to the predetermined chromosome, while the other fetus of the twins is determined to have no aneuploidy with respect to the predetermined chromosome if the ratio of the third cell-free fetal DNA fraction to the first cell-free fetal DNA fraction is equal to the third threshold or the fourth threshold, or between the third threshold and the fourth threshold.

In embodiments of the present disclosure, the third threshold is 0.35 and the fourth threshold is 0.7.

In embodiments of the present disclosure, the predetermined chromosome is at least one selected from chromosomes 18, 21 and 23.

In a still further aspect, the present invention provides a method for determining a chromosome aneuploidy of twins which includes:

performing sequencing on cell-free nucleic acids contained in a peripheral blood sample obtained from a pregnant woman with twins, so as to obtain a sequencing result consisting of a plurality of sequencing data and determining therefrom the fraction of cell-free fetal nucleic acids as a first cell-free fetal DNA fraction by the method of the invention as hereinbefore described;

determining a fraction $x_i$ of the number of sequencing data derived from chromosome i in the sequencing result to total sequencing data, where i represents a serial number of the chromosome, and i is any integer in the range of 1 to 22;

determining a T score of the chromosome i according to $T_i=(x_i-\mu_i)/\sigma_i$, where i represents the serial number of the chromosome and i is any integer in the range of 1 to 22, $\mu_i$ represents an average percentage of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof,

determining an L score of the chromosome i according to $L_i=\log(d(T_i,a))/\log(d(T2_i,a))$, where i represents the serial number of the chromosome and i is any integer in the range of 1 to 22, $T2_i = (x_i-\mu_i*(1+fra/2))/\sigma_i$; $d(T_i,a)$ and $d(T2_i,a)$ represent t distribution probability density function, where a represents degree of freedom and *fra* represents said first cell-free fetal DNA fraction;

plotting a four-quadrant diagram with T as vertical coordinate and L as horizontal coordinate by zoning with a first straight line where T=predetermined fifth threshold and a second straight line where L= predetermined sixth threshold, wherein both fetuses of the twins are determined to have a trisome if a sample under detection is determined to be of a T score and a L score falling into a first quadrant; one fetus of the twins is determined to have a trisome and the other fetus of the twins is determined to be normal if a sample under detection is determined to be of a T score and a L score falling into a second quadrant; both fetuses of the twins are determined to be normal if a sample under detection is determined to be of a T score and a L score falling into a third quadrant and the twins are determined to have given rise to a low fetal fraction if a sample under detection is determined to be of a T score and a L score falling into a fourth quadrant, such that the result is not adopted. The inventors have surprisingly found that determination of whether twins have aneuploidy with respect to a predetermined chromosome can be achieved accurately and efficiently by this method.

In another aspect, the present invention provides a method for detecting fetal chimera which includes:

performing sequencing on cell-free nucleic acids contained in a peripheral blood sample obtained from a pregnant woman with a fetus, optionally a male fetus, so as to obtain a sequencing result consisting of a plurality of sequencing data;

determining a first cell-free fetal DNA fraction based on the sequencing data by the method of the invention hereinbefore described,

determining a third cell-free fetal DNA fraction based on sequencing data derived from a predetermined chromosome in the sequencing result; and

determining whether the fetus under detection has fetal chimera with respect to the predetermined chromosome based on the first cell-free fetal DNA fraction and the third cell-free fetal DNA fraction.

Thereby, whether the fetus under detection has fetal chimera with respect to a specific chromosome can be analyzed accurately.

In embodiments of the present disclosure, the third cell-free fetal DNA fraction is determined by the following formula:

$fra.chri = 2*(chri.ER\%/adjust.chri.ER\%-1)*100\%$, where $fra.chri$ represents the third cell-free fetal DNA fraction, i represents a serial number of the predetermined chromosome and i is any integer in the range of 1 to 22; $chri.ER\%$ represents a percentage of the sequencing data derived from the predetermined chromosome in the sequencing result to total sequencing data; $adjust.chri.ER\%$ represents an average percentage of sequencing data of cell-free nucleic acids derived from the predetermined chromosome in a peripheral blood sample obtained from a pregnant woman predetermined to be with a normal fetus to total sequencing data thereof. Thereby, whether the fetus under detection has fetal chimera with respect to the specific chromosome can be analyzed with further improved efficiency.

In embodiments of the present disclosure, determining whether the fetus under detection has fetal chimera with respect to the predetermined chromosome based on the first cell-free fetal DNA fraction and the third cell-free fetal DNA fraction further includes: (a) determining a ratio of the third cell-free fetal DNA fraction to the first cell-free fetal DNA fraction; and (b) determining whether the fetus under detection has chimera with respect to the predetermined chromosome by comparing the ratio determined in (a) with a plurality of predetermined thresholds. Thereby, whether the fetus under detection has fetal chimera with respect to the specific chromosome can be analyzed with further improved efficiency.

In embodiments of the present disclosure, the plurality of predetermined thresholds includes at least one selected from:

a seventh threshold, determined based on a plurality of control samples with the predetermined chromosome known to be a complete monosome,

an eighth threshold, determined based on a plurality of control samples with the predetermined chromosome known to be a monosome chimera,

a ninth threshold, determined based on a plurality of control samples with the predetermined chromosome known to be normal,

a tenth threshold, determined based on a plurality of control samples with the predetermined chromosome known to be a complete trisome.

The predetermined chromosome of the fetus under detection is determined to be a complete monosome, if the ratio of the third cell-free fetal DNA fraction to the first cell-free fetal DNA fraction is lower than the seventh threshold;

the predetermined chromosome of the fetus under detection is determined to be a monosome chimera, if the ratio of the third cell-free fetal DNA fraction to the first cell-free fetal DNA fraction is not lower than the seventh threshold and not greater than the eighth threshold;

the predetermined chromosome of the fetus under detection is determined to be normal, if the ratio of the third cell-free fetal DNA fraction to the first cell-free fetal DNA fraction is greater than the eighth threshold and lower than the ninth threshold;

the predetermined chromosome of the fetus under detection is determined to be a trisome chimera, if the ratio of the third cell-free fetal DNA fraction to the first cell-free fetal DNA fraction is not lower than the ninth threshold and not greater than the tenth threshold; and

the predetermined chromosome of the fetus under detection is determined to be a complete trisome, if the ratio of the third cell-free fetal DNA fraction to the first cell-free fetal DNA fraction is greater than the tenth threshold.

In embodiments of the present disclosure, the seventh threshold is greater than -1 and lower than 0, for example -0.85;

the eighth threshold is greater than the seventh threshold and lower than 0, for example -0.3;
the ninth threshold is greater than 0 and lower than 1, for example 0.3; and
the tenth threshold is greater than the ninth threshold and lower than 1, for example 0.85.

Thereby whether the fetus under detection has fetal chimera with respect to a specific chromosome can be analyzed with further improved efficiency.

In an additional aspect, the present invention provides a method for detecting fetal chimera which includes:

performing sequencing on cell-free nucleic acids contained in a peripheral blood sample obtained from a pregnant woman with a fetus so as to obtain a sequencing result consisting of a plurality of sequencing data and determining therefrom the fraction of cell-free fetal nucleic acids as a first cell-free fetal DNA fraction by the method of the invention as hereinbefore described;

determining a fraction $x_i$ of the number of sequencing data derived from chromosome i in the sequencing result to total sequencing data, where i represents a serial number of the chromosome, and i is any integer in the range of 1 to 22;

determining a T score of the chromosome i according to $T_i(x_i-\mu_i)/\sigma_i$, where i represents the serial number of the chromosome and i is any integer in the range of 1 to 22, $\mu_i$ represents an average value of percentages of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof;

determining an L score of the chromosome i according to $L_i = \log(d(T_i,a))/\log(d(T2_i,a))$, where i represents the serial number of the chromosome and i is any integer in the range of 1 to 22, $T2_i = (x_i-\mu_i{}^*(1+fra/2))/\sigma_i$; $d(T_i,a)$ and $d(T2_i,a)$ represent t distribution probability density function, a represents degree of freedom and *fra* represents said first cell-free fetal DNA fraction;

plotting a four-quadrant diagram with T as vertical coordinate and L as horizontal coordinate by zoning with a third straight line where T=predetermined eleventh threshold and a fourth straight line where L= predetermined twelfth threshold, when the T score is not greater than 0,

wherein the fetus is determined to have a complete monosome or monosome chimera with respect to the predetermined chromosome, if a sample under detection is determined to be of a T score and a L score falling into a first quadrant;

the fetus is determined to have a monosome chimera with respect to the predetermined chromosome, if a sample under detection is determined to be of a T score and a L score falling into a second quadrant;

the fetus is determined to be normal with respect to the predetermined chromosome, if a sample under detection is determined to be of a T score and a L score falling into a third quadrant and

the fetus is determined to have given rise to a low fetal fraction if a sample under detection is determined to be of a T score and a L score falling into a fourth quadrant, such that the result is not adopted,

plotting a four-quadrant diagram with T as vertical coordinate and L as horizontal coordinate by zoning with a fifth straight line where T=predetermined thirteenth threshold and a sixth straight line where L= predetermined fourteenth threshold, when the T score is greater than 0,

wherein the fetus is determined to have a complete trisome or trisome chimera with respect to the predetermined chromosome, if a sample under detection is determined to be of a T score and a L score falling into a first quadrant;

the fetus is determined to have a trisome chimera with respect to the predetermined chromosome, if a sample under detection is determined to be of a T score and a L score falling into a second quadrant;

the fetus is determined to be normal with respect to the predetermined chromosome, if a sample under detection is determined to be of a T score and a L score falling into a third quadrant;

the fetus is determined to have given rise to a low fetal fraction if a sample under detection is determined to be of a T score and a L score falling into a fourth quadrant, such that the result is not adopted,

optionally, the eleventh threshold and the thirteenth threshold are each independently 3, and the twelfth threshold and the fourteenth threshold are each independently 1.

Thereby, situations of fetal chimera can be analyzed efficiently.

Additional aspects and advantages of embodiments of present disclosure will be given in part in the following descriptions, become apparent in part from the following descriptions, or be learned from the practice of the embodiments of the present disclosure.

## BRIEF DESCRIPTION OF THE DRAWINGS

These and other aspects and advantages of embodiments of the present disclosure will become apparent and more readily appreciated from the following descriptions made with reference to the drawings, in which:

Fig. 1 is a flow chart showing a method for determining a fraction of cell-free nucleic acids in a biological sample according to an embodiment of the present disclosure;

Fig. 2 is a flow chart showing a method for determining the number of cell-free nucleic acids in a length falling into a predetermined range according to an embodiment of the present disclosure;

Fig. 3 is a flow chart showing a method for determining the length of the cell-free nucleic acid according to an embodiment of the present disclosure;

Fig. 4 is a flow chart showing a method for determining a predetermined range according to an embodiment of the present disclosure;

Fig. 5 is a flow chart showing a method for determining a fraction of cell-free nucleic acids from a predetermined source in a biological sample according to an embodiment of the present disclosure;

Fig. 6 is a flow chart showing a method for determining a predetermined function according to an embodiment of the present disclosure;

Fig. 7 is a structural diagram of a device for determining a fraction of cell-free nucleic acids from a predetermined source in a biological sample by a method according to an embodiment of the present disclosure;

Fig. 8 is a structural diagram of a counting apparatus for use in carrying out a method according to an embodiment of the present disclosure;

Fig. 9 is a structural diagram of a first length determining unit for use in carrying out a method according to an embodiment of the present disclosure;

Fig. 10 is a structural diagram of a predetermined range determining apparatus for use in carrying out a method according to an embodiment of the present disclosure;

Fig. 11 is a structural diagram of an apparatus for determining a fraction of cell-free nucleic acids for use in carrying out a method according to an embodiment of the present disclosure;

Fig. 12 is a structural diagram of a predetermined function determining apparatus for use in carrying out a method according to an embodiment of the present disclosure;

Fig. 13 is a linear fitting diagram of correlation coefficient between the cell-free fetal DNA fraction estimated by chromosome Y and the percentage of DNA molecules present in the length range 185bp~204bp for each sample obtained from 37 pregnant women known to have a normal male fetus, according to an embodiment of the present invention; and

Figs. 14 to end are four-quadrant diagrams for T scores and L scores of 11 samples under detection according to an embodiment of the present invention.

## DETAILED DESCRIPTION

Embodiments of the present disclosure will be described in detail below, which are explanatory, illustrative, and used to generally explain the present disclosure, thus shall not be construed to limit the present disclosure.

### Method for determining a fraction of cell-free fetal nucleic acids in a peripheral blood sample from a pregnant woman

As hereinbefore indicated, the inventors have surprisingly found that the fraction of fetal nucleic acids in a peripheral blood sample obtained from a pregnant woman can be accurately and efficiently determined by the method of the present disclosure.

The fraction of cell-free fetal nucleic acids, means the fraction of the number of cell-free fetal nucleic acids to the total number of cell-free nucleic acids in the peripheral blood obtained from the pregnant woman, which sometimes also may be known as "cell-free fetal DNA fraction in the peripheral blood obtained from the pregnant woman" or "cell-free fetal DNA fraction".

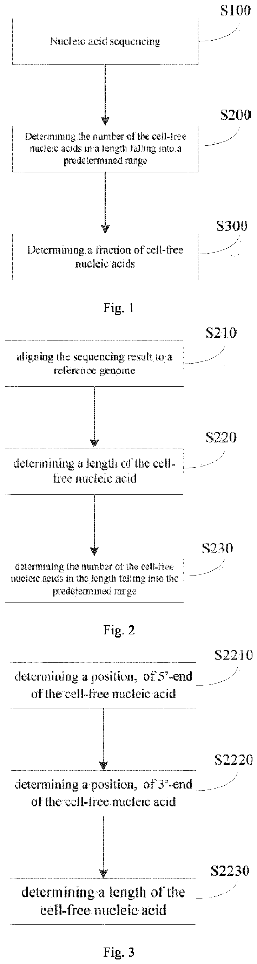

With reference to Fig. 1, the method includes the following steps.

### S100: nucleic acid sequencing

Cell-free nucleic acids in the biological sample are sequenced so as to obtain a sequencing result consisting of a plurality of sequencing data. The biological sample is a peripheral blood sample obtained from a pregnant female. The cell-free nucleic acid from the predetermined source is cell-free fetal nucleic acids in the peripheral blood sample.

It should be noted that the term "sequencing data" used herein refers to "sequence reads", which corresponds to nucleic acids subjected to sequencing. The sequencing result includes lengths of the cell-free nucleic acids.

In embodiments of the present disclosure, the cell-free nucleic acids in the biological sample are sequenced by paired-end sequencing, single-end sequencing or single molecule sequencing. Thereby, lengths of the cell-free nucleic acids may be obtained easily, which is conducive to subsequent steps.

**S200: determining the number of the cell-free nucleic acids in a length falling into a predetermined range**

The number of the cell-free nucleic acids in the length falling into the predetermined range in the biological sample is determined based on the sequencing result.

It should be noted that, term "length" used herein refers to "length of nucleic acid (read)" in base-pairs (bp).

In embodiments of the present disclosure, with reference to Fig. 2, S200 further includes steps as follows:

S210: the sequencing result is aligned to a reference genome. Specifically, the sequencing result is aligned to the reference genome, so as to construct a dataset consisting of a plurality of uniquely-mapped reads, where each read in the dataset can be mapped to a single position of the reference genome; preferably, there is no mismapped read or at most one mismapped read or at most two misaligned reads.

S220: a length of the cell-free nucleic acid is determined. Specifically, a length of the cell-free nucleic acid corresponding to each uniquely-mapped read in the dataset is determined.

S230: the number of the cell-free nucleic acids falling into the predetermined range is determined. Specifically, the number of the cell-free nucleic acids of a length falling into the predetermined range is determined.

Thereby, the number of the cell-free nucleic acids of a length falling into the predetermined range in the biological sample can be determined easily, which gives rise to an accurate and reliable result and good reproducibility.

In embodiments of the present disclosure, in S220, the length of each read uniquely mapped to the reference genome is determined as the length of the cell-free nucleic acid corresponding to the read. Thereby, the length of the cell-free nucleic acid corresponding to each uniquely-mapped read in the dataset can be determined accurately.

In embodiments of the present disclosure, in the case that the cell-free nucleic acids in the biological sample are sequenced by paired-end sequencing, with reference to Fig. 3, S220 includes the following steps.

S2210: a position, corresponding to the reference genome, of the 5'-end of the cell-free nucleic acid is determined. Specifically, the position is determined, corresponding to the reference genome, of the 5'-end of the cell-free nucleic acid based on sequencing data for the 5' end of each uniquely-mapped read obtained in the paired-end sequencing.

S2220: a position, corresponding to the reference genome, of the 3'-end of the cell-free nucleic acid is determined. Specifically, the position is determined, corresponding to the reference genome, of the 3'-end of the cell-free nucleic acid based on sequencing data at the other end of the same uniquely-mapped read obtained in the paired-end sequencing.

S2230: the length of the cell-free nucleic acid is determined. Specifically, the length of the cell-free nucleic acid is determined based on the position of the 5'-end of the cell-free nucleic acid and the position of the 3'-end of the cell-free nucleic acid.

Thereby, the length of the cell-free nucleic acid corresponding to each uniquely-aligned read in the dataset can be determined accurately.

**S300: determining the fraction of the cell-free fetal nucleic acids**

The fraction of the cell-free fetal nucleic acids in the sample based on the number of the cell-free nucleic acids of a length falling into the predetermined range is determined.

Further, the method according to the present invention further includes determining the predetermined range (S400, not shown in Figures). The predetermined range is determined based on a plurality of control samples, in each of which the fraction of the cell-free fetal nucleic acids is known. Thereby, the predetermined range can be determined as an accurate and reliable result. In embodiments of the present disclosure, the predetermined range is determined based on at least 20 control samples.

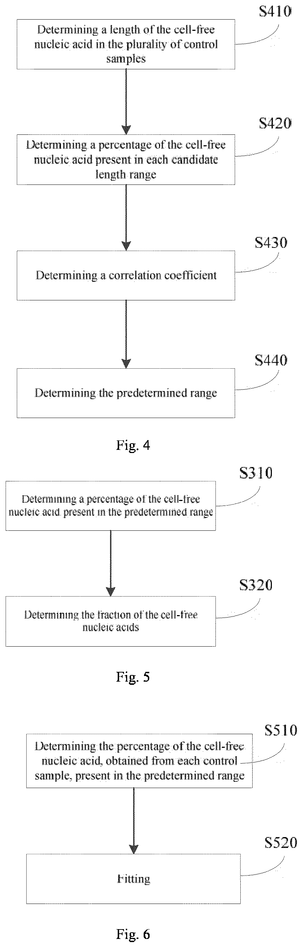

In embodiments of the present disclosure, with reference to Fig. 4, S400 includes the following steps.

S410: determining lengths of the cell-free nucleic acids in the plurality of control samples.

S420: determining the percentage of the cell-free nucleic acids present in each candidate length range. Specifically, a plurality of candidate length ranges are set, and the percentage of the cell-free nucleic acids, obtained from each of the plurality of control samples, present in each candidate length range is determined.

S430: a correlation coefficient is determined. Specifically, there is determined a correlation coefficient for each candidate length range, based on the percentage of the cell-free nucleic acids obtained from each of the plurality of control samples present in each candidate length range, and the known fraction of cell-free fetal nucleic acids in each of the control samples; and

S440: the predetermined range is determined. Specifically, a candidate length range with the largest correlation coefficient is determined as the predetermined range.

Thereby, the predetermined range can be determined accurately and efficiently.

In embodiments of the present disclosure, the candidate length range is of a span of 1 bp to 20 bp.

In embodiments of the present disclosure, the plurality of candidate length ranges is of a step size of 1 bp to 2 bp.

In embodiments of the present disclosure, with reference to Fig. 5, S300 further includes the following steps.

S310: a percentage of the cell-free nucleic acids present in the predetermined range is determined. Specifically, the percentage of the cell-free nucleic acids present in the predetermined range is determined based on the number of cell-free nucleic acids in the length falling into the predetermined range.

S320: the fraction of the cell-free fetal nucleic acids in the sample is determined. Specifically, the fraction of the cell-free fetal nucleic acids in the sample is determined based on the percentage of the cell-free nucleic acids present in the predetermined range, according to a predetermined function, in which the predetermined function is determined based on the plurality of control samples.

Thereby, the fraction of the cell-free fetal nucleic acids in the sample can be determined efficiently, which gives rise to an accurate and reliable result and good reproducibility.

In embodiments of the present disclosure, the method further includes determining the predetermined function (S500, not shown in Figures).

In some specific embodiments of the present disclosure, with reference to Fig. 6, S500 includes the following steps.

S510: the percentage of the cell-free nucleic acids obtained from each control sample present in the predetermined range is determined.

S520: the percentage of the cell-free nucleic acids obtained from each control sample present in the predetermined range is fitted with the known fraction of cell-free fetal nucleic acid to determine the predetermined function.

Thereby, the predetermined function can be determined accurately and efficiently, which is conducive to subsequent steps.

In embodiments of the present disclosure, the percentage of the cell-free nucleic acids, obtained from each control sample, present in the predetermined range is fitted with the known fraction of cell-free fetal nucleic acid by a linear fitting.

As noted above, by way of example, the predetermined range is 185 bp to 204 bp. As illustrated by the exemplification, the predetermined function may be d=0.0334*p+1.6657, where d represents the fraction of cell-free fetal nucleic acids, and p represents a percentage of cell-free nucleic acid present in the predetermined range. The fraction of cell-free fetal nucleic acids in the sample can be efficiently determined based on the predetermined function, which gives rise to an accurate and reliable result and good reproducibility.

It should be noted that expression "the percentage of the cell-free nucleic acids present in the predetermined range" refers to the percentage of the number of the cell-free nucleic acids distributed in a certain predetermined length range to total number of the cell-free nucleic acids in the biological sample.

In embodiments of the present disclosure, the control sample is a peripheral blood sample obtained from a pregnant woman with a normal male fetus, in which the fraction of the cell-free fetal nucleic acids is known to be determined by chromosome Y. Thereby, the predetermined range is determined accurately.

In embodiments of the present disclosure, the control sample is a peripheral blood sample obtained from a pregnant woman with a normal fetus. Thereby, the predetermined range is determined accurately.

In embodiments of the present disclosure, the fraction of cell-free fetal nucleic acids in the control sample is a cell-free fetal DNA fraction which is estimated by chromosome Y. Thereby, the predetermined range can be determined by efficiently utilizing the fraction of cell-free fetal nucleic acids of the control sample, and then the number of the cell-free nucleic acids in the length falling into the predetermined range and the cell-free fetal DNA fraction in a sample obtained from a pregnant woman under detection can be further determined.

In other embodiments of the present disclosure, the method may further include the following steps.

1) Whole genome sequencing (WGS): the sample under detection is subjected to whole genome sequencing using a high-throughput platform. Cell-free fetal DNAs in plasma, which are relatively short and in which only a small amount exceeds 300bp in length, are sequenced by single-end sequencing or paired-end sequencing as lengths of all cell-free fetal DNAs need to be obtained, and the entire cell-free DNA molecule is required to be sequenced if by single-ended sequencing.

2) Obtaining uniquely-mapped reads: reads of a test sample are aligned to a reference genome sequence.

3) Obtaining the length of DNA corresponding to each uniquely-mapped read based on aligning information of each uniquely-mapped read.

4) Selecting one or more ranges with high correlation: one or more ranges with high correlation is/are selected according to the length distribution of DNA molecules.

5) Obtaining a function formula: there is obtained the function formula between a percentage of DNA present in the one or more ranges with high correlation obtained in 4) and the known cell-free fetal DNA fraction.

6) Obtaining a percentage of DNA present in one or more selected ranges, i.e. the percentage of DNA present in one or more length ranges.

7) Obtaining a cell-free fetal DNA fraction of the sample under detection based on the function formula and the percentage of DNA in the sample under detection present in one or more length ranges.

Specifically, Step 4) includes the following steps:

I. Selecting a control sample, i.e. a sample in which the cell-free fetal DNA fraction is known.

II. All the samples are subjected to WGS sequencing and length information of DNA molecules represented by each uniquely-aligned read is obtained by uniquely aligning information obtained by uniquely aligning the reads to a chromosome.

III. The number of DNA molecules with a length selected up to Mbp (M represents a maximum length value, cell-free DNA molecule may have a length up to 400bp) is obtained for all the control samples.

IV. A plurality of window ranges (length ranges) are obtained through moving a window in a certain window length in accordance with a certain step size, and a percentage of DNA molecules present in each window range, i.e. a percentage of DNA molecules present in each length range, is calculated. It should be noted that the number of DNA molecules present in each window range, i.e. distributed in each length range being divided by total number of DNA molecules is defined as the percentage of DNA molecules present in each window range. For example, 1bp, 5bp, 10bp or 15bp may be taken as the window and any size selected from 1bp to the window length may be taken as the step size. Specifically, as an example, if 5bp was taken as the window and 2bp was taken as the step size, then distributions of DNA molecules in [1bp,5bp], [2bp,6bp], [4bp,8bp], [6bp,10bp] and so on may be obtained. As another example, if 5bp was taken as the window and 5bp was taken as the step size, then distributions of DNA molecules in [1bp,5bp], [6bp,10bp], [11bp, 15bp] and so on may be obtained. "Total number of DNA molecules" described hereinbefore refers to the total number of all DNA molecules with different length.

V. A window range or a combination of window ranges (i.e. a length range or a plurality of length ranges) in which the percentage of DNA molecules present is highly correlated with the known cell-free fetal DNA fraction is determined, and a function formula is established.

## Device for determining a fraction of cell-free fetal nucleic acids in a biological sample

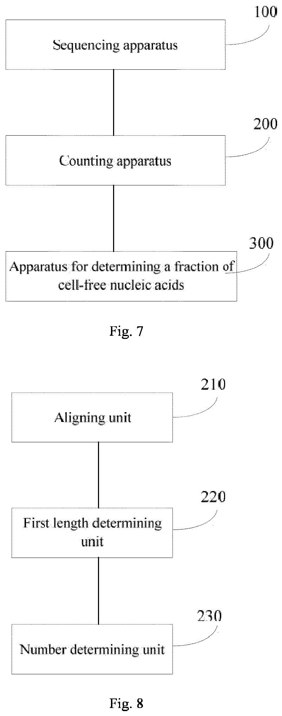

Also now disclosed, but not forming part of the claimed invention, is a device for determining a fraction of cell-free fetal nucleic acids in a peripheral blood sample from a pregnant female by a method of the invention. With reference to Fig. 7, the device includes: a sequencing apparatus 100, a counting apparatus 200 and an apparatus 300 for determining a fraction of cell-free fetal nucleic acids.

Specifically, the sequencing apparatus 100 is configured to sequence cell-free nucleic acids contained in the biological sample, so as to obtain a sequencing result consisting of a plurality of sequencing data. The counting apparatus 200 is connected to the sequencing apparatus 100 and configured to determine the number of the cell-free nucleic acids in a length falling into a predetermined range in the biological sample based on the sequencing result. The apparatus 300 for determining a fraction of cell-free fetal nucleic acids is connected to the counting apparatus 200 and configured to determine the fraction of the cell-free fetal nucleic acids based on the number of the cell-free nucleic acids of a length falling into the predetermined range.

As noted above, the cell-free nucleic acids in the biological sample are sequenced by paired-end sequencing, single-end sequencing or single molecule sequencing. Thereby, lengths of the cell-free nucleic acids may be obtained easily, which is conducive to subsequent steps.

In embodiments of the present disclosure, with reference to Fig. 8, the counting apparatus 200 further includes: an aligning unit 210, a first length determining unit 220 and a number determining unit 230. Specifically, the aligning unit 210 is configured to align the sequencing result to a reference genome so as to construct a dataset consisting of a plurality of uniquely-mapped reads, where each read in the dataset can be mapped to a single position of the reference genome. The first length determining unit 220 is connected to the aligning unit 210 and configured to determine the length of the cell-free nucleic acid corresponding to each uniquely-mapped read in the dataset. The number determining unit 230 is connected to the first length determining unit 220 and configured to determine the number of the cell-free nucleic acids of a length falling into the predetermined range. Thereby, the number of the cell-free nucleic acids of a length falling into the predetermined range in the biological sample can be determined easily, which gives rise to an accurate and reliable result and good reproducibility.

In embodiments of the present disclosure, the first length determining apparatus 220 is configured to determine the length of each read uniquely mapped to the reference genome as the length of the cell-free nucleic acid corresponding to the read. Thereby, the length of the cell-free nucleic acid corresponding to each uniquely-mapped read in the dataset can be determined accurately.

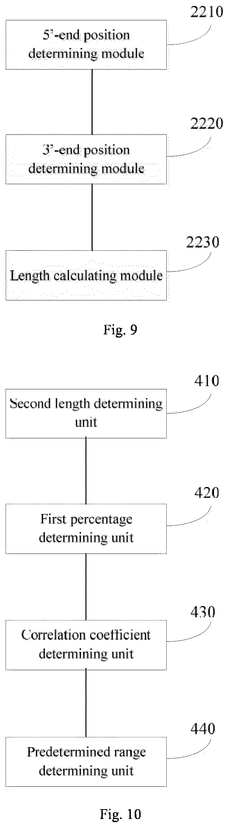

In embodiments of the present disclosure, with reference to Fig. 9, in the case that the cell-free nucleic acids in the biological sample are sequenced by paired-end sequencing, the first length determining unit 220 further includes: a 5'-end position determining module 2210, a 3'-end position determining module 2220 and a length calculating module 2230. Specifically, the 5'-end position determining module 2210 is configured to determine a position corresponding to the reference genome of the 5'-end of the cell-free nucleic acid, based on sequencing data at one end of each uniquely-mapped read obtained in the paired-end sequencing. The 3'-end position determining module 2220 is connected to the 5'-end position determining module 2210 and configured to determine a position corresponding to the reference genome of the 3'-end of the cell-free nucleic acid, based on sequencing data at the other end of same uniquely-mapped read obtained in the paired-end sequencing. The length calculating module 2230 is connected to the 3'-end position determining module 2220 and configured to determine the length of the cell-free nucleic acid based on the position of the 5'-end of the cell-free nucleic acid and the position of the 3'-end of the cell-free nucleic acid. Thereby, the length of the cell-free nucleic acid corresponding to each uniquely-mapped read in the dataset can be determined accurately.

In embodiments of the present disclosure, the device further includes a predetermined range determining apparatus 400 configured to determine the predetermined range based on a plurality of control samples, in each of which the fraction of the cell-free fetal nucleic acids is known, optionally, the predetermined range is determined based on at least 20 control samples.

In embodiments of the present disclosure, with reference to Fig. 10, the predetermined range determining apparatus 400 further includes: a second length determining unit 410, a first percentage determining unit 420, a correlation coefficient determining unit 430 and a predetermined range determining unit 440. Specifically, the second length determining unit 410 is configured to determine lengths of the cell-free nucleic acids in the plurality of control samples. The first percentage determining unit 420 is connected to the second length determining unit 410 and configured to set a plurality of candidate length ranges and determine a percentage of the cell-free nucleic acids, obtained from each of the plurality of control samples, present in each candidate length range. The correlation coefficient determining unit 430 is connected to the first percentage determining unit 420 and configured to determine a correlation coefficient between each candidate length range and the fraction of the cell-free fetal nucleic acid, based on the percentage of the cell-free nucleic acids, obtained from each of the plurality of control samples, present in each candidate length range and the fraction of the cell-free fetal nucleic acids in the control samples. The predetermined range determining unit 440 is connected to the correlation coefficient determining unit 430 and configured to select a candidate length range with the largest correlation coefficient as the predetermined range. Thereby, the predetermined range can be determined accurately and efficiently.

In embodiments of the present disclosure, the candidate length range is of a span of 1 bp to 20 bp.

In embodiments of the present disclosure, the plurality of candidate length ranges is of a step size of 1 bp to 2 bp.

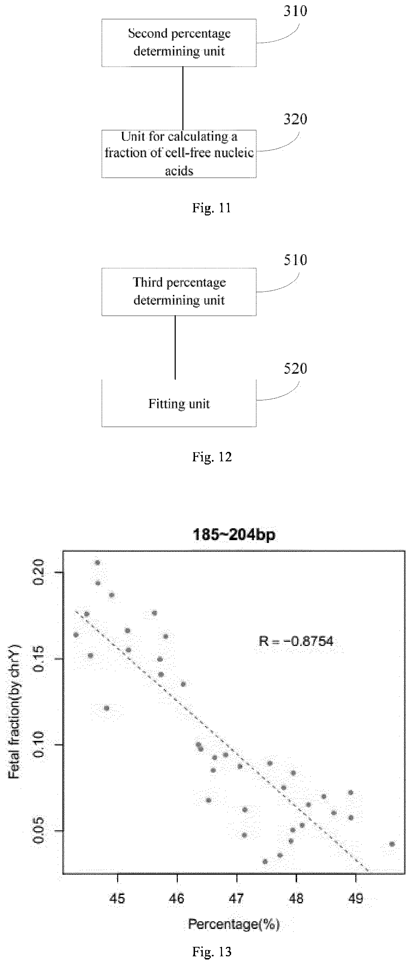

In embodiments of the present disclosure, with reference to Fig. 11, the apparatus for determining the fraction of cell-free fetal nucleic acids 300 further includes: a second percentage determining unit 310 and a unit 320 for calculating a fraction of cell-free fetal nucleic acids. Specifically, the second percentage determining unit 310 is configured to determine a percentage of the cell-free nucleic acids present in the predetermined range based on the number of cell-free nucleic acids in the length falling into the predetermined range. The unit 320 for calculating a fraction of cell-free fetal nucleic acids is connected to the second percentage determining unit 310 and configured to determine the fraction of the cell-free fetal nucleic acids in the biological sample, based on the percentage of the cell-free nucleic acids present in the predetermined range, according to a predetermined function, in which the predetermined function is determined based on the plurality of control samples. Thereby, the fraction of the cell-free fetal nucleic acids in the biological sample can be determined efficiently, which gives rise to an accurate and reliable result and good reproducibility.

In embodiments of the present disclosure, the device further includes a predetermined function determining apparatus 500. With reference to Fig. 12, the predetermined function determining apparatus 500 includes: a third percentage determining unit 510 and a fitting unit 520. Specifically, the third percentage determining unit 510 is configured to determine the percentage of the cell-free nucleic acids, obtained from each control sample, present in the predetermined range. The fitting unit 520 is connected to the third percentage determining unit 510 and configured to fit the percentage of the cell-free nucleic acids, obtained from each control sample, present in the predetermined range, with the known fraction of the cell-free fetal nucleic acid to determine the predetermined function. Thereby, the predetermined function can be determined accurately and reliably, which is conducive to subsequent steps. In embodiments of the present disclosure, the percentage of the cell-free nucleic acids, obtained from each control sample, present in the predetermined range is fitted with the known fraction of the cell-free fetal nucleic acid from the predetermined source by a linear fitting.

**Method for determining the sex of twins**

As noted hereinbefore, in another aspect, the present invention provides a method for determining the sex of twins. Such a method includes: performing sequencing on cell-free nucleic acids contained in a peripheral blood sample obtained from a pregnant woman with twins, so as to obtain a sequencing result consisting of a plurality of sequencing data; determining a first cell-free fetal DNA fraction based on the sequencing data, by the method of the_ invention hereinbefore described; determining a second cell-free fetal DNA fraction based on a sequencing data derived from chromosome Y in the sequencing result; and determining the sex of the twins based on the first cell-free fetal DNA fraction and the second cell-free fetal DNA fraction. The inventors have surprisingly found that the sex of twins in a pregnant woman can be accurately and efficiently determined by this method.

In embodiments of the present disclosure, the second cell-free fetal DNA fraction is determined according to the following formula:

$$fra.chry = (chry.ER\% - Female.chry.ER\%) / (Man.chry.ER\% - Female.chry.ER\%) * 100\%,$$

where *fra.chry* represents the second cell-free fetal DNA fraction, *chry.ER%* represents a percentage of the sequencing data derived from chromosome Y in the sequencing result to total sequencing data; *Female.chry.ER%* represents an average percentage of sequencing data of cell-free nucleic acids aligned to chromosome Y in a peripheral blood sample obtained from a pregnant woman predetermined to be with a normal female fetus to total sequencing data thereof; and *Man.chry.ER%* represents an average percentage of sequencing data of cell-free nucleic acids derived from chromosome Y in a peripheral blood sample obtained from a healthy man to total sequencing data thereof.

Thereby, the second cell-free fetal DNA fraction can be determined accurately.

In embodiments of the present disclosure, determining the sex of the twins based on the first cell-free fetal DNA fraction and the second cell-free fetal DNA fraction further includes: (a) determining a ratio of the second cell-free fetal DNA fraction to the first cell-free fetal DNA fraction; and (b) determining the sex of the twins by comparing the ratio determined in (a) with a predetermined first threshold and a predetermined second threshold.

The first threshold is determined based on a plurality of control samples obtained from pregnant women known with female twins, and the second threshold is determined based on a plurality of control samples obtained from pregnant women known with male twins.

Both fetuses of the twins are determined as female if the ratio of the second cell-free fetal DNA fraction to the first cell-free fetal DNA fraction is lower than the first threshold, both fetuses of the twins are determined as male if the ratio of the second cell-free fetal DNA fraction to the first cell-free fetal DNA fraction is greater than the second threshold, and the twins are determined as including a male fetus and a female fetus if the ratio of the second cell-free fetal DNA fraction to the first cell-free fetal DNA fraction is equal to the first threshold or the second threshold, or between the first threshold and the second threshold.

In embodiments of the present disclosure, the first threshold is 0.35 and the second threshold is 0.7.

Also now disclosed but not forming part of the claimed invention is a system for determining the sex of twins in accordance with a method of the invention. In embodiments of the present disclosure, the system includes:

a first cell-free fetal DNA fraction determining device, being the device hereinbefore for determining the fraction of cell-free fetal nucleic acids in the biological sample, and configured to sequence cell-free nucleic acids contained in a peripheral blood sample obtained from a pregnant woman with twins, so as to obtain a sequencing result consisting of a plurality of sequencing data, and configured to determine a first cell-free fetal DNA fraction based on the sequencing data;
a second cell-free fetal DNA fraction determining device, configured to determine a second cell-free fetal DNA fraction based on sequencing data derived from chromosome Y in the sequencing result; and
a sex determining device, configured to determine the sex of the twins based on the first cell-free fetal DNA fraction and the second cell-free fetal DNA fraction.

In embodiments of the present disclosure, the sex determining device further includes: a ratio determining unit, configured to determine a ratio of the second cell-free fetal DNA fraction to the first cell-free fetal DNA fraction; and a comparison unit, configured to compare the ratio determined by the ratio determining unit with a first predetermined threshold and a second predetermined threshold, so as to determine the sex of the twins. Thereby, the sex of the twins can be determined efficiently.

## Method for detecting a chromosome aneuploidy of twins

As hereinbefore indicated, in a further aspect, the present invention provides a method for detecting a chromosome aneuploidy of twins. Such a method includes:

performing sequencing on cell-free nucleic acids contained in a peripheral blood sample obtained from a pregnant woman with twins, so as to obtain a sequencing result consisting of a plurality of sequencing data;
determining a first cell-free fetal DNA fraction, based on the sequencing data, by the method of the invention hereinbefore described;
determining a third cell-free fetal DNA fraction, based on sequencing data derived from a predetermined chromosome in the sequencing result; and
determining whether the twins under detection have aneuploidy with respect to the predetermined chromosome based on the first cell-free fetal DNA fraction and the third cell-free fetal DNA fraction.

Thereby, the chromosome aneuploidy of twins can be detected acurately and efficiently.

In embodiments of the present disclosure, the third cell-free fetal DNA fraction is determined according to the following formula:

$$fra.chri = 2*(chri.ER\% / adjust.chri.ER\% - 1)*100\%,$$

where *fra.chi* represents the third cell-free fetal DNA fraction, i represents a serial number of the predetermined chromosome, and i is any integer in the range of 1 to 22; *chri.ER%* represents a percentage of the sequencing data derived from the predetermined chromosome in the sequencing result to total sequencing data; *adjust.chri.ER%* represents an average percentage of sequencing data of cell-free nucleic acids derived from the predetermined chromosome in a peripheral blood sample obtained from a pregnant woman predetermined to be with normal twins to total sequencing data thereof. Thereby, the third cell-free fetal DNA fraction can be determined accurately.

In embodiments of the present disclosure, determining whether the twins under detection have aneuploidy with respect to the predetermined chromosome based on the first cell-free fetal DNA fraction and the third cell-free fetal DNA fraction further includes: (a) determining a ratio of the third cell-free fetal DNA fraction to the first cell-free fetal DNA fraction; and (b) determining whether the twins under detection have aneuploidy with respect to the predetermined chromosome by comparing the ratio determined in (a) with a predetermined third threshold and a predetermined fourth threshold. Thereby, the chromosome aneuploidy of twins can be detected efficiently.

The third threshold is determined based on a plurality of control samples obtained from pregnant women with twins known not to have aneuploidy with respect to the predetermined chromosome, and the fourth threshold is determined based on a plurality of control samples obtained from pregnant women with twins known to have aneuploidy with respect to the predetermined chromosome.

Both fetuses of the twins are determined to have no aneuploidy with respect to the predetermined chromosome if the ratio of the third cell-free fetal DNA fraction to the first cell-free fetal DNA fraction is lower than the third threshold, both fetuses of the twins are determined to have aneuploidy with respect to the predetermined chromosome if the ratio of the third cell-free fetal DNA fraction to the first cell-free fetal DNA fraction is greater than the fourth threshold, and one fetus of the twins is determined to have aneuploidy with respect to the predetermined chromosome, while the other fetus of the twins is determined to have no aneuploidy with respect to the predetermined chromosome if the ratio of the third cell-free fetal DNA fraction to the first cell-free fetal DNA fraction is equal to the third threshold or the fourth threshold, or between the third threshold and the fourth threshold.

In embodiments of the present disclosure, the third threshold is 0.35 and the fourth threshold is 0.7.

In embodiments of the present disclosure, the predetermined chromosome is at least one selected from chromosomes 18, 21 and 23.

Also now disclosed, but not forming part of the claimed invention, is a system for determining a chromosome aneuploidy of twins in accordance with a method as above. The system includes:

a first cell-free fetal DNA fraction determining device, being the device hereinbefore for determining the fraction of cell-free nucleic acids in the biological sample, and configured to sequence cell-free nucleic acids contained in a peripheral blood sample obtained from a pregnant woman with twins, so as to obtain a sequencing result consisting of a plurality of sequencing data, and configured to determine a first cell-free fetal DNA fraction based on the sequencing data;
a third cell-free fetal DNA fraction determining device, configured to determine a third cell-free fetal DNA fraction based on a sequencing data derived from a predetermined chromosome in the sequencing result; and a first aneuploidy determining device, configured to determine whether the twins under detection have aneuploidy with respect to the predetermined chromosome based on the first cell-free fetal DNA fraction and the third cell-free fetal DNA fraction. The inventors have surprisingly found that the chromosome aneuploidy of twins can be detected accurately and efficiently by such a system according to the present disclosure.

In embodiments of the present disclosure, the first aneuploidy determining device further includes:

a ratio determining unit, configured to determine a ratio of the third cell-free fetal DNA fraction to the first cell-free fetal DNA fraction; and

a comparison unit, configured to compare the ratio determined by the ratio determining unit with a predetermined third threshold and a predetermined fourth threshold, so as to determine whether the twins under detection have aneuploidy with respect to the predetermined chromosome. Thereby, the chromosome aneuploidy of twins can be detected efficiently.

In a still further aspect, the present disclosure provides a method for determining a chromosome aneuploidy of twins which method includes:

performing sequencing on cell-free nucleic acids contained in a peripheral blood sample obtained from a pregnant woman with twins, so as to obtain a sequencing result consisting of a plurality of sequencing data and determining therefrom the fraction of cell-free fetal nuceic acids as a first cell-free DNA fraction by the method of the invention as hereinbefore described;

determining a fraction $x_i$ of the number of sequencing data derived from chromosome i in the sequencing result to total sequencing data, where i represents a serial number of the chromosome, and i is any integer in the range of 1 to 22;

determining a T score of the chromosome i according to $T_i = (x_i - \mu_i)/\sigma_i$, where i represents the serial number of the chromosome and is any integer in the range of 1 to 22, $\mu_i$ represents an average percentage of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof,

determining an L score of the chromosome i according to $L_i = \log(d(T_i, a))/\log(d(T2_i, a))$, where i represents the serial number of the chromosome and i is any integer in the range of 1 to 22, $T2_i = (x_i - \mu_i*(1+fra/2))/\sigma_i$; $d(T_i, a)$ and $d(T2_i, a)$ represent t distribution probability density function, where a represents degree of freedom and *fra* represents said first cell-free fetal DNA fraction;

plotting a four-quadrant diagram with T as vertical coordinate and L as horizontal coordinate by zoning with a first straight line where T=predetermined fifth threshold and a second straight line where L= predetermined sixth threshold, wherein both fetuses of the twins are determined to have a trisome if a sample under detection is determined to be of a T score and a L score falling into a first quadrant; one fetus of the twins is determined to have a trisome and the other fetus of the twins is determined to be normal if a sample under detection is determined to be of a T score and a L score falling into a second quadrant; both fetuses of the twins are determined to be normal if a sample under detection is determined to be of a T score and a L score falling into a third quadrant; the twins are determined to have given rise to a low fetal fraction if a sample under detection is determined to be of a T score and a L score falling into a fourth quadrant, such that the result is not adopted. The inventors have surprisingly found that the determination of whether twins under detection have aneuploidy with respect to a predetermined chromosome can be achieved accurately and efficiently by this method.

Also now disclosed, but not forming part of the claimed invention, is a system for determining a chromosome aneuploidy of twins in accordance with the invention which includes:

an $x_i$ value determining device, configured to sequence cell-free nucleic acids contained in a peripheral blood sample obtained from a pregnant woman with twins, so as to obtain a sequencing result consisting of a plurality of sequencing data, and configured to determine a fraction $x_i$ of the number of sequencing data derived from chromosome i in the sequencing result to total sequencing data, where i represents a serial number of the chromosome and i is any integer in the range of 1 to 22;

a T score determining device, configured to determine a T score of the chromosome i according to $T_i = (x_i - \mu_i)/\sigma_i$, where i represents the serial number of the chromosome and i is any integer in the range of 1 to 22, $\mu_i$ represents an average percentage of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof,

an L score determining device, configured to determine an L score of the chromosome i according to $L_i =$ log($d(T_i,a)$)/log($d(T2_i,a)$), where i represents the serial number of the chromosome and i is any integer in the range of 1 to 22, T2$_i$ = ($x_i-\mu_i$*(1+$fra$/2))/$\sigma_i$; $d(T_i,a)$ and $d(T2_i,a)$ represent t distribution probability density function, where a represents degree of freedom, and $fra$ represents a first cell-free fetal DNA fraction determined by the method of the invention hereinbefore described;

a second aneuploidy determining device, configured to plot a four-quadrant diagram with T as vertical coordinate and L as horizontal coordinate by zoning with a first straight line where T=predetermined fifth threshold and a second straight line where L= predetermined sixth threshold, wherein both fetuses of the twins are determined to have a trisome if a sample under detection is determined to be of a T score and a L score falling into a first quadrant; one fetus of the twins is determined to have a trisome and the other fetus of the twins is determined to be normal if a sample under detection is determined to be of a T score and a L score falling into a second quadrant; both fetuses of the twins are determined to be normal if a sample under detection is determined to be of a T score and a L score falling into a third quadrant; the twins are determined to have a low fetal fraction if a sample under detection is determined to be of a T score and a L score falling into a fourth quadrant, such that the result is not adopted.

It should be noted that, "reference database" described in "$\mu_i$ represents an average value of percentages of sequencing data of the chromosome i selected as a reference system in a reference database to total sequencing data thereof' refers to cell-free nucleic acids in a peripheral blood sample obtained from a pregnant woman with a normal fetus (female fetus, male fetus, single fetus or twins) or sequencing data (reads).

The "sequencing data" expressed in "sequencing data derived from chromosome Y" means reads obtained in sequencing.

In some specific embodiments of the present disclosure, terms "$x_i$", "$ER_i$" and "Chri.ER%" are changeable herein, that is, $x_i$ may be a result obtained after subjected to GC correction. Specifically, ER and GC content of each chromosome can be fitted by using known data obtained from normal samples to obtain a relation formula, i.e. $ER_i = f_i(GC_i)+\varepsilon_i$. A mean value $\overline{ER_i}$ of ER is calculated. For a sample to be analyzed, an ER value after correction is calculated according to the following formula and based on the above relation formula and ER and GC of the sample.

$$\overline{\overline{ER}}_{ij} = \overline{ER}_i + \overline{\varepsilon}_i = \overline{ER}_i + ER_{ij} - f_i(GC_{ij})$$

**Method for detecting fetal chimera**

In another aspect, the present invention provides a method for detecting fetal chimera which includes:

performing sequencing on cell-free nucleic acids contained in a peripheral blood sample obtained from a pregnant woman with a fetus, optionally a male fetus, so as to obtain a sequencing result consisting of a plurality of sequencing data;

determining a first cell-free fetal DNA fraction, based on the sequencing data, by the method of the invention hereinbefore described,

determining a third cell-free fetal DNA fraction based on sequencing data derived from a predetermined chromosome in the sequencing result; and

determining whether the fetus under detection has fetal chimera with respect to the predetermined chromosome based on the first cell-free fetal DNA fraction and the third cell-free fetal DNA fraction.

Thereby, whether the fetus under detection has fetal chimera with respect to a specific chromosome can be analyzed accurately.

In embodiments of the present disclosure, the third cell-free fetal DNA fraction is determined by the following formula:

$fra.chri$ = 2*($chri.ER$%/$adjust.chri.ER$%-1)*100%, where $fra.chri$ represents the third cell-free fetal DNA fraction, i represents a serial number of the predetermined chromosome and i is any integer in the range of 1 to 22; $chri.ER$% represents a percentage of the sequencing data derived from the predetermined chromosome in the sequencing result to total sequencing data; $adjust,chri.ER$% represents an average percentage of sequencing data of cell-free nucleic acids derived from the predetermined chromosome in a peripheral blood sample obtained from a pregnant woman predetermined to be with a normal fetus to total sequencing data thereof. Thereby, whether the fetus under detection has fetal chimera with respect to the specific chromosome can be analyzed with further improved efficiency.

In embodiments of the present disclosure, determining whether the fetus under detection has fetal chimera with respect to the predetermined chromosome based on the first cell-free fetal DNA fraction and the third cell-free fetal DNA fraction further includes: (a) determining a ratio of the third cell-free fetal DNA fraction to the first cell-free fetal DNA fraction; and (b) determining whether the fetus under detection has chimera with respect to the predetermined chromosome by comparing the ratio determined in (a) with a plurality of predetermined thresholds. Thereby, whether the fetus under detection has fetal chimera with respect to a specific chromosome can be analyzed with further improved efficiency.

In embodiments of the present disclosure, the plurality of predetermined thresholds includes at least one selected from:

a seventh threshold, determined based on a plurality of control samples with the predetermined chromosome known to be a complete monosome,
an eighth threshold, determined based on a plurality of control samples with the predetermined chromosome known to be a monosome chimera,
a ninth threshold, determined based on a plurality of control samples with the predetermined chromosome known to be normal,
a tenth threshold, determined based on a plurality of control samples with the predetermined chromosome known to be a complete trisome.

The predetermined chromosome of the fetus under detection is determined to be a complete monosome, if the ratio of the third cell-free fetal DNA fraction to the first cell-free fetal DNA fraction is lower than the seventh threshold;

the predetermined chromosome of the fetus under detection is determined to be a monosome chimera, if the ratio of the third cell-free fetal DNA fraction to the first cell-free fetal DNA fraction is not lower than the seventh threshold and not greater than the eighth threshold;
the predetermined chromosome of the fetus under detection is determined to be normal, if the ratio of the third cell-free fetal DNA fraction to the first cell-free fetal DNA fraction is greater than the eighth threshold and lower than the ninth threshold;
the predetermined chromosome of the fetus under detection is determined to be a trisome chimera, if the ratio of the third cell-free fetal DNA fraction to the first cell-free fetal DNA fraction is not lower than the ninth threshold and not greater than the tenth threshold; and
the predetermined chromosome of the fetus under detection is determined to be a complete trisome, if the ratio of the third cell-free fetal DNA fraction to the first cell-free fetal DNA fraction is greater than the tenth threshold.

In embodiments of the present disclosure, the seventh threshold is greater than -1 and lower than 0, for example -0.85;

the eighth threshold is greater than the seventh threshold and lower than 0, for example -0.3;
the ninth threshold is greater than 0 and lower than 1, for example 0.3;
the tenth threshold is greater than the ninth threshold and lower than 1, for example 0.85.

Thereby whether the fetus under detection has fetal chimera with respect to a specific chromosome can be analyzed with further improved efficiency.

Additionally now disclosed, but not forming part of the claimed invention, is a system for detecting fetal chimera as above. The system includes:

a first cell-free fetal DNA fraction determining device, being the device hereinbefore for determining the fraction of cell-free nucleic acids in the biological sample, and configured to sequence cell-free nucleic acids contained in a peripheral blood sample obtained from a pregnant woman with fetus, so as to obtain a sequencing result consisting of a plurality of sequencing data, and configured to determine a first cell-free fetal DNA fraction based on the sequencing data;
a third cell-free fetal DNA fraction determining device, configured to determine a third cell-free fetal DNA fraction based on sequencing data derived from a predetermined chromosome in the sequencing result; and
a chimera determining device, configured to determine whether the fetus under detection has fetal chimera with respect to the predetermined chromosome based on the first cell-free fetal DNA fraction and the third cell-free fetal DNA fraction.

Such a system for detecting fetal chimera may further include:

a ratio determining unit, configured to determine a ratio of the third cell-free fetal DNA fraction to the first cell-free fetal DNA fraction; and

a comparison unit, configured to compare the ratio determined by the ratio determining unit with a plurality of predetermined thresholds, so as to determine whether the fetus under detection has chimera with respect to the predetermined chromosome.

In an additional aspect, the present invention provides a method for detecting fetal chimera which includes:

performing sequencing on cell-free nucleic acids contained in a peripheral blood sample obtained from a pregnant woman with a fetus, so as to obtain a sequencing result consisting of a plurality of sequencing data and determining therefrom the fraction of cell-free fetal nucleic acids as a first cell-free fetal DNA fraction by the method of the invention hereinbefore described;

determining a fraction $x_i$ of the number of sequencing data derived from chromosome i in the sequencing result to total sequencing data, where i represents a serial number of the chromosome, and i is any integer in the range of 1 to 22;

determining a T score of the chromosome i according to $T_i = (x_i - \mu_i)/\sigma_i$, where i represents the serial number of the chromosome and i is any integer in the range of 1 to 22, $\mu_i$ represents an average value of percentages of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof;

determining an L score of the chromosome i according to $L_i = log(d(T_i, a))/log(d(T2_i, a))$, where i represents the serial number of the chromosome and i is any integer in the range of 1 to 22, $T2_i = (x_i - \mu_i*(1+fra/2))/\sigma_i$; $d(T_i, a)$ and $d(T2_i, a)$ represent t distribution probability density function, a represents degree of freedom and *fra* represents said first cell-free fetal DNA fraction;

plotting a four-quadrant diagram with T as vertical coordinate and L as horizontal coordinate by zoning with a third straight line where T=predetermined eleventh threshold and a fourth straight line where L= predetermined twelfth threshold, when the T score is not greater than 0,

wherein the fetus is determined to have a complete monosome or monosome chimera with respect to the predetermined chromosome, if a sample under detection is determined to be of a T score and a L score falling into a first quadrant;

the fetus is determined to have a monosome chimera with respect to the predetermined chromosome, if a sample under detection is determined to be of a T score and a L score falling into a second quadrant;

the fetus is determined to be normal with respect to the predetermined chromosome, if a sample under detection is determined to be of a T score and a L score falling into a third quadrant; and

the fetus is determined to have given rise to a low fetal fraction if a sample under detection is determined to be of a T score and a L score falling into a fourth quadrant, such that the result is not adopted,

plotting a four-quadrant diagram with T as vertical coordinate and L as horizontal coordinate by zoning with a fifth straight line where T=predetermined thirteenth threshold and a sixth straight line where L= predetermined fourteenth threshold, when the T score is greater than 0,

wherein the fetus is determined to have a complete trisome or trisome chimera with respect to the predetermined chromosome, if a sample under detection is determined to be of a T score and a L score falling into a first quadrant;

the fetus is determined to have a trisome chimera with respect to the predetermined chromosome, if a sample under detection is determined to be of a T score and a L score falling into a second quadrant;

the fetus is determined to be normal with respect to the predetermined chromosome, if a sample under detection is determined to be of a T score and a L score falling into a third quadrant; and

the fetus is determined to have given rise to a low fetal fraction if a sample under detection is determined to be of a T score and a L score falling into a fourth quadrant, such that the result is not adopted.

Optionally, the eleventh threshold and the thirteenth threshold are each independently 3, and the twelfth threshold and the fourteenth threshold are each independently 1.

Thereby, situations of fetal chimera can be analyzed efficiently.

It should be noted that, the expression "normal male fetus/female fetus/ fetus" means that the chromosome of the fetus is normal, for example, "normal male fetus" refers to a male fetus with normal chromosomes. Moreover, "normal male fetus /female fetus/ fetus" may refer to a single fetus or twins, for example, "normal male fetus" may be normal single fetus or normal twins; "normal fetus" neither limits the sex of the fetus nor limits the fetus being single fetus or twins.

Embodiments of the present disclosure will be described in detail below with reference to examples, but it should be appreciated to those skilled in the art that the following examples are merely used to illustrate the present disclosure, thus shall not be construed to limit the scope of the present disclosure. An example in which the specific condition is not specified will be carried out under normal condition or condition recommended by the manufacturer.

**EP 3 178 941 B1**

Reagents or instruments with no specified manufacturer are conventional products available from the market.

**Example 1**

Cell-free fetal DNA fractions in plasma samples obtained from 11 pregnant women under detection were estimated in accordance with the invention as follows.

1) Sample collection and treatment

2 ml peripheral blood samples, extracted during pregnancy from each of 11 pregnant women under detection and 37 pregnant women known to have male fetuses, were subjected to plasma separation, so as to obtain a plasma sample of each pregnant woman.

2) Library construction

The library was constructed according to plasma library construction requirements of Complete Genomics Inc.

3) Sequencing

The sequencing process was carried out strictly following the standard operating procedure of Complete Genomics Inc.

4) Data analyzing

Length distribution of DNA fragments was analyzed with reads obtained in the paired-end sequencing; the process was as shown in Fig. 1. Specific steps were as follows:

a) The lengths of DNA fragments in the peripheral blood sample obtained from each of 11 pregnant women under detection and 37 pregnant women known to have male fetuses were calculated. Specifically, 19 bp at one end of each uniquely-mapped read and 12 bp at the other end of each uniquely-mapped read were choosen to determine start and end positions corresponding to the reference genome of the uniquely-mapped read, and thus the length of DNA fragment was obtained based on the start and end positions corresponding to the reference genome of the uniquely-aligned read.

b) For the peripheral blood sample obtained from each of 37 pregnant women known to have a male fetus, a plurality of window ranges (length range) were obtained through moving a window in a certain window length in accordance with a certain step size; the percentage of DNA molecules present in each window range, i.e. the percentage of DNA molecules present in each length range, was calculated. It should be noted that the number of DNA molecules present in each window range, i.e. distributed in each length range, being divided by total number of DNA molecules was defined as the percentage of DNA molecules present in each window range. For example, 1bp, 5bp, 10bp or 15bp may be taken as the window and any size selected from 1bp to the window length may be taken as the step size. Specifically, as an example, if 5bp was taken as the window and 2bp was taken as the step size, then distributions of DNA molecules in [1bp,5bp], [2bp,6bp], [4bp,8bp], [6bp,10bp] and so on may be obtained. As another example, if 5bp was taken as the window and 5bp was taken as the step size, then distributions of DNA molecules in [1bp,5bp], [6bp,10bp], [11bp, 15bp] and so on may be obtained.

c) One or more ranges was/were selected, in which the percentages of DNA molecules present for peripheral blood samples obtained from 37 pregnant women known to have male fetuses were strongly correlated to the known fetal fraction. For a certain length range, cell-free fetal DNA fractions in peripheral blood samples obtained from 37 pregnant women known to have male fetuses were estimated by chromosome Y (for the specific estimation method reference may be made to Jiang et al. Noninvasive Fetal Trisomy (NIFTY) test: an advanced noninvasive prenatal diagnosis methodology for fetal autosomal and sex chromosomal aneuploidies. BMC Med Genomics. 2012 Dec 1;5:57 and a correlation coefficient between each cell-free fetal DNA fraction and percentage of DNA molecules in a length falling into M was calculated. Further, a length range M with a maximum absolute value of correlation coefficient was selected, for example, M=185~204bp, the correlation coefficient R=-0.87, as shown in Fig. 13; or M=121~150bp, the correlation coefficient R=-0.6199.

d) Functional relationship between the percentage of DNA molecules in a peripheral blood sample obtained from a pregnant woman, present in the length range M and cell-free fetal DNA fraction (record as $d$) was determined. Specifically, with respect to the above 37 samples with known cell-free fetal DNA fractions $d$ (samples obtained from 37 pregnant women known to have male fetuses), a linear fitting graph was ploted using the percentages $p_i$(i=1,

2, ..., 37) of DNA molecules present in the length range 185~204bp and cell-free fetal DNA fractions $d_i$(i=1,2,..,37), such that a relationship formula therebetween was obtained: d-a*p+b, where d=0.0334*p+1.6657.

e) Length distribution of DNA fragments and the percentage of DNA molecules present in M were obtained for each sample obtained from the 11 pregnant women under detection. The percentage *p* of DNA fragments present in the length range 185bp~204bp was obtained for each sample obtained from the pregnant women under detection, and results are shown in Table 1 below.

f) Cell-free fetal DNA fractions of samples under detection were estimated. Cell-free fetal DNA fraction $d_j$ of each sample under detection was calculated directly according to the percentage $p_j$ (j was a label of the sample under detection) of DNA fragments present in the length range 185bp~204bp obtained as above for each sample under detection and the relationship formula d=a*p+b.

g) Estimated results of cell-free fetal DNA fractions of the samples under detection are shown in Table 1; for samples obtained from 37 pregnant women known to have male fetuses, the cell-free fetal DNA fractions estimated by chrY were basically in conformity with those obtained by the method of the invention.

Table 1

| Percentage of DNA molecules present in 185bp~204bp (%) | Cell-free fetal DNA fraction (estimated by chrY) | Cell-free fetal DNA fraction (estimated by the percentage of DNA molecules present in 185bp~204bp, i.e. the method of the present invention) | Sample |
|---|---|---|---|
| 45.33932509 | 0.14872 | 0.145567224 | test sample |
| 45.85055949 | 0.12618 | 0.129861913 | test sample |
| 47.80808312 | 0.06752 | 0.06972606 | test sample |
| 46.78175523 | 0.10438 | 0.101255234 | test sample |
| 47.76095629 | 0.07148 | 0.071173814 | test sample |
| 45.18367884 | 0.15494 | 0.150348735 | test sample |
| 45.61282777 | 0.13413 | 0.13716512 | test sample |
| 48.50026322 | 0.04923 | 0.04846203 | test sample |
| 47.30076126 | 0.08555 | 0.085311176 | test sample |
| 47.12684798 | 0.09149 | 0.090653857 | test sample |
| 47.59287602 | 0.07892 | 0.076337302 | test sample |

**Example 2:**

Determination of the sex of twins and the detection of the chromosome aneuploidy of twins were carried out using peripheral blood samples obtained from 11 pregnant women with twins as described in Example 1, according to the methods hereinbefore disclosed for determing sex of twins and determining a chromosome aneuploidy of twins and based on the results of cell-free fetal DNA fractions obtained in Example 1.

1. Determination of sex of fetus of pregnant woman under detection

Sex of each fetus of 11 pregnant women under detection was determined based on the results of cell-free fetal DNA fractions determined in Example 1 and according to the following steps:

a) fra.chry was calculated by chromosome Y;
b) fra.size was estimated by differences within a predetermined region between Mother and Fetal;
c) Determination standard:

I. If a value of fra.chry/fra.size is lower than 0.35, both fetuses of the twins are female;
II. If error! No reference source is found. If the value of fra.chry/fra.size is not lower than 0.35 and not greater than 0.7, the twins include a male fetus and a female fetus;
III. If error! No reference source is found. If the value of fra.chry/fra.size is greater than 0.7, both fetuses of the twins are male.

Results are shown in the table below:
Detection results of sex of fetus of 11 pregnant women under detection

| Sample No. | fra.chry | fra.size | fra.chry/fra.size | Detection result | Karyotype results |
|---|---|---|---|---|---|
| S1 | 0.131431 | 0.118 | 1.113822 | [Male,Male] | [Male,Male] |
| S2 | -0.00014 | 0.126652 | -0.00114 | [Female,Female] | [Female,Female] |
| S3 | -0.00019 | 0.125 | -0.00151 | [Female,Female] | [Female,Female] |
| S4 | 0.141033 | 0.150125 | 0.939435 | [Male,Male] | [Male,Male] |
| S5 | 0.071202 | 0.088074 | 0.808433 | [Male,Male] | [Male,Male] |
| S6 | 0.06227 | 0.133 | 0.468195 | [Female,Male] | [Female,Male] |
| S7 | -0.00044 | 0.132 | -0.0033 | [Female,Female] | [Female,Female] |
| S8 | -0.00396 | 0.074 | -0.05353 | [Female,Female] | [Female,Female] |
| S9 | 0.185233 | 0.172 | 1.076936 | [Male,Male] | [Male,Male] |
| S10 | 0.072432 | 0.086 | 0.842233 | [Male,Male] | [Male,Male] |
| S11 | 0.000339 | 0.092792 | 0.003653 | [Female,Female] | [Female,Female] |

1. Determination of chromosome aneuploidy of twins by fetal fractions

Chromosome aneuploidy of twins of 11 pregnant women under detection was determined by fetal fractions, based on the results of cell-free fetal DNA fractions determined in Example 1 and according to the following steps:

a) fra.chry was calculated by chromosome i (i=13,18,21);
b) fra.size was estimated by differences within a predetermined region between Mother and Fetal;
c) Determination standard:

I. If error! No reference source is found. If the value of fra.chry/fra.size is lower than 0.35, chromosome i in both fetuses of the twins are normal;
II. If error! No reference source is found. If the value of fra.chry/fra.size is not lower than 0.35 and not greater than 0.7, chromosome i in one fetus of the twins is trisome and in the other fetus of the twins is normal;
III. If error! No reference source is found. If the value of fra.chry/fra.size is greater than 0.7, chromosome i in both fetuses of the twins are trisome.

Results are shown in the table below:
Determination results of chromosome aneuploidy of twins of 11 pregnant women under detection by fetal fractions

| Sample No. | fra.chr13 | fra.chr18 | fra.chr21 | fra.size | fra.chr13/fra.size | fra.chr18/fra.size | fra.chr21/fra.size | Detection result | Karyotype results |
|---|---|---|---|---|---|---|---|---|---|
| S1 | 0.113979 | 0.017835 | -0.01762 | 0.118 | 0.965921 | 0.151144 | -0.14933 | [T13,T13] | [T13,T13] |
| S2 | -0.00586 | 0.004833 | 0.009971 | 0.126652 | -0.0463 | 0.03816 | 0.078728 | [normal,normal] | [normal,normal] |
| S3 | 0.001131 | 0.003996 | 0.121607 | 0.125 | 0.009048 | 0.031968 | 0.972856 | [T21,T21] | [T21,T21] |
| S4 | -0.01175 | -0.01269 | 0.004841 | 0.150125 | -0.07829 | -0.08455 | 0.032246 | [normal,normal] | [normal,normal] |
| S5 | 2.20E-05 | -0.01032 | 0.011321 | 0.088074 | 0.00025 | -0.11721 | 0.128539 | [normal,normal] | [normal,normal] |
| S6 | 0.040963 | -0.00491 | -0.00723 | 0.133 | 0.307992 | -0.03693 | -0.05438 | [T13,normal] | [T13,normal] |
| S7 | 0.005557 | -0.01147 | 0.0754 | 0.132 | 0.042098 | -0.08688 | 0.571212 | [T21,normal] | [T21,normal] |
| S8 | 0.014679 | 0.031445 | -0.01884 | 0.074 | 0.198365 | 0.424932 | -0.25462 | [T18,normal] | [T18,normal] |
| S9 | 0.010402 | 0.082992 | 0.007482 | 0.172 | 0.060477 | 0.482512 | 0.0435 | [T18,normal] | [T18,normal] |
| S10 | 0.001905 | 0.074566 | -0.00051 | 0.086 | 0.022151 | 0.867052 | -0.00592 | [T18,T18] | [T18,T18] |
| S11 | 0.014591 | -0.00375 | -0.01728 | 0.092792 | 0.157244 | -0.04041 | -0.1862 | [normal,normal] | [normal,normal] |

2. Determination of chromosome aneuploidy of twins of pregnant woman under detection by T score & L score

Chromosome aneuploidy of twins of 11 pregnant women under detection was determined by T score & L score, based on the results of cell-free fetal DNA fractions determined in Example 1 and according to the following steps:

a) Whole genome sequencing (WGS): the sample under detection was subjected to whole genome sequencing using the Illumina high-throughput platform.

b) Position information of effective reads was obtained: reads of a test sample were uniquely mapped to the reference genome sequence hg19 to obtain position information, corresponding to the reference genome, of the uniquely-mapped reads.

c) Percentage of effective reads was obtained: Percentage of effective reads in each chromosome to total effective reads obtained in b) was obtained.

d) GC Correction:

ER and GC contents of each chromosome were fitted by using known data obtained from normal samples to obtain a relation formula: $ER_i = f_i(GC_i) + \varepsilon_i$, and a mean value $\overline{ER_i}$ of ER was calculated. For a sample to be analyzed, an ER value after correction was calculated according to the above relation formula and ER and GC of the sample.

$$\overline{\overline{ER}}_{ij} = \overline{ER}_i + \varepsilon_i = \overline{ER}_i + ER_{ij} - f_i(GC_{ij})$$

e) T score was calculated: $T_i = (x_i - \mu_i)/\sigma_i$

where $i$ : a serial number of chromosome (i=13,18,21);

$x_i$ : percentage of effective reads of the chromosome i in an analytic sample;

$\mu_i$ : an average percentage of effective reads of the chromosome i selected as a reference system in a reference database;

$\sigma_i$ : a standard deviation of percentages of effective reads of chromosome i selected as the reference system in the reference database;

f) L score was calculated:

L score of chromosome i was determined according to the formula:

$L_i = \log(d(T_i,a))/\log(d(T2_i,a))$, where i represents the serial number of the chromosome, $T2_i = (x_i - \mu_i^*(1 + fra/2))/\sigma_i$; $d(T_i,a)$ and $d(T2_i,a)$ represent t distribution probability density function, a represents degree of freedom, $fra$ represents the first cell-free fetal DNA fraction determined by the method in Example 1.

g) A four-quadrant diagram was ploted with T as vertical coordinate and L as horizontal coordinate by zoning with a first straight line where T=3 and a second straight line where L=0.8 (a sample with fetal frection <5% was determined to not meet the quality control), details were described as follows:

I. Both fetuses of the twins were determined to have a trisome if a sample under detection was determined to be of a T score and a L score (T>3, L>0.8) falling into a first quadrant;

II. One fetus of the twins was determined to have a trisome and the other fetus of the twins was determined to be normal if a sample under detection was determined to be of a T score and a L score (T>3,L≤0.8) falling into a second quadrant;

III. Both fetuses of the twins were determined to be normal if a sample under detection was determined to be of a T score and a L score (T≤3,L≤0.8) falling into a third quadrant;

IV. The twins were determined to have given rise to a low fetal fraction if a sample under detection was determined to be of a T score and a L score (T≤3,L>0.8) falling into a fourth quadrant; such a sample did not meet the quality control.

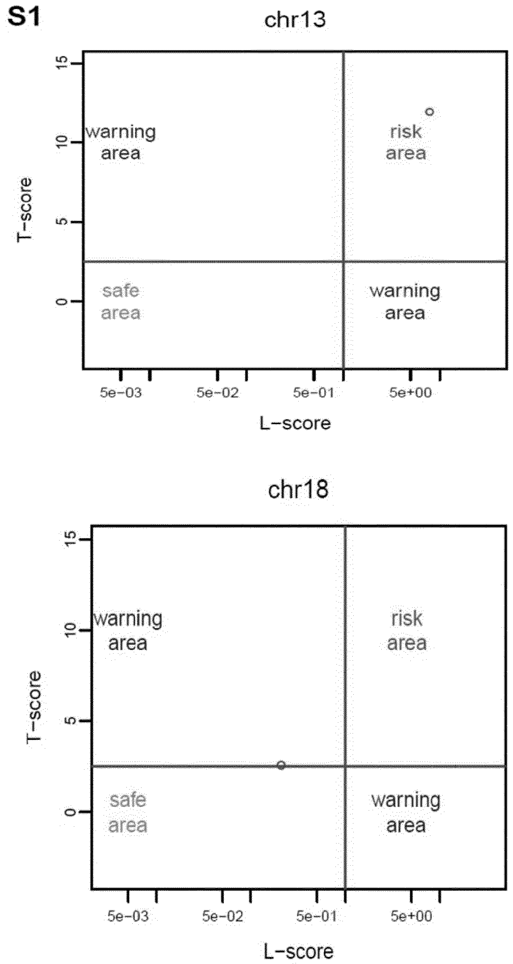

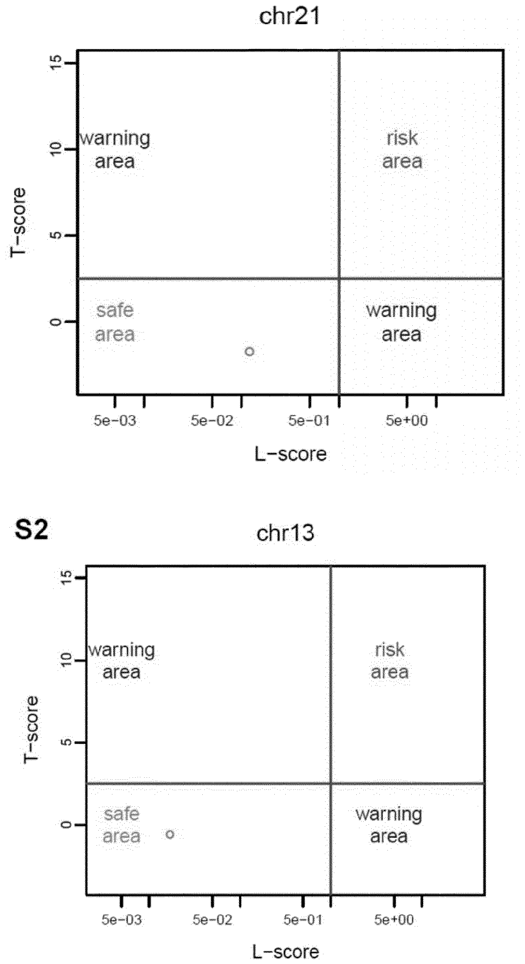

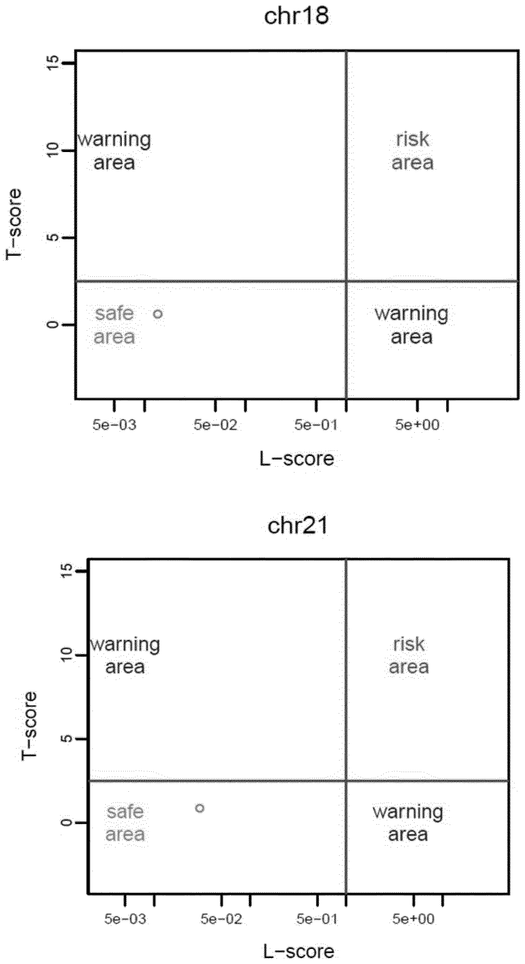

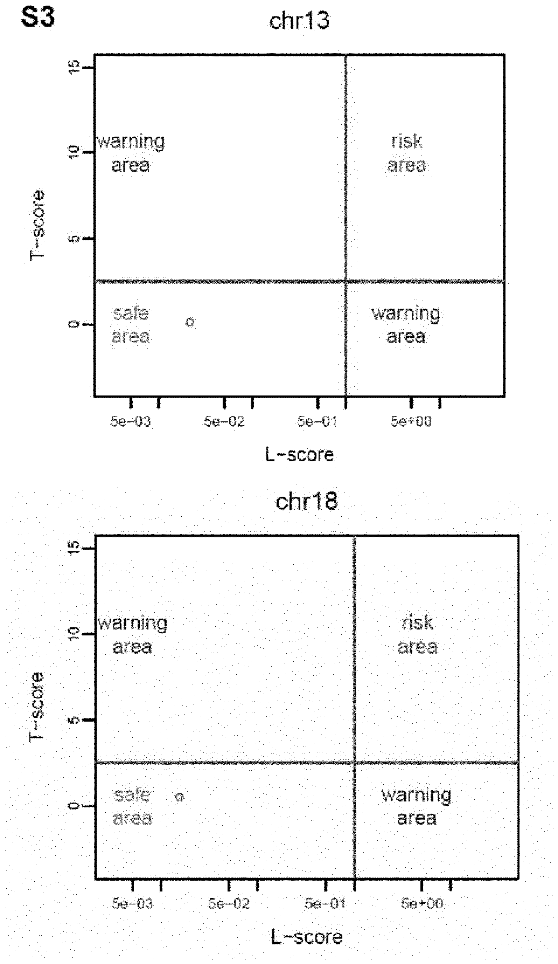

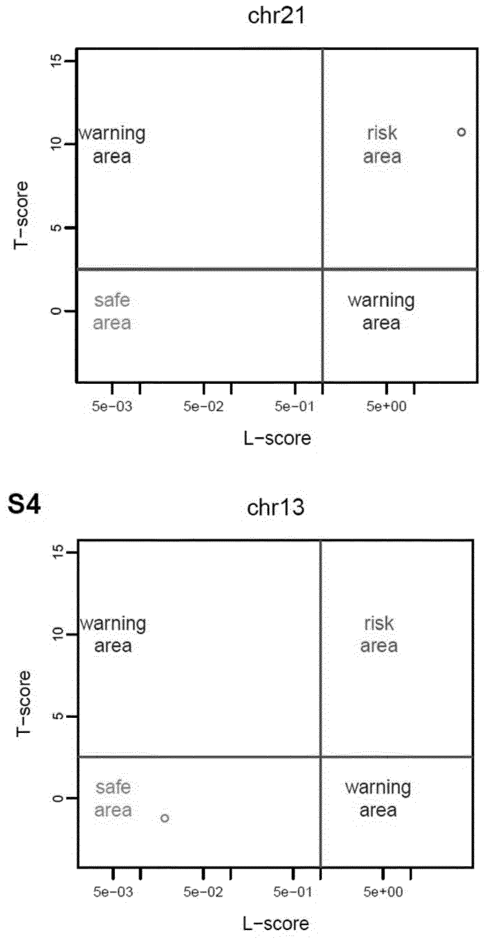

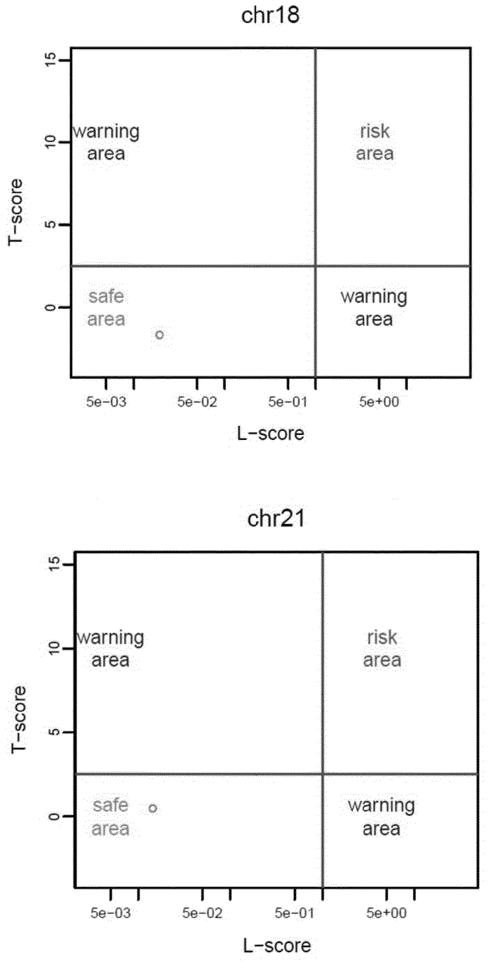

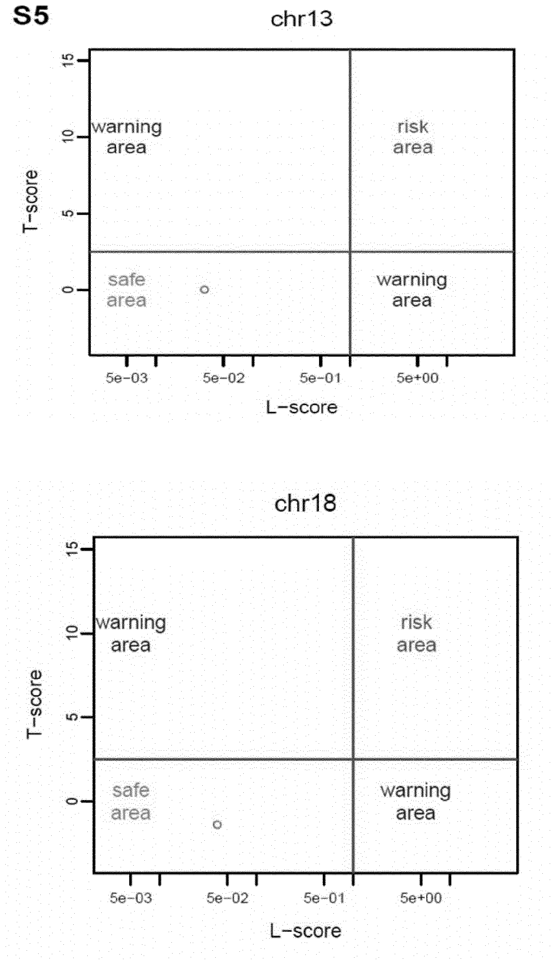

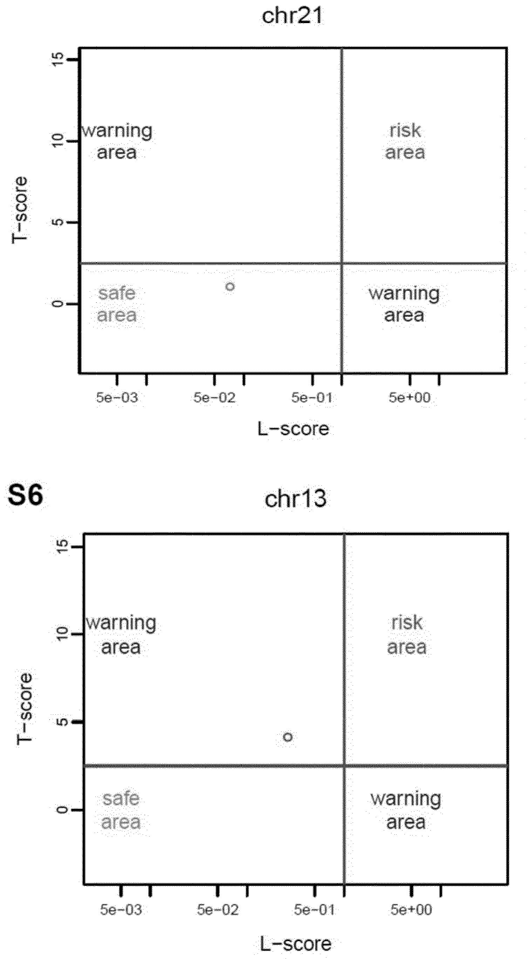

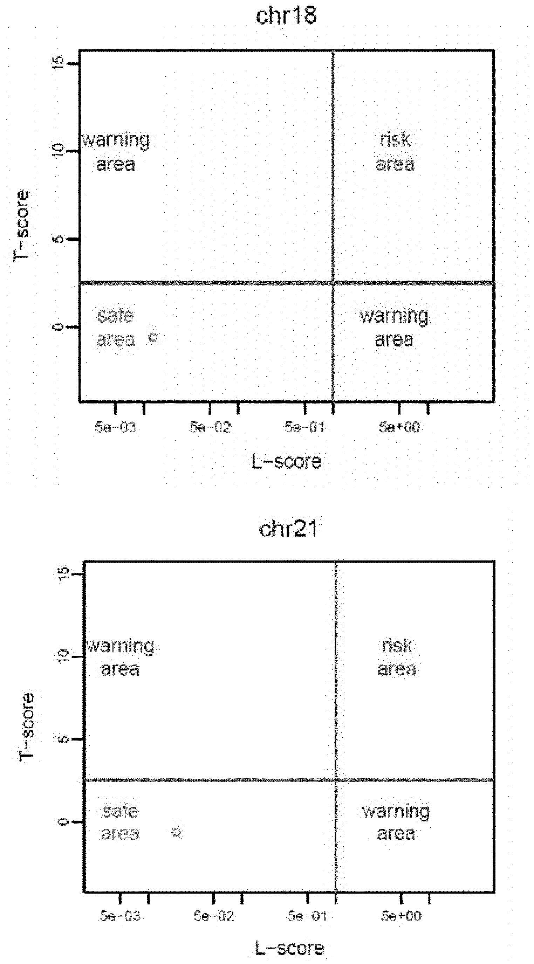

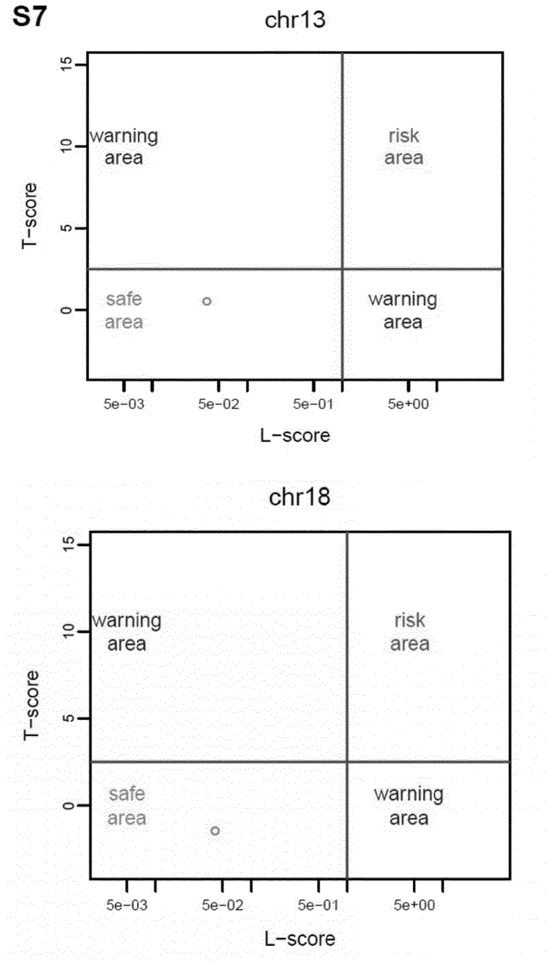

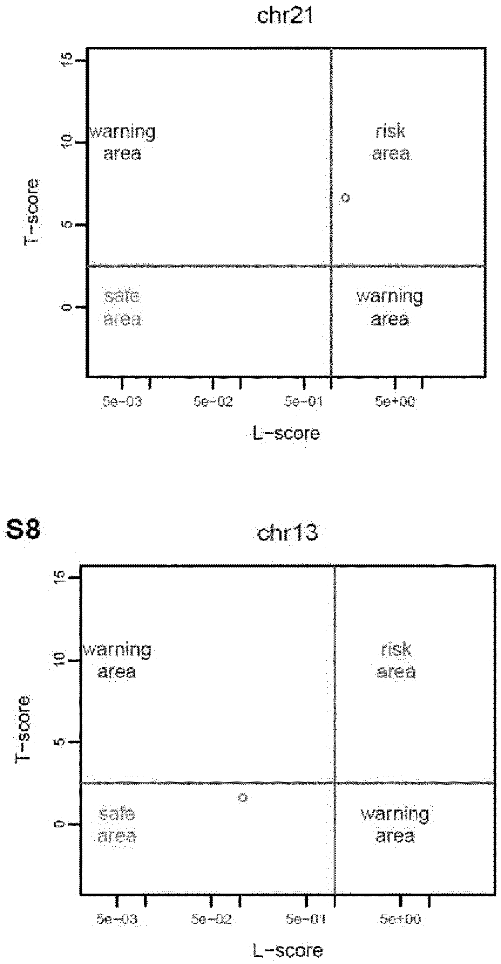

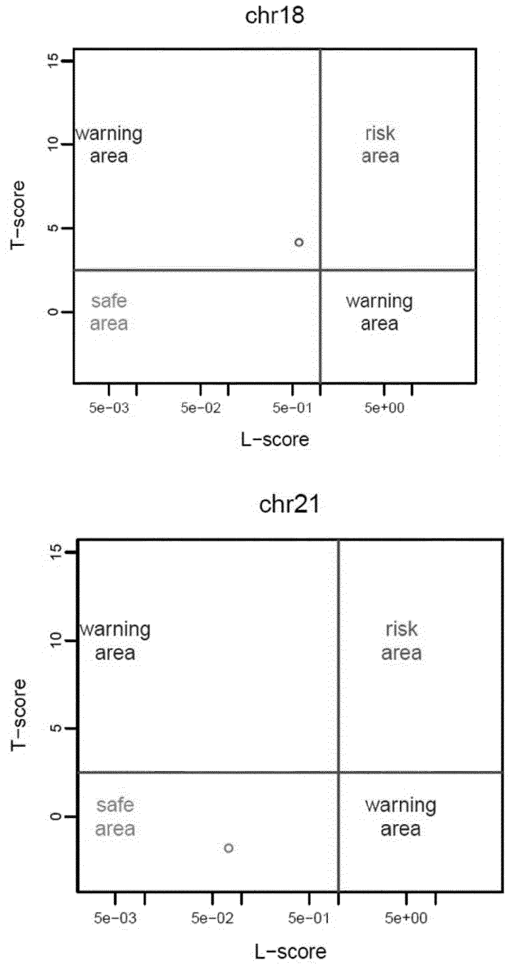

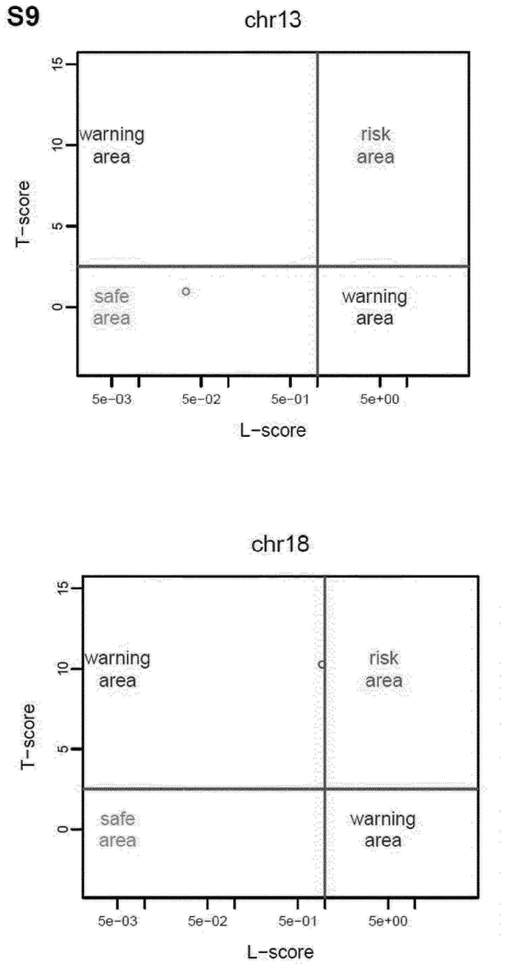

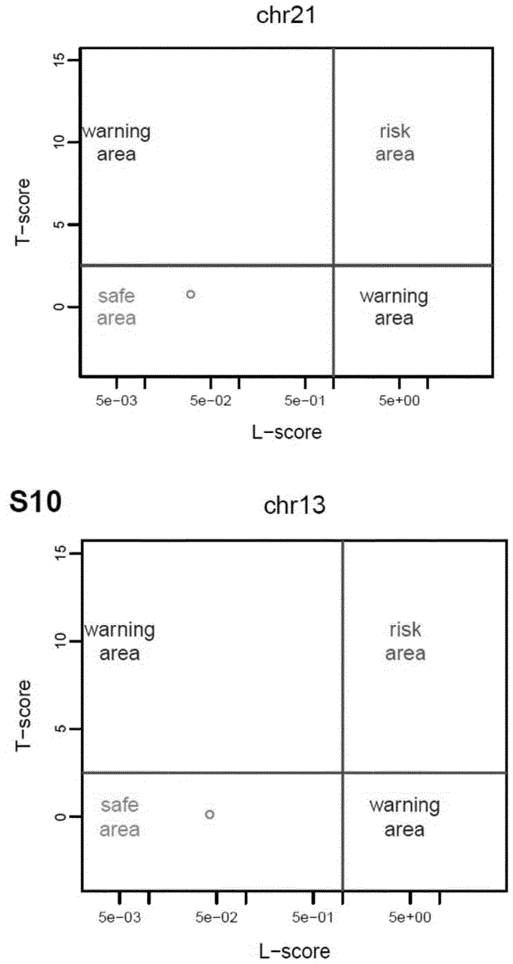

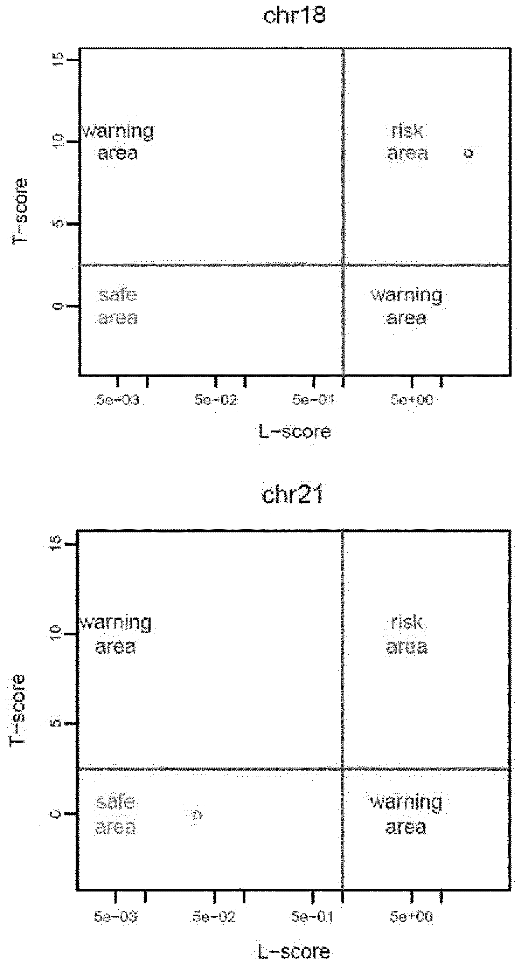

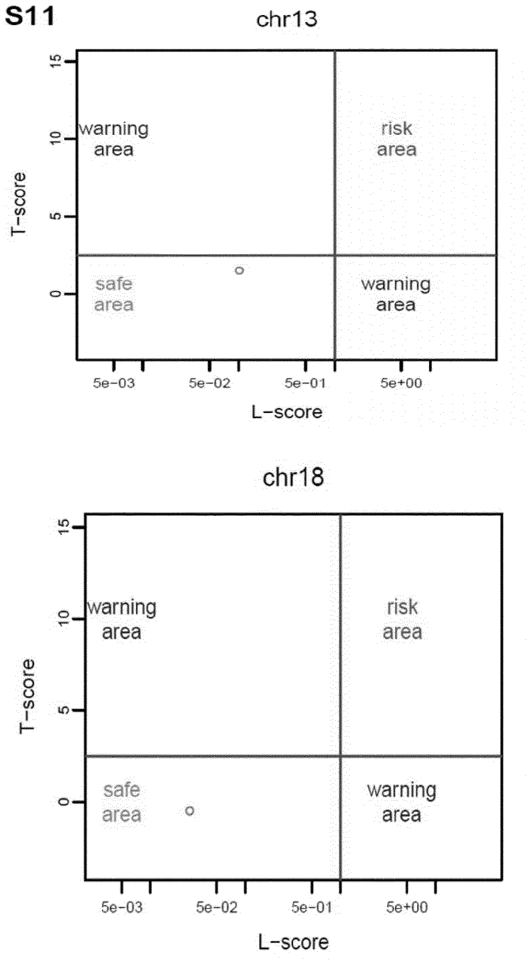

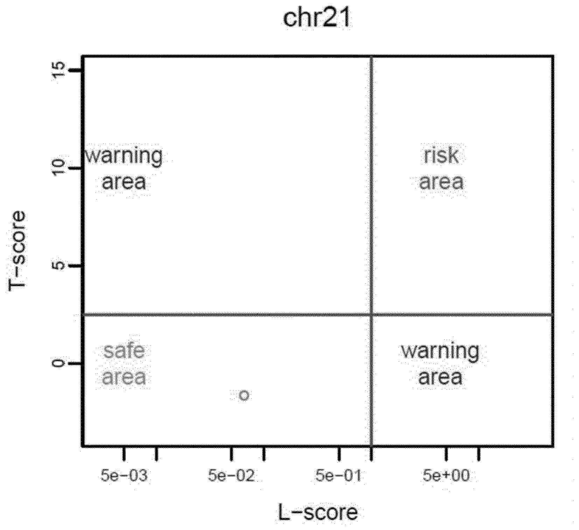

The four-quadrant diagrams ploted with T scores & L scores for the 11 samples tested are shown in Figs. 14 to end. It can be seen from these figures that the chromosome aneuploidy of twins of the 11 pregnant women under detection determined by T score & L score is in conformity with that determined in step 2 by fetal fraction.

**Example 3: Chimera detection**

In the following examples, a mixture of DNA fragments obtained from an abortion tissue and plasma obtained from a woman not pregnant in a certain proportion was used to simulate a sample obtained from a pregnant woman. Chromosome number abnormality (trisome, complete monosome, trisome chimera, monosome chimera) of fetus (male) was detected according to the following method, which includes steps as follows.

1) Whole genome sequencing (WGS): the sample under detection was subjected to whole genome sequencing using the high-throughput platform.

2) Position information of effective reads was obtained: reads of a test sample were uniquely aligned to the reference genome sequence to obtain position information, corresponding to the reference genome, of the uniquely-mapped reads.

3) Fraction of uniquely-mapped reads in each chromosome and percentage of the number of guanines (G bases) and cytosines (C bases) of uniquely-mapped reads to total number of bases in each chromosome were obtained: the percentage of the number of effective reads in each chromosome in a sample under detection to the total number of effective reads thereof and the percentage of the number of G and C bases in effective reads in each chromosome to total number of bases thereof, using position information and base information of effective reads, were obtained.

4) DNA fraction was calculated by chromosome i, which was calculated as $fra.chri$,

$fra.chri$ = 2*($chrt.ER\%$/$adjust.chri.ER\%$-1)*100%, where $fra,chri$ represents a cell-free fetal DNA fraction, i represents a serial number of a predetermined chromosome and i is any integer in the range of 1 to 22;

$chri.ER\%$ : $ER\%$ (short for effective reads rate, percentage of uniquely-mapped reads) of chromosome i in a sample;

$adjust.chri.ER\%$ : a theoretical value of $ER\%$ of chromosome i in a normal sample;

5) Fetal fraction was calculated by chromosome Y, which was calculated as $fra.chry$;

$fra.chry$ = ($chry.ER\%$ - $Female.chry.ER\%$)/($Man.chry.ER\%$-$Female.chry.ER\%$)*100%, where $chry.ER\%$ : $ER\%$ (short for effective reads rate, percentage of uniquely-mapped reads) of chromosome Y in a sample under detection;

$Female.chry.ER\%$ : an average of $ER\%$ of chromosome Y in a sample under detection obtained from a pregnant woman with a female fetus;

$Man.chry.ER\%$ : an average of $ER\%$ of chromosome Y in a sample under detection obtained from a man.

6) Determination standard:

I. If fra.chri/fra.chry<A1 (A1 is a certain constant, and A1>-1, such as -0.85), chromosome i of a fetus is a complete monosome;

II. If $fra.chri$ / $fra.chry$ $\in$ $[A_1, A_2]$ (A1 and A2 are certain constants, and -1<A1<A2<0, such as [-0.85,-0.3]), chromosome i of a fetus is a monosome chimera;

III. If $fra.chri$ / $fra.chry$ $\in$ $[A_2, A_3]$ (A2 and A3 are certain constants, and A2<0<A3, such as [-0.3,0.3]), chromosome i of a fetus is normal;

IV. If $fra.chri$ / $fra.chry$ $\in$ $[A_3, A_4]$ (A3 and A4 are certain constants, and 0<A3<A4<1, such as [0.3,0.85]), chromosome i of a fetus is a trisome chimera;

V. If fra.chri/fra.chry>A4 (A4 is a certain constant, such as 0.85), chromosome i of a fetus is a complete trisome.

3.1 Chromosome aneuploidy was investigated in 19 samples (M1'''M19) each of which was a mixture of DNA fragments obtained from an abortion tissue and plasma obtained from a woman not pregnant (details shown in table A) and 2 plasma samples (N1, N2) respectively obtained from 2 pregnant women with a male fetus (the male fetus in one pregnant woman had a T18 chimera, and the male fetus in the other pregnant woman had a T21 chimera).

1) Sample collection and treatment

2 ml peripheral blood was extracted for plasma separation.

2) Library construction

The library was constructed according to plasma library construction requirements of Complete Genomics Inc.

3) Sequencing

The sequencing process was carried out strictly following the standard operating procedure of Complete Genomics Inc.

4) Data analysing

a) Whole genome sequencing (WGS): the sample under detection was subjected to whole genome sequencing using the high-throughput platform (Lengths of all cell-free DNA molecules were obtained by single-end sequencing or paired-end sequencing, which was important. The entire cell-free DNA molecule was required to be sequenced if by single-ended sequencing)

b) Position information of effective reads was obtained: reads of a test sample were uniquely aligned to the reference genome sequence to obtain position information, corresponding to the reference genome, of the uniquely-mapped reads.

c) Percentage of effective reads was obtained: Percentage of effective reads in each chromosome to total effective reads obtained in b) was obtained.

d) GC Correction:

ER and GC contents of each chromosome were fitted by using known data obtained from normal samples to obtain a relation formula: $ER_i = f_i(GC_i) + \varepsilon_i$, and a mean value $\overline{ER_i}$ of ER was calculated. For a sample to be analyzed, an ER value after correction was calculated according to the above relation formula and ER and GC of the sample.

$$\overline{\overline{ER}}_{ij} = \overline{ER}_i + \overline{\varepsilon}_i = \overline{ER}_i + ER_{ij} - f_i(GC_{ij})$$

e) fra.chri was calculated by chromosome i (i=13,18,21);

f) fra.chry was calculated by chromosome Y;

g) fra.size was calculated by the method for determining the cell-free fetal DNA fraction of the present disclosure;

h) T score was calculated: $T_i = (x_i - \mu_i)/\sigma_i$,

where $i$ : a serial number of chromosome (i=1, 2...22);

$x_i$: a percentage of effective reads of the chromosome i in an analytic sample;

$\mu_i$ : an average percentage of effective reads of the chromosome i selected as a reference system in a reference database;

$\sigma_i$ : a standard deviation of percentages of effective reads of chromosome i selected as the reference system in the reference database;

i) L score was calculated:

T2 value was firstly calculated: $T2_i = (x_i - \mu_i*(1+fra/2))/\sigma_i$,

L score was then calculated: $L_i = \log(d(T_i, a))/\log(d(T2_i, a))$,

where $d(T_i, a)$ and $d(T2_i, a)$ represent t distribution probability density function, a represents degree of freedom, *fra* represents fra.chry or fra.size.

♦ Chromosome aneuploidy of a sample under detection was determined by *fra.chry* which was estimated by chromosome Y (as shown in table A).

a) Determination standard:

I. If fra.chri/fra.chry<-0.85, chromosome i of a fetus is a complete monosome;

II. If *fra.chri / fra.chry* $\in$ [-0. 85,-0.3], chromosome i of a fetus is a monosome chimera;

III. If *fra.chri/fra.chry* $\in$ [-0.3,0.3], chromosome i of a fetus is normal;

IV. If *fra.chri / fra.chry* $\in$ [0.3,0.85], chromosome i of a fetus is a trisome chimera;

V. If fra.chri/fra.chry>0.85, chromosome i of a fetus is a complete trisome.

♦ Chromosome aneuploidy of a sample under detection was determined by T score & L score (*fra.chry was* estimated by chromosome Y) (as shown in table B)

a) A four-quadrant diagram was ploted based on T scores and L scores;

b)When T≤0, a four-quadrant diagram was ploted with T as vertical coordinate and absolute value of L as horizontal coordinate by zoning with a straight line where T=3 and a straight line where L=1 (a sample with fetal fraction <5% was determined to not meet the quality control),

I. The fetus was determined to have a monosome or monosome chimera if a sample under detection was determined to be of a T score and a L score (T>3,L>1) falling into a first quadrant;

II. The fetus was determined to have a monosome chimera if a sample under detection was determined to be of a T score and a L score (T>3,L≤1) falling into a second quadrant;

III. The fetus was determined to be normal if a sample under detection was determined to be of a T score and a L score (T≤3,L≤1) falling into a third quadrant;

IV. The fetus was determined to have given rise to a low fetal fraction if a sample under detection was determined to be of a T score and a L score (T≤3,L>1) falling into a fourth quadrant; such a sample did not meet the quality control.

when T>0, a four-quadrant diagram was ploted with T as vertical coordinate and L as horizontal coordinate by zoning with a straight line where T=3 and a straight line where L=0.8 (a sample with fetal fraction <5% was determined to not meet the quality control),

I. The fetus was determined to have a trisome or a trisome chimera if a sample under detection was determined to be of a T score and a L score (T>3,L>1) falling into a first quadrant;

II. The fetus was determined to have a trisome chimera if a sample under detection was determined to be of a T score and a L score (T>3,L≤1) falling into a second quadrant;

III. The fetus was determined to be normal if a sample under detection was determined to be of a T score and a L score (T≤3,L≤1) falling into a third quadrant;

IV. The fetus was determined to have given rise to a low fetal fraction if a sample under detection was determined to be of a T score and a L score (T≤3,L>1) falling into a fourth quadrant; such a sample did not meet the quality control.

♦ Chromosome aneuploidy of a sample under detection was determined by *fra. size* which was estimated by the method for determining the cell-free fetal DNA fraction of the present disclosure (as shown in table C)

b) Determination standard:

I. If fra.chri/fra.chry<-0.85, chromosome i of a fetus is a complete monosome;

II. If *fra.chri / fra.chry* ∈ [-0.85,-0.3], chromosome i of a fetus is a monosome chimera;

III. If *fra.chri / fra.chry* ∈ [-0.3,0.3], chromosome i of a fetus is normal;

IV. If *fra.chri / fra.chry* ∈ [0.3,0.85], chromosome i of a fetus is a trisome chimera;

V. If fra.chri/fra.chry>0.85, chromosome i of a fetus is a complete trisome

♦ Chromosome aneuploidy of a sample under detection was determined by T score & L score (fra. size was estimated by the method for determining the cell-free fetal DNA fraction of the present disclosure) (as shown in table D, Fig. 2)

a) A four-quadrant diagram was ploted based on T scores and L scores;

b) When T≤0, a four-quadrant diagram was ploted with T as vertical coordinate and absolute value of L as horizontal coordinate by zoning with a straight line where T=3 and a straight line where L=1 (a sample with fetal fraction <5% was determined to not meet the quality control),

I. The fetus was determined to have a monosome or a monosome chimera if a sample under detection was determined to be of a T score and a L score (T>3,L>1) falling into a first quadrant;

II. The fetus was determined to have a monosome chimera if a sample under detection was determined to be of a T score and a L score (T>3,L≤1) falling into a second quadrant;

III. The fetus was determined to be normal if a sample under detection was determined to be of a T score and a L score (T≤3,L≤1) falling into a third quadrant;

IV. The fetus was determined to have a low fetal fraction if a sample under detection was determined to be of a T score and a L score (T≤3,L>1) falling into a fourth quadrant; such a sample did not meet the quality control;

When T>0, a four-quadrant diagram was ploted with T as vertical coordinate and L as horizontal coordinate by zoning with a straight line where T=3 and a straight line where L=1 (a sample with fetal fraction <5% was determined to not meet the quality control),

I. The fetus was determined to have a trisome or a trisome chimera if a sample under detection was determined to be of a T score and a L score (T>3,L>1) falling into a first quadrant;

II. The fetus was determined to have a trisome chimera if a sample under detection was determined to be of a T score and a L score (T>3,L≤1) falling into a second quadrant;

III. The fetus was determined to be normal if a sample under detection was determined to be of a T score and a L score (T≤3,L≤1) falling into a third quadrant;

IV. The fetus was determined to have given rise to a low fetal fraction if a sample under detection was determined to be of a T score and a L score (T≤3,L>1) falling into a fourth quadrant; such a sample did not meet the quality control.

In this embodiment, a negative sample was a plasma sample obtained from a normal woman who was not pregnant; a positive sample was prepared by mixing DNA fragments which were obtained by randomly breaking DNAs from an abortion tissue in accordance with a size ranging from 150bp to 200bp and plasma obtained from a normal woman who was not pregnant; (T21, T18 each represent a male fetus; T13 represents a female fetus); a positive chimeric sample was prepared by mixing placental tissue DNA fragments (which were obtained by randomly breaking DNAs from a placental tissue in accordance with a size ranging from 150bp to 200bp), Chinese cell line DNA fragments (which were obtained by randomly breaking DNAs from the Chinese cell line in accordance with a size ranging from 150bp to 200bp) and plasma obtained from a normal woman; (T21, T18 each represent a male fetus; T13 represents a female fetus).

| Sample No. | Abortion tissue karyotype | Fetal fraction | Chimeric ratio | Expression form |
| --- | --- | --- | --- | --- |
| M1 | trisome 13 | 3.5% | 0 | T13-3.5% |
| M2 | trisome 13 | 5% | 0 | T13-5% |
| M3 | trisome 13 | 8% | 0 | T13-8% |
| M4 | trisome 13 | 8% | 0 | T13-8% |
| M5 | trisome 18 | 10% | 30% | T18-10%-30% |
| M6 | trisome 18 | 10% | 70% | T18-10%-70% |
| M7 | trisome 18 | 10% | 70% | T18-10%-70% |
| M8 | trisome 18 | 10% | 70% | T18-10%-70% |
| M9 | trisome 18 | 3.5% | 0 | T18-3.5% |
| M10 | trisome 18 | 5% | 0 | T18-5% |
| M11 | trisome 18 | 5% | 0 | T18-5% |
| M12 | trisome 18 | 8% | 0 | T18-8% |
| M13 | trisome 18 | 10% | 30% | T21-10%-30% |
| M14 | trisome 18 | 10%- | 70% | T21-10%-70% |
| M15 | trisome 18 | 10% | 70% | T21-10%-70% |
| M16 | trisome 18 | 10% | 70% | T21-10%-70% |
| M17 | trisome 18 | 3.5% | 0 | T21-3.5% |
| M18 | trisome 18 | 5% | 0 | T21-5% |
| M19 | trisome 18 | 8% | 0 | T21-8% |

| Sample No. | Karyotype results |
| --- | --- |
| N1 | 47,XN+18[3]/46,XN[20] |

(continued)

| Sample No. | Karyotype results |
|------------|-------------------|
| N2 | 47,XN+21[301/46,XN[18] |

Table A Chromosome aneuploidy detection by mixed DNA fraction estimated by chromosome Y

| Sample No. | fra.chr13 | fra.chr18 | fra.chr21 | fra.chry | fra.chr13/fra.chry | fra.chr18/fra.chry | fra.chr21/fra.chry | Detection result |
|---|---|---|---|---|---|---|---|---|
| M1 | 0.03531 | -4.8E-06 | -0.00742 | 0.00069 | | | | T13-3.5% |
| M2 | 0.04731 | -0.01207 | 0.007764 | -0.00002 | | | | T13-5% |
| M3 | 0.08136 | -0.00477 | -0.00129 | -0.0002 | | | | T13-8% |
| M4 | 0.0796 | -0.00738 | -0.0013 | -0.00157 | | | | T13-8% |
| M5 | -0.01079 | 0.03401 | -0.00442 | 0.105215 | -0.10259 | 0.323243 | -0.04197 | T18-10%-30% |
| M6 | -0.00192 | 0.069573 | -0.00043 | 0.090435 | -0.02127 | 0.769319 | -0.00471 | T18-10%-70% |
| M7 | 0.001756 | 0.058363 | -0.00968 | 0.087785 | 0.020009 | 0.664844 | -0.11022 | T18-10%-70% |
| M8 | -0.01081 | 0.082063 | -0.00917 | 0.106115 | -0.1019 | 0.773343 | -0.08638 | T18-10%-70% |
| M9 | 0.007816 | 0.0341 | -0.00038 | 0.03723 | 0.209951 | 0.915928 | -0.01009 | T18-3.5% |
| M10 | 0.013116 | 0.0512 | -0.00607 | 0.04843 | 0.270834 | 1.057196 | -0.12525 | T18-5% |
| M11 | 0.000956 | 0.0513 | -0.01176 | 0.0566 | 0.016899 | 0.90636 | -0.2077 | T18-5% |
| M12 | -0.00237 | 0.07693 | -0.004 | 0.08877 | -0.02674 | 0.866622 | -0.04501 | T18-8% |
| M13 | 0.001246 | -0.01028 | 0.04323 | 0.110455 | 0.011285 | -0.09311 | 0.391381 | T21-10%-30% |
| M14 | 0.000266 | -0.01586 | 0.06554 | 0.092805 | 0.002871 | -0.17095 | 0.706212 | T21-10%-70% |
| M15 | -0.00041 | 0.004795 | 0.07461 | 0.110035 | -0.00376 | 0.043578 | 0.678057 | T21-10%-70% |
| M16 | -0.00235 | -0.00759 | 0.06985 | 0.097155 | -0.02422 | -0.07817 | 0.718954 | T21-10%-70% |
| M17 | 0.005776 | 0.010345 | 0.0481 | 0.03478 | 0.166086 | 0.297445 | 1.382979 | T21-3.5% |
| M18 | 0.007786 | 0.006345 | 0.0557 | 0.04853 | 0.160447 | 0.130747 | 1.147744 | T21-5% |
| M19 | 0.009846 | -0.00828 | 0.07449 | 0.06855 | 0.143639 | -0.12086 | 1.086652 | T21-8% |
| N1 | 0.007053 | 0.043212 | 0.000938 | 0.110337 | 0.063922 | 0.391637 | 0.008501 | 47,XN+18[3]/46,XN[20] |
| N2 | -0.01105 | 0.002921 | 0.081665 | 0.167834 | -0.06581 | 0.017404 | 0.486582 | 47,XN+21[30]/46,XN[18] |

Note:
fra.chr13: represents a mixed DNA fraction estimated by chromosome 13;
fra.chr18: represents a mixed DNA fraction estimated by chromosome 18;
fra.chr21: represents a mixed DNA fraction estimated by chromosome 21;
fra.chry: represents a mixed DNA fraction calculated by chromosome Y;
T21-10%-30%: DNA fragments obtained from a cell line with trisome 21 mixed with female plasma in accordance with a fraction of 10% and a chimeric ratio of 30%.

Table B Chromosome aneuploidy detection by T score & L score (mixed DNA fraction was estimated by chromosome Y)

| Sample No. | T.chr13 | L.chr13 | T.chr18 | L.chr18 | T.chr21 | L.chr21 | fra.chry | Detection result |
|---|---|---|---|---|---|---|---|---|
| M1 | 4.345751 | | -0.00053 | | -0.60057 | | 0.00069 | T13-3.5% |
| M2 | 5.436515 | | -1.216 | | 0.52145 | | -0.00002 | T13-5% |
| M3 | 10.46607 | | -0.47762 | | -0.09072 | | -0.0002 | T13-8% |
| M4 | 9.126057 | | -0.70384 | | -0.08565 | | -0.00157 | T13-8% |
| M5 | -1.43378 | 0.018171 | 4.16099 | 0.292408 | -0.39025 | 0.023403 | 0.105215 | T18-10%-30% |
| M6 | -0.20845 | 0.015392 | 7.977908 | 12.56388 | -0.03313 | 0.031699 | 0.090435 | T18-10%- 70% |
| M7 | 0.210445 | 0.015766 | 7.151443 | 4.550099 | -0.82976 | 0.035673 | 0.087785 | T18-10%- 70% |
| M8 | -1.19968 | 0.019721 | 8.763087 | 10.86877 | -0.7035 | 0.030472 | 0.106115 | T18-10%- 70% |
| M9 | 0.963351 | 0.189495 | 3.96625 | 9.33211 | -0.03142 | 0.162273 | 0.03723 | T18-3.5% |
| M10 | 1.8534 | 0.251063 | 6.429505 | 19.56264 | -0.51602 | 0.082144 | 0.04843 | T18-5% |
| M11 | 0.112697 | 0.031697 | 6.659763 | 23.63495 | -1.01942 | 0.072816 | 0.0566 | T18-5% |
| M12 | -0.24379 | 0.016926 | 8.332999 | 25.4093 | -0.2922 | 0.035987 | 0.08877 | T18-8% |
| M13 | 0.164157 | 0.010591 | -1.22048 | 0.017938 | 3.76578 | 0.491369 | 0.110455 | T21-10%-30% |
| M14 | 0.037473 | 0.013915 | -1.90428 | 0.036927 | 5.667693 | 4.554072 | 0.092805 | T21-10%-70% |
| M15 | -0.05039 | 0.011486 | 0.536781 | 0.017359 | 5.897363 | 3.910694 | 0.110035 | T21-10%-70% |
| M16 | -0.28883 | 0.01242 | -0.86821 | 0.018234 | 6.167815 | 5.406755 | 0.097155 | T21-10%-70% |
| M17 | 0.581767 | 0.19567 | 1.077291 | 0.427888 | 3.582341 | 4.827084 | 0.03478 | T21-3.5% |
| M18 | 0.93815 | 0.145201 | 0.654071 | 0.129238 | 3.958377 | 8.227322 | 0.04853 | T21-5% |
| M19 | 1.201005 | 0.051788 | -1.03581 | 0.032569 | 6.795784 | 21.00502 | 0.06855 | T21-8% |
| N1 | 0.703062 | 0.022374 | 5.418144 | 0.435182 | 0.081371 | 0.02066 | 0.110337 | 47,XN+18[3]/46,XN[20] |

| Sample No. | T.chr13 | L.chr13 | T.chr18 | L.chr18 | T.chr21 | L.chr21 | fra.chry | Detection result |
|---|---|---|---|---|---|---|---|---|
| N2 | -0.97092 | 0.012246 | 0.355485 | 0.005614 | 7.170205 | 0.903486 | 0.167834 | 47,XN+21[30]/46,XN[18] |

Note:
T.chr13: T score of chromosome 13;
L.chr13: L score of chromosome 13;
T.chr18: T score of chromosome 18;
L.chr18: L score of chromosome 18;
T.chr21: T score of chromosome 21;
L.chr21: L score of chromosome 21;
fra.chry: mixed fetal DNA fraction estimated by chromosome Y;
T21-10%-30%: DNA fragments obtained from a cell line with trisome 21 mixed with female plasma in accordance with a fraction of 10% and a chimeric ratio of 30%

Table C Chromosome aneuploidy detection by fra.size

| Sample No. | fra.chr13 | fra.chr18 | fra.chr21 | fra.size | fra.chr13/fra.size | fra.chr18/fra.size | fra.chr21/fra.size | Detection result |
|---|---|---|---|---|---|---|---|---|
| M1 | 0.03531 | -4.8E-06 | -0.00742 | 0.0369 | 0.956911 | -0.00013 | -0.20097 | T13-3.5% |
| M2 | 0.04731 | -0.01207 | 0.007764 | 0.05519 | 0.857221 | -0.21879 | 0.140682 | T13-5% |
| M3 | 0.08136 | -0.00477 | -0.00129 | 0.08462 | 0.961475 | -0.05643 | -0.01519 | T13-8% |
| M4 | 0.0796 | -0.00738 | -0.0013 | 0.07332 | 1.085652 | -0.10072 | -0.01767 | T13-8% |
| M5 | -0.01079 | 0.03401 | -0.00442 | 0.1071 | -0.10078 | 0.317554 | -0.04123 | T18-10%-30% |
| M6 | -0.00192 | 0.069573 | -0.00043 | 0.0928 | -0.02073 | 0.749713 | -0.00459 | T18-10%-70% |
| M7 | 0.001756 | 0.058363 | -0.00968 | 0.1058 | 0.016602 | 0.551638 | -0.09145 | T18-10%-70% |
| M8 | -0.01081 | 0.082063 | -0.00917 | 0.1233 | -0.0877 | 0.665558 | -0.07434 | T18-10%-70% |
| M9 | 0.007816 | 0.0341 | -0.00038 | 0.0432 | 0.180937 | 0.789352 | -0.0087 | T18-3.5% |
| M10 | 0.013116 | 0.0512 | -0.00607 | 0.05688 | 0.230599 | 0.900141 | -0.10664 | T18-5% |
| M11 | 0.000956 | 0.0513 | -0.01176 | 0.0438 | 0.021838 | 1.171233 | -0.2684 | T18-5% |
| M12 | -0.00237 | 0.07693 | -0.004 | 0.06726 | -0.03529 | 1.14377 | -0.05941 | T18-8% |
| M13 | 0.001246 | -0.01028 | 0.04323 | 0.0705 | 0.017681 | -0.14588 | 0.613191 | T21-10%-30% |
| M14 | 0.000266 | -0.01586 | 0.06554 | 0.1056 | 0.002524 | -0.15024 | 0.620644 | T21-10%-70% |
| M15 | -0.00041 | 0.004795 | 0.07461 | 0.1205 | -0.00343 | 0.039794 | 0.61917 | T21-10%-70% |
| M16 | -0.00235 | -0.00759 | 0.06985 | 0.1165 | -0.0202 | -0.06519 | 0.599571 | T21-10%-70% |
| M17 | 0.005776 | 0.010345 | 0.0481 | 0.0409 | 0.141234 | 0.252938 | 1.176039 | T21-3.5% |
| M18 | 0.007786 | 0.006345 | 0.0557 | 0.04543 | 0.171395 | 0.139669 | 1.226062 | T21-5% |
| M19 | 0.009846 | -0.00828 | 0.07449 | 0.07848 | 0.125465 | -0.10557 | 0.949159 | T21-8% |
| N1 | 0.007053 | 0.043212 | 0.000938 | 0.102547 | 0.068778 | 0.421387 | 0.009147 | 47,XN+18[3]/46,XN[20] |
| N2 | -0.01105 | 0.002921 | 0.081665 | 0.198228 | -0.05572 | 0.014736 | 0.411975 | 47,XN+21[30]/46,XN[18] |

Note:
fra.chr13: represents a mixed DNA fraction estimated by chromosome 13;
fra.chr18: represents a mixed DNA fraction estimated by chromosome 18;
fra.chr21: represents a mixed DNA fraction estimated by chromosome 21;
fra.size: represents a mixed DNA fraction estimated by the method of the present disclosure;
T21-10%-30%: DNA fragments obtained from a cell line with trisome 21 mixed with female plasma in accordance with a fraction of 10% and a chimeric ratio of 30%.

EP 3 178 941 B1

Table D Chromosome aneuploidy detection by T score & L score (fra.size)

| Sample No. | T.chr13 | L.chr13 | T.chr18 | L.chr18 | T.chr21 | L.21 | fra.size | Detection result |
|---|---|---|---|---|---|---|---|---|
| M1 | 4.345751 | 10.86269 | -0.00053 | 0.06264 | -0.60057 | 0.110064 | 0.0369 | T13-3.5% |
| M2 | 5.436515 | 13.40864 | -1.216 | 0.040421 | 0.52145 | 0.080194 | 0.05519 | T13-5% |
| M3 | 10.46607 | 36.3034 | -0.47762 | 0.013724 | -0.09072 | 0.026097 | 0.08462 | T13-8% |
| M4 | 9.126057 | 29.68988 | -0.70384 | 0.019292 | -0.08565 | 0.033903 | 0.07332 | T13-8% |
| M5 | -1.43378 | 0.015183 | 4.16099 | 0.216731 | -0.39025 | 0.019427 | 0.1071 | T18-10%-30% |
| M6 | -0.20845 | 0.01467 | 7.977908 | 10.52499 | -0.03313 | 0.030232 | 0.0928 | T18-10%-70% |
| M7 | 0.210445 | 0.011014 | 7.151443 | 1.813942 | -0.82976 | 0.026168 | 0.1058 | T18-10%-70% |
| M8 | -1.19968 | 0.015259 | 8.763087 | 4.176647 | -0.7035 | 0.023665 | 0.1233 | T18-10%-70% |
| M9 | 0.963351 | 0.136353 | 3.96625 | 6.810597 | -0.03142 | 0.126392 | 0.0432 | T18-3.5% |
| M10 | 1.8534 | 0.166895 | 6.429505 | 20.92116 | -0.51602 | 0.062989 | 0.05688 | T18-5% |
| M11 | 0.112697 | 0.051911 | 6.659763 | 13.06329 | -1.01942 | 0.107019 | 0.0438 | T18-5% |
| M12 | -0.24379 | 0.028322 | 8.332999 | 19.64862 | -0.2922 | 0.058661 | 0.06726 | T18-8% |
| M13 | 0.164157 | 0.025146 | -1.22048 | 0.037995 | 3.76578 | 2.372928 | 0.0705 | T21-10%-30% |
| M14 | 0.037473 | 0.010899 | -1.90428 | 0.030091 | 5.667693 | 2.443191 | 0.1056 | T21-10%-70% |
| M15 | -0.05039 | 0.009693 | 0.536781 | 0.014533 | 5.897363 | 2.522845 | 0.1205 | T21-10%-70% |
| M16 | -0.28883 | 0.008906 | -0.86821 | 0.013317 | 6.167815 | 2.195736 | 0.1165 | T21-10%-70% |
| N1 | 0.703062 | 0.030365 | 5.418144 | 0.576578 | 0.081371 | 0.02724 | 0.102547 | 47,XN+18[3]/46,XN[20] |
| N2 | -0.97092 | 0.013866 | 0.355485 | 0.007248 | 7.170205 | 0.553012 | 0.198228 | 47,XN+21[30]/46,XN[18] |

Note:
T.chr13: T score of chromosome 13;
L.chr13: L score of chromosome 13;
T.chr18: T score of chromosome 18;
L.chr18: L score of chromosome 18;
T.chr21: T score of chromosome 21;
L.chr21: L score of chromosome 21;
fra.size: mixed DNA fraction estimated by the method of the present disclosure;
T21-10%-30%: DNA fragments obtained from a cell line with trisome 21 mixed with female plasma in accordance with a fraction of 10% and a chimeric ratio of 30%.

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example," "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

**Claims**

1. A method for determining the fraction of cell-free fetal nucleic acids in a peripheral blood sample from a pregnant woman, comprising:

   (i) performing sequencing on cell-free nucleic acids contained in the sample so as to obtain a sequencing result consisting of a plurality of sequencing data;
   (ii) determining the number of the cell-free nucleic acids of a length falling into a predetermined range in the sample based on the sequencing result; and
   (iii) determining the fraction of cell-free fetal nucleic acids in the sample based on the number of cell-free nucleic acids of a length falling into the predetermined range, wherein said predetermined range is determined prior to step (i) by the following steps:

      (a) determining lengths of the cell-free nucleic acids in a plurality of control peripheral blood samples from pregnant women in each of which the fraction of cell-free fetal nucleic acids is known;
      (b) setting a plurality of candidate length ranges and determining the percentage of cell-free nucleic acids obtained from each of the plurality of control samples present in each candidate length range;
      (c) determining a correlation coefficient for each candidate length range based on the percentage of cell-free nucleic acids obtained from each of the plurality of control samples present in each candidate length range and the known fraction of cell-free fetal nucleic acids in each of the control samples; and
      (d) determining at least one candidate length range or a combination of the candidate length ranges as the predetermined range based on the maximum correlation coefficient.

2. A method according to claim 1, wherein determining the number of the cell-free nucleic acids of a length falling into the predetermined range in the sample based on the sequencing result further comprises:

   (a) aligning the sequencing result to a reference genome, so as to construct a dataset consisting of a plurality of uniquely-mapped reads, where each read in the dataset can be mapped to a single position of the reference genome;
   (b) determining the length of the cell-free nucleic acid corresponding to each uniquely-mapped read in the dataset; and
   (c) determining the number of the cell-free nucleic acids of a length falling into the predetermined range.

3. A method according to claim 2 , wherein determining the length of the cell-free nucleic acid corresponding to each uniquely-mapped read in the dataset further comprises:
   determining the length of each read uniquely-mapped to the reference genome as the length of the cell-free nucleic acid corresponding to the read.

4. A method according to claim 2, wherein cell-free nucleic acids in the sample are sequenced by paired-end sequencing and determining the length of the cell-free nucleic acid corresponding to each uniquely-mapped read in the dataset further comprises:

   determining the position corresponding to the reference genome of the 5'-end of the cell-free nucleic acid based on the sequencing data for the 5' end of each uniquely-mapped read obtained in the paired-end sequencing;
   determining the position corresponding to the reference genome of the 3'-end of the cell-free nucleic acid based on the sequencing data at the other end of the same uniquely-mapped read obtained in the paired-end sequencing; and
   determining the length of the cell-free nucleic acid based on the positions of the 5'-end and 3'-end of the cell-free nucleic acid.

5. A method according to claim 1, wherein determining the fraction of cell-free fetal nucleic acids in the sample based on the number of the cell-free nucleic acids of a length falling into the predetermined range further comprises:

determining the percentage of cell-free nucleic acids present in the predetermined range based on the number of cell-free nucleic acids of a length falling into the predetermined range; and
determining the fraction of cell-free fetal nucleic acids in the sample, based on the percentage of the cell-free nucleic acids present in the predetermined range according to a predetermined function,
wherein the predetermined function is determined based on the plurality of control samples.

6. A method according to claim 5, wherein the predetermined function is obtained by following steps:

(i) determining the percentage of cell-free nucleic acids obtained from each control sample present in the predetermined range; and
(ii) fitting the percentage of cell-free nucleic acids obtained from each control sample present in the predetermined range with the known fraction of cell-free fetal nucleic acids to determine said function; optionally wherein the percentage of cell-free nucleic acids obtained from each control sample present in the predetermined range is fitted with the known fraction of cell-free fetal nucleic acids by a linear fitting.

7. A method according to claim 1, wherein the predetermined range is 185 bp to 204 bp.

8. A method according to claim 5, wherein the predetermined function is d=0.0334*p+1.6657, where d represents a fraction of cell-free fetal nucleic acids, and p represents the percentage of cell-free nucleic acid present in the predetermined range.

9. A method according to claim 8, wherein the control samples are peripheral blood samples obtained from pregnant women with a normal male fetus, in which the fraction of cell-free nucleic acids which are cell-free fetal nucleic acids is known to be determined by chromosome Y.

10. A method according to claim 1, wherein said fraction of cell-free fetal nucleic acids is determined in a peripheral blood sample obtained from a pregnant woman with twins as a first cell-free fetal DNA fraction and which further comprises:

(a) determining a second cell-free fetal DNA fraction based on sequencing data derived from chromosome Y in the sequencing result; and
(b) determining sex of the twins based on the first cell-free fetal DNA fraction and said second cell-free fetal DNA fraction.

11. A method according to claim 10, wherein said second cell-free fetal DNA fraction is determined according to the following formula:

$$fra.chry = (chry.ER\% - Female.chry.ER\%)/(Man.chry.ER\% - Female.chry.ER\%)*100\% \quad ,$$

where *fra.chry* represents said second cell-free fetal DNA fraction, *chry.ER*% represents a percentage of the sequencing data derived from chromosome Y in the sequencing result to total sequencing data;
*Female.chry.ER*% represents an average percentage of sequencing data of cell-free nucleic acids aligning to chromosome Y in a peripheral blood sample obtained from a pregnant woman predetermined to be with a normal female fetus to total sequencing data thereof; and *Man.chry.ER*% represents an average percentage of sequencing data of cell-free nucleic acids derived from chromosome Y in a peripheral blood sample obtained from a healthy man to total sequencing data thereof.

12. A method according to claim 10 or claim 11, wherein determining the sex of the twins based on the first cell-free fetal DNA fraction and said second cell-free fetal DNA fraction further comprises:

(a) determining a ratio of said second cell-free fetal DNA fraction to the first cell-free fetal DNA fraction; and
(b) determining the sex of the twins by comparing the ratio determined in (a) with a predetermined first threshold and a predetermined second threshold,

wherein the first threshold has been determined based on a plurality of control samples obtained from pregnant women known to have female twins and the second threshold has been determined based on a plurality of control samples obtained from pregnant women known to have male twins, and wherein

both fetuses of the twins are determined as female if the ratio of said second cell-free fetal DNA fraction to the first cell-free fetal DNA fraction is lower than the first threshold,

both fetuses of the twins are determined as male if the ratio of said second cell-free fetal DNA fraction to the first cell-free fetal DNA fraction is greater than the second threshold, and

the twins are determined as including a male fetus and a female fetus if the ratio of said second cell-free fetal DNA fraction to the first cell-free fetal DNA fraction is equal to the first threshold or the second threshold, or between the first threshold and the second threshold.

13. A method as claimed in claim 12 wherein the first threshold is 0.35 and the second threshold is 0.7.

14. A method according to claim 1, wherein said fraction of cell-free fetal nucleic acids is determined in a peripheral blood sample obtained from a pregnant woman with twins as a first cell-free fetal DNA fraction and which further comprises:

(a) determining a third cell-free fetal DNA fraction, based on sequencing data derived from a predetermined chromosome in the sequencing result; and
(b) determining whether the twins under detection have aneuploidy with respect to the predetermined chromosome based on the first cell-free fetal DNA fraction and said third cell-free fetal DNA fraction.

15. A method according to claim 14, wherein said third cell-free fetal DNA fraction is determined according to the following formula:
$fra.chri = 2*(chri.ER\% / adjust.chri.ER\%-1)*100\%$, where $fra.chri$ represents said third cell-free fetal DNA fraction, i represents a serial number of the predetermined chromosome, and i is any integer in the range of 1 to 22; $chri.ER\%$ represents a percentage of the sequencing data derived from the predetermined chromosome in the sequencing result to total sequencing data; $adjust.chri.ER\%$ represents an average percentage of sequencing data of cell-free nucleic acids derived from the predetermined chromosome in a peripheral blood sample obtained from a pregnant woman predetermined to be with normal twins to total sequencing data thereof.

16. A method according to claim 14 or claim 15, wherein determining whether the twins under detection have aneuploidy with respect to the predetermined chromosome based on the first cell-free fetal DNA fraction and said third cell-free fetal DNA fraction further comprises:

(a) determining a ratio of said third cell-free fetal DNA fraction to the first cell-free fetal DNA fraction; and
(b) determining whether the twins under detection have aneuploidy with respect to the predetermined chromosome by comparing the ratio determined in (a) with a predetermined third threshold and a predetermined fourth threshold,

and wherein the third threshold has been determined based on a plurality of control samples obtained from pregnant women with twins known not to have aneuploidy with respect to the predetermined chromosome and the fourth threshold has been determined based on a plurality of control samples obtained from pregnant women with twins known to have aneuploidy with respect to the predetermined chromosome, and wherein

both fetuses of the twins are determined to have no aneuploidy with respect to the predetermined chromosome if the ratio of said third cell-free fetal DNA fraction to the first cell-free fetal DNA fraction is lower than the third threshold,

both fetuses of the twins are determined to have aneuploidy with respect to the predetermined chromosome if the ratio of said third cell-free fetal DNA fraction to the first cell-free fetal DNA fraction is greater than the fourth threshold, and

one fetus of the twins is determined to have aneuploidy with respect to the predetermined chromosome, while the other fetus of the twins is determined to have no aneuploidy with respect to the predetermined chromosome if the ratio of said third cell-free fetal DNA fraction to the first cell-free fetal DNA fraction is equal to the third threshold or the fourth threshold, or between the third threshold and the fourth threshold.

17. A method as claimed in claim 1, wherein the third threshold is 0.35 and the fourth threshold is 0.7.

18. A method according to claim 14, wherein the predetermined chromosome is at least one selected from chromosomes 18, 21 and 23.

19. A method according to claim 1, wherein said fraction of cell-free fetal nucleic acids is determined in a peripheral blood sample obtained from a pregnant woman with twins as a first cell-free fetal DNA fraction and which further comprises:

(a) determining a fraction $x_i$ of the number of sequencing data derived from chromosome i in the sequencing result to total sequencing data, where i represents a serial number of the chromosome, and i is any integer in the range of 1 to 22;

(b) determining a T score of the chromosome i according to $T_i = (x_i - \mu_i)/(\sigma_i$, where i represents the serial number of the chromosome and i is any integer in the range of 1 to 22, $\mu_i$ represents an average percentage of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof,

(c) determining an L score of the chromosome i according to $L_i = \log(d(T_i, a)) / \log(d(T2_i, a))$, where i represents the serial number of the chromosome and i is any integer in the range of 1 to 22, $T2_i = (x_i - \mu_i * (1 + fra/2))/\sigma_i$; $d(T_i, a)$ and $d(T2_i, a)$ represent t distribution probability density function, where a represents degree of freedom, *fra* represents said first cell-free fetal DNA fraction,

(d) plotting a four-quadrant diagram with T as vertical coordinate and L as horizontal coordinate by zoning with a first straight line where T=predetermined fifth threshold and a second straight line where L= predetermined sixth threshold,

wherein

both fetuses of the twins are determined to have a trisome if a sample under detection is determined to be of a T score and a L score falling into a first quadrant;

one fetus of the twins is determined to have a trisome and the other fetus of the twins is determined to be normal if a sample under detection is determined to be of a T score and a L score falling into a second quadrant;

both fetuses of the twins are determined to be normal if a sample under detection is determined to be of a T score and a L score falling into a third quadrant, and

the twins are determined to have given rise to a low fetal fraction if a sample under detection is determined to be of a T score and a L score falling into a fourth quadrant such that the result is not adopted.

20. A method according to claim 1, wherein said fraction of cell-free fetal nucleic acids is determined in a peripheral blood sample obtained from a pregnant woman with a fetus as a first cell-free fetal DNA fraction and which further comprises:

(a) determining a third cell-free fetal DNA fraction based on sequencing data derived from a predetermined chromosome in the sequencing result; and

(b) determining whether the fetus under detection has fetal chimera with respect to the predetermined chromosome based on the first cell-free fetal DNA fraction and said third cell-free fetal DNA fraction.

21. A method according to claim 20, wherein said third cell-free fetal DNA fraction is determined by the following formula: *fra.chri* = 2*(*chri.ER%* / *adjust.chri.ER%*-1) *100%, where *fra.chri* represents said third cell-free fetal DNA fraction, i represents a serial number of the predetermined chromosome and i is any integer in the range of 1 to 22; *chri.ER%* represents a percentage of the sequencing data derived from the predetermined chromosome in the sequencing result to total sequencing data; *adjust.chri.ER%* represents an average percentage of sequencing data of cell-free nucleic acids derived from the predetermined chromosome in a peripheral blood sample obtained from a pregnant woman predetermined to be with a normal fetus to total sequencing data thereof.

22. A method according to claim 20 or claim 21, wherein determining whether the fetus under detection has fetal chimera with respect to the predetermined chromosome based on the first cell-free fetal DNA fraction and said third cell-free fetal DNA fraction further comprises:

(a) determining a ratio of said third cell-free fetal DNA fraction to the first cell-free fetal DNA fraction; and

(b) determining whether the fetus under detection has chimera with respect to the predetermined chromosome by comparing the ratio determined in (a) with a plurality of predetermined thresholds.

23. A method according to claim 22, wherein the plurality of predetermined thresholds comprises at least one selected from:

a seventh threshold determined based on a plurality of control samples with the predetermined chromosome known to be a complete monosome,

an eighth threshold determined based on a plurality of control samples with the predetermined chromosome known to be a monosome chimera,

a ninth threshold determined based on a plurality of control samples with the predetermined chromosome known to be normal,

a tenth threshold determined based on a plurality of control samples with the predetermined chromosome known to be a complete trisome,

and wherein

the predetermined chromosome of the fetus under detection is determined to be a complete monosome if the ratio of said third cell-free fetal DNA fraction to the first cell-free fetal DNA fraction is lower than the seventh threshold;

the predetermined chromosome of the fetus under detection is determined to be a monosome chimera if the ratio of said third cell-free fetal DNA fraction to the first cell-free fetal DNA fraction is not lower than the seventh threshold and not greater than the eighth threshold;

the predetermined chromosome of the fetus under detection is determined to be normal if the ratio of said third cell-free fetal DNA fraction to the first cell-free fetal DNA fraction is greater than the eighth threshold and lower than the ninth threshold;

the predetermined chromosome of the fetus under detection is determined to be a trisome chimera, if the ratio of said third cell-free fetal DNA fraction to the first cell-free fetal DNA fraction is not lower than the ninth threshold and not greater than the tenth threshold; and

the predetermined chromosome of the fetus under detection is determined to be a complete trisome, if the ratio of said third cell-free fetal DNA fraction to the first cell-free fetal DNA fraction is greater than the tenth threshold.

24. A method according to claim 23, wherein

the seventh threshold is greater than -1 and lower than 0, for example -0.85;

the eighth threshold is greater than the seventh threshold and lower than 0, for example -0.3;

the ninth threshold is greater than 0 and lower than 1, for example 0.3; and

the tenth threshold is greater than the ninth threshold and lower than 1, for example 0.85.

25. A method according to claim 1, wherein said fraction of cell-free fetal nucleic acids is determined in a peripheral blood sample obtained from a pregnant woman with a fetus as a first cell-free fetal DNA fraction and which further comprises:

(a) determining a fraction $x_i$ of the number of sequencing data derived from chromosome i in the sequencing result to total sequencing data, where i represents a serial number of the chromosome, and i is any integer in the range of 1 to 22;

(b) determining a T score of the chromosome i according to $T_i = (x_i - \mu_i)/\sigma_i$, where i represents the serial number of the chromosome and i is any integer in the range of 1 to 22, $\mu_i$ represents an average value of percentages of sequencing data of the chromosome i selected as a reference system in a reference database to total sequencing data thereof, $\sigma_i$ represents a standard deviation of percentages of the sequencing data of the chromosome i selected as the reference system in the reference database to total sequencing data thereof,

(c) determining an L score of the chromosome i according to

$L_i = \log(d(T_i, a))/\log(d(T2_i, a))$, where i represents the serial number of the chromosome and i is any integer in the range of 1 to 22,

$T2_i = (x_i - \mu_i * (1 + fra/2))/\sigma_i$; $d(T_i, a)$ and $d(T2_i, a)$ represent t distribution probability density function, where a represents degree of freedom, *fra* represents said first cell-free fetal DNA fraction;

(d) plotting a four-quadrant diagram with T as vertical coordinate and L as horizontal coordinate by zoning with a third straight line where T=predetermined eleventh threshold and a fourth straight line where L= predetermined twelfth threshold, when the T score is not greater than 0,
wherein

the fetus is determined to have a complete monosome or monosome chimera with respect to the predetermined chromosome if a sample under detection is determined to be of a T score and a L score falling into a first quadrant;

the fetus is determined to have a monosome chimera with respect to the predetermined chromosome if a sample under detection is determined to be of a T score and a L score falling into a second quadrant;

the fetus is determined to be normal with respect to the predetermined chromosome, if a sample under detection is determined to be of a T score and a L score falling into a third quadrant; and

the fetus is determined to have given rise to a low fetal fraction if a sample under detection is determined to be of a T score and a L score falling into a fourth quadrant such that the result is not adopted,

(e) plotting a four-quadrant diagram with T as vertical coordinate and L as horizontal coordinate by zoning with a first straight line where T=predetermined thirteenth threshold and a second straight line where L= predetermined fourteenth threshold, when the T score is greater than 0, wherein

the fetus is determined to have a complete trisome or trisome chimera with respect to the predetermined chromosome if a sample under detection is determined to be of a T score and a L score falling into a first quadrant;

the fetus is determined to have a trisome chimera with respect to the predetermined chromosome if a sample under detection is determined to be of a T score and a L score falling into a second quadrant;

the fetus is determined to be normal with respect to the predetermined chromosome if a sample under detection is determined to be of a T score and a L score falling into a third quadrant; and

the fetus is determined to have given rise to a low fetal fraction if a sample under detection is determined to be of a T score and a L score falling into a fourth quadrant such that the result is not adopted.

26. A method according to claim 25, wherein the eleventh threshold and the thirteenth threshold are each independently 3 and the twelfth threshold and the fourteenth threshold are each independently 1.


**Patentansprüche**

1. Verfahren zur Bestimmung des Anteils zellfreier fetaler Nukleinsäuren in einer Probe von peripherem Blut von einer schwangeren Frau, wobei das Verfahren folgendes umfasst:

(i) Durchführen einer Sequenzierung an in der Probe enthaltenen zellfreien Nukleinsäuren, um ein Sequenzierungsergebnis zu erhalten, das eine Mehrzahl von Sequenzierungsdaten umfasst;

(ii) Bestimmen der Anzahl zellfreier Nukleinsäuren einer Länge, die in einem vorbestimmten Bereich in der Probe liegt, auf der Basis des Sequenzierungsergebnisses; und

(iii) Bestimmen des Anteils zellfreier fetaler Nukleinsäuren in der Probe auf der Basis der Anzahl zellfreier Nukleinsäuren einer Länge, die in dem vorbestimmten Bereich liegt, wobei der vorbestimmte Bereich vor Schritt (i) durch die folgenden Schritte bestimmt wird:

(a) Bestimmen der Längen der zellfreien Nukleinsäuren in einer Mehrzahl von Stichproben von peripherem Blut von schwangeren Frauen, wobei in jeder dieser Proben der Anteil der zellfreien Nukleinsäuren bekannt ist;

(b) Festlegen einer Mehrzahl von Kandidatenlängenbereichen und Bestimmen des prozentualen Anteils der aus jeder der Mehrzahl von Stichproben erhaltenen zellfreien Nukleinsäuren, die in jedem Kandidatenlängenbereich vorhanden sind;

(c) Bestimmen eines Korrelationskoeffizienten für jeden Kandidatenlängenbereich auf der Basis des prozentualen Anteils zellfreier Nukleinsäuren, der aus jeder der Mehrzahl von Stichproben erhalten wird, die in jedem Kandidatenlängenbereich vorhanden sind, sowie des bekannten Anteils zellfreier fetaler Nukleinsäuren in jeder der Stichproben; und

(d) Bestimmen mindestens eines Kandidatenlängenbereichs oder einer Kombination der Kandidatenlängenbereiche als den vorbestimmten Bereich auf der Basis des maximalen Korrelationskoeffizienten.

**2.** Verfahren nach Anspruch 1, wobei das Bestimmen der Anzahl der zellfreien Nukleinsäuren einer Länge, die in dem vorbestimmten Bereich in der Probe liegt, auf der Basis des Sequenzierungsergebnisses ferner folgendes umfasst:

(a) Abstimmen des Sequenzierungsergebnisses auf ein Referenzgenom, um einen Datensatz zu konstruieren, der aus einer Mehrzahl eindeutig abgebildete Ablesewerte besteht, wobei jeder Ablesewert in dem Datensatz an eine einzelne Position des Referenzgenoms abgebildet werden kann;
(b) Bestimmen der Länge der zellfreien Nukleinsäure entsprechend jedem eindeutig abgebildeten Ablesewert in dem Datensatz; und
(c) Bestimmen der Anzahl der zellfreien Nukleinsäuren einer Länge, die in dem vorbestimmten Bereich liegt.

**3.** Verfahren nach Anspruch 2, wobei das Bestimmen der Länge der zellfreien Nukleinsäure entsprechend jedem eindeutig abgebildeten Ablesewert in dem Datensatz ferner folgendes umfasst:
Bestimmen der Länge jedes eindeutig auf das Referenzgenom abgebildeten Ablesewertes als die Länge der zellfreien Nukleinsäure entsprechend dem Ablesewert.

**4.** Verfahren nach Anspruch 2, wobei die zellfreien Nukleinsäuren in der Probe durch Paired-End-Sequenzierung sequenziert werden, und wobei das Bestimmen der Länge der zellfreien Nukleinsäure entsprechend jedem eindeutig abgebildeten Ablesewert in dem Datensatz ferner folgendes umfasst:

Bestimmen der Position, die dem Referenzgenom des 5'-Endes der zellfreien Nukleinsäure entspricht, auf der Basis der Sequenzierungsdaten für das 5'-Ende jedes in der Paired-End-Sequenzierung erhaltenen eindeutig abgebildeten Ablesewertes;
Bestimmen der Position, die dem Referenzgenom des 3'-Endes der zellfreien Nukleinsäure entspricht, auf der Basis der Sequenzierungsdaten an dem anderen Ende des gleichen in der Paired-End-Sequenzierung erhaltenen eindeutig abgebildeten Ablesewertes; und
Bestimmen der Länge der zellfreien Nukleinsäure auf der Basis der Positionen des 5'-Endes und des 3'-Endes der zellfreien Nukleinsäure.

**5.** Verfahren nach Anspruch 1, wobei das Bestimmen des Anteils zellfreier fetaler Nukleinsäuren in der Probe auf der Basis der Anzahl zellfreier Nukleinsäuren einer Länge, die in dem vorbestimmten Bereich liegt, ferner folgendes umfasst:

Bestimmen des prozentualen Anteils der zellfreien Nukleinsäuren, die in dem vorbestimmten Bereich vorhanden sind, auf der Basis der Anzahl zellfreier Nukleinsäuren einer Länge, die in dem vorbestimmten Bereich liegt; und
Bestimmen des Anteils zellfreier fetaler Nukleinsäuren in der Probe auf der Basis des prozentualen Anteils der in dem vorbestimmten Bereich vorhandenen zellfreien Nukleinsäuren gemäß einer vorbestimmten Funktion, wobei die vorbestimmte Funktion auf der Basis der Mehrzahl von Stichproben bestimmt wird.

**6.** Verfahren nach Anspruch 5, wobei die vorbestimmte Funktion durch die folgenden Schritte erhalten wird:

(i) Bestimmen des aus jeder Stichprobe erhaltenen prozentualen Anteils zellfreier Nukleinsäuren, die in dem vorbestimmten Bereich vorhanden sind; und
(ii) Abstimmen des aus jeder Stichprobe erhaltenen prozentualen Anteils zellfreier Nukleinsäuren, die in dem vorbestimmten Bereich vorhanden sind, mit dem bekannten Anteil zellfreier fetaler Nukleinsäuren, um die genannte Funktion zu bestimmen; wobei optional der aus jeder Stichprobe erhaltene prozentuale Anteil zellfreier Nukleinsäuren, die in dem vorbestimmten Bereich vorhanden sind, mit dem bekannten Anteil zellfreier fetaler Nukleinsäuren durch lineare Abstimmung abgestimmt wird.

**7.** Verfahren nach Anspruch 1, wobei der vorbestimmte Bereich 185 bp bis 204 bp ist.

**8.** Verfahren nach Anspruch 5, wobei die vorbestimmte Funktion d=0,0334*p+1,6657 ist, wobei d einen Anteil zellfreier fetaler Nukleinsäuren bezeichnet, und wobei p den prozentualen Anteil in dem vorbestimmten Bereich vorhandener zellfreier Nukleinsäuren darstellt.

**9.** Verfahren nach Anspruch 8, wobei die Stichproben Proben von peripherem Blut sind, die von schwangeren Frauen mit einem normalen männlichen Fötus erhalten werden, wobei der Anteil zellfreier Nukleinsäuren, bei denen es sich um zellfreie fetale Nukleinsäuren handelt, als durch das Chromosom Y bestimmt bekannt ist.

**10.** Verfahren nach Anspruch 1, wobei der Anteil zellfreier fetaler Nukleinsäuren in einer Probe von peripherem Blut bestimmt wird, die von einer schwangeren Frau mit Zwillingen erhalten wird als ein erster zellfreier fetaler DNA-Anteil, und wobei das Verfahren ferner folgendes umfasst:

(a) Bestimmen eines zweiten zellfreien fetalen DNA-Anteils auf der Basis der von Chromosom Y in dem Sequenzierungsergebnis abgeleiteten Sequenzierungsdaten; und
(b) Bestimmen des Geschlechts der Zwillinge auf der Basis des ersten zellfreien fetalen DNA-Anteils und des zweiten zellfreien fetalen DNA-Anteils.

**11.** Verfahren nach Anspruch 10, wobei der zweite zellfreie fetale DNA-Anteil gemäß der folgenden Formel bestimmt wird:

$$fra.chry = (chry.ER\% - Female.chry.ER\%) \, / \, (Man.chry.ER\% - Female.chry.ER\%)*100\%,$$

wobei *fra.chry* den genannten zweiten zellfreien fetalen DNA-Anteil darstellt, wobei *chry.ER%* einen aus Chromosom Y in dem Sequenzierungsergebnis abgeleiteten prozentualen Anteil der Sequenzierungsdaten zu den Sequenzierungsdaten insgesamt darstellt;
wobei *Female.chry.ER%* einen durchschnittlichen prozentualen Anteil der Sequenzierungsdaten von zellfreien Nukleinsäuren darstellt, abgestimmt auf Chromosom Y in einer Probe von peripherem Blut, die von einer schwangeren Frau erhalten worden ist, für die ein normaler weiblicher Fetus vorbestimmt worden ist, zu den Sequenzierungsdaten davon insgesamt; und wobei *Man.chry.ER%* einen durchschnittlichen prozentualen Anteil der Sequenzierungsdaten von zellfreien Nukleinsäuren darstellt, abgestimmt auf Chromosom Y in einer Probe von peripherem Blut, die von einem gesunden Man erhalten worden ist, zu den Sequenzierungsdaten davon insgesamt.

**12.** Verfahren nach Anspruch 10 oder Anspruch 11, wobei die Bestimmung des Geschlechts der Zwillinge auf der Basis des ersten zellfreien fetalen DNA-Anteils und des zweiten zellfreien fetalen DNA-Anteils ferner folgendes umfasst:

(a) Bestimmen eines Verhältnisses des zweiten zellfreien fetalen DNA-Anteils zu dem ersten zellfreien fetalen DNA-Anteil; und
(b) Bestimmen des Geschlechts der Zwillinge durch Vergleichen des in (a) bestimmten Verhältnisses mit einem ersten Schwellenwert und mit einem vorbestimmten zweiten Schwellenwert,

wobei der erste Schwellenwert auf der Basis einer Mehrzahl von Stichproben bestimmt worden ist, die von schwangeren Frauen erhalten worden sind, bei denen bekannt war, dass sie weibliche Zwillinge tragen, und wobei der zweite Schwellenwert auf der Basis einer Mehrzahl von Stichproben bestimmt worden ist, die von schwangeren Frauen erhalten worden sind, bei denen bekannt war, dass sie männliche Zwillinge tragen,
und wobei
beide Föten der Zwillinge als weiblich bestimmt werden, wenn das Verhältnis des zweiten zellfreien fetalen DNA-Anteils zu dem ersten zellfreien fetalen DNA-Anteil kleiner ist als der erste Schwellenwert,
beide Föten der Zwillinge als männlich bestimmt werden, wenn das Verhältnis des zweiten zellfreien fetalen DNA-Anteils zu dem ersten zellfreien fetalen DNA-Anteil größer ist als der zweite Schwellenwert, und
für die Zwillinge bestimmt wird, dass sie einen männlichen Fötus und einen weiblichen Fötus umfassen, wenn das Verhältnis des zweiten zellfreien fetalen DNA-Anteils zu dem ersten zellfreien fetalen DNA-Anteil gleich dem ersten Schwellenwert oder gleich dem zweiten Schwellenwert ist oder zwischen dem ersten Schwellenwert und dem zweiten Schwellenwert liegt.

**13.** Verfahren nach Anspruch 12, wobei der erste Schwellenwert 0,35 ist, und wobei der zweite Schwellenwert 0,7 ist.

**14.** Verfahren nach Anspruch 1, wobei der Anteil zellfreier fetaler Nukleinsäuren in einer Probe von peripherem Blut, die von einer schwangeren Frau mit Zwillingen erhalten wird, als ein erster zellfreier fetaler DNA-Anteil bestimmt wird, und wobei das Verfahren ferner folgendes umfasst:

(a) Bestimmen eines dritten zellfreien fetalen DNA-Anteils auf der Basis von Sequenzierungsdaten, die von einem vorbestimmten Chromosom in dem Sequenzierungsergebnis abgeleitet werden; und
(b) Bestimmen, ob die untersuchten Zwillinge Aneuploidie in Bezug auf das vorbestimmte Chromosom aufweisen, auf der Basis des ersten zellfreien fetalen DNA-Anteils und des dritten zellfreien fetalen DNA-Anteils.

15. Verfahren nach Anspruch 14, wobei der dritte zellfreie fetale DNA-Anteil gemäß der folgenden Formel bestimmt wird: *fra.chri = 2\*(chri.ER%/adjust.chri.ER% - 1)\*100%*, wobei *fra.chri* den genannten dritten zellfreien fetalen DNA-Anteil darstellt, wobei i eine laufende Nummer des vorbestimmten Chromosoms darstellt, und wobei i eine beliebige ganze Zahl im Bereich von 1 bis 22 ist; wobei *chri.ER%* einen prozentualen Anteil der von dem vorbestimmten Chromosom abgeleiteten Sequenzierungsdaten in dem Sequenzierungsergebnis zu den Sequenzierungsdaten insgesamt darstellt; wobei *adjust.chri.ER%* einen durchschnittlichen prozentualen Anteil der Sequenzierungsdaten zellfreier Nukleinsäuren darstellt, die von dem vorbestimmten Chromosom in einer Probe von peripherem Blut, die von einer schwangeren Frau erhalten worden ist, für die normale Zwillinge vorbestimmt worden sind, zu den Sequenzierungsdaten davon insgesamt.

16. Verfahren nach Anspruch 14 oder Anspruch 15, wobei das Bestimmen, ob die untersuchten Zwillinge Aneuploidie in Bezug auf das vorbestimmte Chromosom aufweisen, auf der Basis des ersten zellfreien fetalen DNA-Anteils und des dritten zellfreien fetalen DNA-Anteils, ferner folgendes umfasst:

(a) Bestimmen eines Verhältnisses des dritten zellfreien fetalen DNA-Anteils zu dem ersten zellfreien fetalen DNA-Anteil; und
(b) Bestimmen, ob die untersuchten Zwillinge Aneuploidie in Bezug auf das vorbestimmte Chromosom aufweisen, durch Vergleichen des in (a) bestimmten Verhältnisses mit einem vorbestimmten dritten Schwellenwert und einem vorbestimmten vierten Schwellenwert,

und wobei der dritte Schwellenwert auf der Basis einer Mehrzahl von Stichproben bestimmt worden ist, die von schwangeren Frauen mit Zwillingen erhalten worden sind, für die bekannt ist, dass sie keine Aneuploidie in Bezug auf das vorbestimmte Chromosom aufweisen, und wobei der vierte Schwellenwert auf der Basis einer Mehrzahl von Stichproben bestimmt worden ist, die von schwangeren Frauen mit Zwillingen erhalten worden sind, für die bekannt ist, dass sie Aneuploidie in Bezug auf das vorbestimmte Chromosom aufweisen, und wobei
für beide Föten der Zwillinge bestimmt wird, dass sie keine Aneuploidie in Bezug auf das vorbestimmte Chromosom aufweisen, wenn das Verhältnis des dritten zellfreien fetalen DNA-Anteils zu dem ersten zellfreien fetalen DNA-Anteil kleiner ist als der dritte Schwellenwert,
für beide Föten der Zwillinge bestimmt wird, dass sie Aneuploidie in Bezug auf das vorbestimmte Chromosom aufweisen, wenn das Verhältnis des dritten zellfreien fetalen DNA-Anteils zu dem ersten zellfreien fetalen DNA-Anteil größer ist als der vierte Schwellenwert, und
für einen Fötus der Zwillinge bestimmt wird, dass er Aneuploidie in Bezug auf das vorbestimmte Chromosom aufweist, während für den anderen Fötus der Zwillinge bestimmt wird, dass er keine Aneuploidie in Bezug auf das vorbestimmte Chromosom aufweist, wenn das Verhältnis des dritten zellfreien fetalen DNA-Anteils zu dem ersten zellfreien fetalen DNA-Anteil gleich dem dritten Schwellenwert oder gleich dem vierten Schwellenwert ist oder zwischen dem dritten und dem vierten Schwellenwert liegt.

17. Verfahren nach Anspruch 11, wobei der dritte Schwellenwert 0,35 ist, und wobei der vierte Schwellenwert 0,7 ist.

18. Verfahren nach Anspruch 14, wobei das vorbestimmte Chromosom mindestens ein Chromosom ist, das ausgewählt ist aus den Chromosomen 18, 21 und 23.

19. Verfahren nach Anspruch 1, wobei der Anteil zellfreier fetaler Nukleinsäuren in einer Probe von peripherem Blut bestimmt wird, die von einer schwangeren Frau mit Zwillingen erhalten wird als ein erster zellfreier fetaler DNA-Anteil, und wobei das Verfahren ferner folgendes umfasst:

(a) Bestimmen eines Anteils $x_i$ der Anzahl an Sequenzierungsdaten, die von dem Chromosom i in dem Sequenzierungsergebnis abgeleitet werden zu den Sequenzierungsdaten insgesamt, wobei i eine laufende Nummer des Chromosoms ist, und wobei i eine beliebige ganze Zahl im Bereich von 1 bis 22 ist;
(b) Bestimmen eines T-Scores des Chromosoms i gemäß $T_i = (x_i - \mu_i)/(\sigma_1$, wobei i die laufende Nummer des Chromosoms ist, und wobei i eine beliebige ganze Zahl im Bereich von 1 bis 22 ist, wobei $\mu_i$ einen durchschnittlichen prozentualen Anteil der Sequenzierungsdaten des Chromosoms i darstellt, ausgewählt als ein Referenzsystem in einer Referenzdatenbank, zu den Sequenzierungsdaten davon insgesamt, wobei $\sigma_1$ eine Standardabweichung der prozentualen Anteile der Sequenzierungsdaten des Chromosoms i darstellt, ausgewählt als das Referenzsystem in der Referenzdatenbank, zu den Sequenzierungsdaten davon insgesamt;

(c) Bestimmen eines L-Scores des Chromosoms i gemäß $L_i = log(d(T_i, a)) / log(d(T2_i, a))$, wobei i die laufende Nummer des Chromosoms ist, und wobei i eine beliebige ganze Zahl im Bereich von 1 bis 22 ist, mit $T2_i = (x_1 - \mu_1*(1+fra/2))/\sigma_1$; wobei $d(T_i, a)$ und $d(T2_i, a)$ die Verteilungswahrscheinlichkeitsdichtefunktion t darstellen, wobei a einen Freiheitsgrad darstellt, wobei fra den ersten zellfreien fetalen DNA-Anteil darstellt;

(d) Darstellen eines Diagramms mit vier Quadranten mit T als vertikale Koordinate und mit L als horizontale Koordinate durch Zonenbildung mit einer ersten geraden Linie, wobei T = der vorbestimmte fünfte Schwellenwert ist, und mit einer zweiten geraden Linie, wobei L = der vorbestimmte sechste Schwellenwert ist,

wobei

für beide Föten der Zwillinge bestimmt wird, dass sie ein Trisom aufweisen, wenn für eine untersuchte Probe bestimmt wird, dass sie einen T-Score und einen L-Score aufweist, die in einen ersten Quadranten fallen;

für einen Fötus der Zwillinge bestimmt wird, dass er ein Trisom aufweist, und wobei für den anderen Fötus der Zwillinge bestimmt wird, dass er normal ist, wenn für eine untersuchte Probe bestimmt wird, dass sie einen T-Score und einen L-Score aufweist, die in einen zweiten Quadranten fallen;

für beide Föten der Zwillinge bestimmt wird, dass sie normal sind, wenn für eine untersuchte Probe bestimmt wird, dass sie einen T-Score und einen L-Score aufweist, die in einen dritten Quadranten fallen, und

für die Zwillinge bestimmt wird, dass sie einen niedrigen fetalen Anteil bewirkt haben, wenn für eine untersuchte Probe bestimmt wird, dass sie einen T-Score und einen L-Score aufweist, die in einen vierten Quadranten fallen, so dass das Ergebnis nicht übernommen wird.

20. Verfahren nach Anspruch 1, wobei der Anteil zellfreier fetaler Nukleinsäuren in einer Probe von peripherem Blut bestimmt wird, die von einer schwangeren Frau mit Zwillingen erhalten wird als ein erster zellfreier fetaler DNA-Anteil, und wobei das Verfahren ferner folgendes umfasst:

(a) Bestimmen eines dritten zellfreien fetalen DNA-Anteils auf der Basis von Sequenzierungsdaten, die von einem vorbestimmten Chromosom in dem Sequenzierungsergebnis abgeleitet werden; und

(b) Bestimmen, ob der untersuchte Fötus fetale Chimäre in Bezug auf das vorbestimmte Chromosom aufweist, auf der Basis des ersten zellfreien fetalen DNA-Anteils und des dritten zellfreien fetalen DNA-Anteils.

21. Verfahren nach Anspruch 20, wobei der dritte zellfreie fetale DNA-Anteil durch die folgende Formel bestimmt wird: *fra.chri = 2\*(chri.ER%/adjust.chri.ER% - 1)\*100%*, wobei *fra.chri* den genannten dritten zellfreien fetalen DNA-Anteil darstellt, wobei i eine laufende Nummer des vorbestimmten Chromosoms darstellt, und wobei i eine beliebige ganze Zahl im Bereich von 1 bis 22 ist; wobei *chri.ER%* einen prozentualen Anteil der von dem vorbestimmten Chromosom abgeleiteten Sequenzierungsdaten in dem Sequenzierungsergebnis zu den Sequenzierungsdaten insgesamt darstellt; wobei *adjust.chri.ER%* einen durchschnittlichen prozentualen Anteil der Sequenzierungsdaten zellfreier Nukleinsäuren darstellt, die von dem vorbestimmten Chromosom in einer Probe von peripherem Blut, die von einer schwangeren Frau erhalten worden ist, für die ein normaler Fötus vorbestimmt worden ist, zu den Sequenzierungsdaten davon insgesamt.

22. Verfahren nach Anspruch 20 oder Anspruch 21, wobei das Bestimmen, ob der untersuchte Fötus fetale Chimäre in Bezug auf das vorbestimmte Chromosom aufweist, auf der Basis des ersten zellfreien fetalen DNA-Anteils und des dritten zellfreien fetalen DNA-Anteils, ferner folgendes umfasst:

(a) Bestimmen eines Verhältnisses des dritten zellfreien fetalen DNA-Anteils zu dem ersten zellfreien DNA-Anteil; und

(b) Bestimmen, ob der untersuchte Fötus Chimäre in Bezug auf das vorbestimmte Chromosom aufweist durch Vergleichen des in (a) bestimmten Verhältnisses mit einer Mehrzahl vorbestimmter Schwellenwerte.

23. Verfahren nach Anspruch 22, wobei die Mehrzahl vorbestimmter Schwellenwerte mindestens einen Schwellenwert aufweist, der ausgewählt ist aus:

einem siebten Schwellenwert, der bestimmt wird auf der Basis einer Mehrzahl von Stichproben, wobei für das vorbestimmte Chromosom bekannt ist, dass es ein vollständiges Chromosom ist,

einem achten Schwellenwert, der bestimmt wird auf der Basis einer Mehrzahl von Stichproben, wobei für das vorbestimmte Chromosom bekannt ist, dass es eine Monosom-Chimäre ist,

einem neunten Schwellenwert, der bestimmt wird auf der Basis einer Mehrzahl von Stichproben, wobei für das vorbestimmte Chromosom bekannt ist, dass es normal ist,

einem zehnten Schwellenwert, der bestimmt wird auf der Basis einer Mehrzahl von Stichproben, wobei für das vorbestimmte Chromosom bekannt ist, dass es ein vollständiges Trisom ist, und wobei

das vorbestimmte Chromosom des untersuchten Fötus als ein vollständiges Monosom bestimmt wird, wenn das Verhältnis des dritten zellfreien fetalen DNA-Anteils zu dem ersten zellfreien fetalen DNA-Anteil kleiner ist als der siebte Schwellenwert;

das vorbestimmte Chromosom des untersuchten Fötus als eine Monosom-Chimäre bestimmt wird, wenn das Verhältnis des dritten zellfreien fetalen DNA-Anteils zu dem ersten zellfreien fetalen DNA-Anteil kleiner ist als der achte Schwellenwert;

das vorbestimmte Chromosom des untersuchten Fötus als normal bestimmt wird, wenn das Verhältnis des dritten zellfreien fetalen DNA-Anteils zu dem ersten zellfreien fetalen DNA-Anteil größer ist als der achte Schwellenwert und kleiner als der neunte Schwellenwert;

das vorbestimmte Chromosom des untersuchten Fötus als eine Trisom-Chimäre bestimmt wird, wenn das Verhältnis des dritten zellfreien fetalen DNA-Anteils zu dem ersten zellfreien fetalen DNA-Anteil nicht kleiner ist als der neunte Schwellenwert und nicht größer ist als der zehnte Schwellenwert; und

das vorbestimmte Chromosom des untersuchten Fötus als ein vollständiges Trisom bestimmt wird, wenn das Verhältnis des dritten zellfreien fetalen DNA-Anteils zu dem ersten zellfreien fetalen DNA-Anteil größer ist als der zehnte Schwellenwert.

**24.** Verfahren nach Anspruch 23, wobei

der siebte Schwellenwert größer ist als -1 und kleiner als 0, zum Beispiel -0,85;
der achte Schwellenwert größer ist als der siebte Schwellenwert und kleiner als 0, zum Beispiel -0,3;
der neunte Schwellenwert größer ist als 0 und kleiner ist als 1, zum Beispiel 0,3; und
der zehnte Schwellenwert größer ist als der neunte Schwellenwert und kleiner ist als 1, zum Beispiel 0,85.

**25.** Verfahren nach Anspruch 1, wobei der Anteil zellfreier fetaler Nukleinsäuren in einer Probe von peripherem Blut bestimmt wird, die von einer schwangeren Frau mit einem Fötus erhalten wird als ein erster zellfreier fetaler DNA-Anteil, und wobei das Verfahren ferner folgendes umfasst:

(a) Bestimmen eines Anteils $x_i$ der Anzahl an Sequenzierungsdaten, die von dem Chromosom i in dem Sequenzierungsergebnis abgeleitet werden zu den Sequenzierungsdaten insgesamt, wobei i eine laufende Nummer des Chromosoms ist, und wobei i eine beliebige ganze Zahl im Bereich von 1 bis 22 ist;

(b) Bestimmen eines T-Scores des Chromosoms i gemäß $T_i = (x_i - \mu_i)/\sigma_1$, wobei i die laufende Nummer des Chromosoms ist, und wobei i eine beliebige ganze Zahl im Bereich von 1 bis 22 ist, wobei $\mu_i$ einen durchschnittlichen prozentualen Anteil der Sequenzierungsdaten des Chromosoms i darstellt, ausgewählt als ein Referenzsystem in einer Referenzdatenbank, zu den Sequenzierungsdaten davon insgesamt, wobei $\sigma_1$ eine Standardabweichung der prozentualen Anteile der Sequenzierungsdaten des Chromosoms i darstellt, ausgewählt als das Referenzsystem in der Referenzdatenbank, zu den Sequenzierungsdaten davon insgesamt;

(c) Bestimmen eines L-Scores des Chromosoms i gemäß $L_i = log(d(T_i, a)) / log(d(T2_i, a))$, wobei i die laufende Nummer des Chromosoms ist, und wobei i eine beliebige ganze Zahl im Bereich von 1 bis 22 ist, mit $T2_i = (x_1 - \mu_1*(1+fra/2))/\sigma_1$; wobei $d(T_i, a)$ und $d(T2_i, a)$ die Verteilungswahrscheinlichkeitsdichtefunktion t darstellen, wobei a einen Freiheitsgrad darstellt, wobei *fra* den ersten zellfreien fetalen DNA-Anteil darstellt;

(d) Darstellen eines Diagramms mit vier Quadranten mit T als vertikale Koordinate und mit L als horizontale Koordinate durch Zonenbildung mit einer dritten geraden Linie, wobei T = der vorbestimmte elfte Schwellenwert ist, und mit einer vierten geraden Linie, wobei L = der vorbestimmte zwölfte Schwellenwert ist, wenn der T-Score nicht größer ist als 0, wobei

für den Fötus bestimmt wird, dass er ein vollständiges Monosom oder eine Monosom-Chimäre in Bezug auf das vorbestimmte Chromosom aufweist, wenn für eine untersuchte Probe bestimmt wird, dass sie einen T-Score und einen L-Score aufweist, die in einen ersten Quadranten fallen;

für den Fötus bestimmt wird, dass er eine Monosom-Chimäre in Bezug auf das vorbestimmte Chromosom aufweist, wenn für eine untersuchte Probe bestimmt wird, dass sie einen T-Score und einen L-Score aufweist, die in einen zweiten Quadranten fallen;

für den Fötus bestimmt wird, dass er normal ist, wenn für eine untersuchte Probe bestimmt wird, dass sie einen T-Score und einen L-Score aufweist, die in einen dritten Quadranten fallen, und

für den Fötus bestimmt wird, dass er einen niedrigen fetalen Anteil bewirkt hat, wenn für eine untersuchte Probe bestimmt wird, dass sie einen T-Score und einen L-Score aufweist, die in einen vierten Quadranten fallen, so dass das Ergebnis nicht übernommen wird;

(e) Darstellen eines Diagramms mit vier Quadranten mit T als vertikale Koordinate und mit L als horizontale Koordinate durch Zonenbildung mit einer ersten geraden Linie, wobei T = der vorbestimmte dreizehnte Schwellenwert ist, und mit einer zweiten geraden Linie, wobei L = der vorbestimmte vierzehnte Schwellenwert ist, wenn der T-Score größer ist als 0,
wobei

für den Fötus bestimmt wird, dass er ein vollständiges Trisom oder eine Trisom-Chimäre in Bezug auf das vorbestimmte Chromosom aufweist, wenn für eine untersuchte Probe bestimmt wird, dass sie einen T-Score und einen L-Score aufweist, die in einen ersten Quadranten fallen;
für den Fötus bestimmt wird, dass er eine Trisom-Chimäre in Bezug auf das vorbestimmte Chromosom aufweist, wenn für eine untersuchte Probe bestimmt wird, dass sie einen T-Score und einen L-Score aufweist, die in einen zweiten Quadranten fallen;
für den Fötus bestimmt wird, dass er normal ist, wenn für eine untersuchte Probe bestimmt wird, dass sie einen T-Score und einen L-Score aufweist, die in einen dritten Quadranten fallen; und
für den Fötus bestimmt wird, dass er einen niedrigen fetalen Anteil bewirkt hat, wenn für eine untersuchte Probe bestimmt wird, dass sie einen T-Score und einen L-Score aufweist, die in einen vierten Quadranten fallen, so dass das Ergebnis nicht übernommen wird.

26. Verfahren nach Anspruch 25, wobei der elfte Schwellenwert und der dreizehnte Schwellenwert jeweils unabhängig 3 sind, und wobei der zwölfte Schwellenwert und der vierzehnte Schwellenwert jeweils unabhängig 1 sind.

## Revendications

1. Procédé pour déterminer la fraction d'acides nucléiques foetaux exempts de cellules dans un échantillon de sang périphérique issu d'une femme enceinte, comprenant les étapes consistant à :

(i) effectuer un séquençage sur des acides nucléiques exempts de cellules contenus dans l'échantillon de manière à obtenir un résultat de séquençage consistant en une pluralité de données de séquençage ;
(ii) déterminer le nombre des acides nucléiques exempts de cellules d'une longueur située dans une plage prédéterminée dans l'échantillon sur la base du résultat de séquençage ; et
(iii) déterminer la fraction d'acides nucléiques fœtaux exempts de cellules dans l'échantillon sur la base du nombre d'acides nucléiques exempts de cellules d'une longueur située dans la plage prédéterminée, ladite plage prédéterminée étant déterminée avant l'étape (i) par les étapes suivantes consistant à :

(a) déterminer des longueurs des acides nucléiques exempts de cellules dans une pluralité d'échantillons de sang périphérique de contrôle issus de femmes enceintes dans chacun desquels la fraction d'acides nucléiques fœtaux exempts de cellules est connue ;
(b) fixer une pluralité de plages de longueur candidates et déterminer le pourcentage d'acides nucléiques exempts de cellules obtenus à partir de chacun de la pluralité d'échantillons de contrôle présents dans chaque plage de longueur candidate ;
(c) déterminer un coefficient de corrélation pour chaque plage de longueur candidate sur la base du pourcentage d'acides nucléiques exempts de cellules obtenus à partir de chacun de la pluralité d'échantillons de contrôle présents dans chaque plage de longueur candidate et de la fraction connue d'acides nucléiques fœtaux exempts de cellules dans chacun des échantillons de contrôle ; et
(d) déterminer au moins une plage de longueur candidate ou une combinaison des plages de longueur candidates en tant que plage prédéterminée sur la base du coefficient de corrélation maximum.

2. Procédé selon la revendication 1, l'étape consistant à déterminer le nombre des acides nucléiques exempts de cellules d'une longueur située dans la plage prédéterminée dans l'échantillon sur la base du résultat de séquençage comprenant en outre les étapes consistant à :

(a) aligner le résultat de séquençage à un génome de référence, de manière à construire un ensemble de données consistant en une pluralité de lectures mappées de manière unique, chaque lecture dans l'ensemble de données pouvant être mappée à une position individuelle du génome de référence ;

(b) déterminer la longueur de l'acide nucléique exempt de cellules correspondant à chaque lecture mappée de manière unique dans l'ensemble de données ; et

(c) déterminer le nombre des acides nucléiques exempts de cellules d'une longueur située dans la plage prédéterminée.

3. Procédé selon la revendication 2, l'étape consistant à déterminer la longueur des acides nucléiques exempts de cellules correspondant à chaque lecture mappée de manière unique dans l'ensemble de données comprenant en outre les étapes consistant à :

déterminer la longueur de chaque lecture mappée de manière unique au génome de référence en tant que longueur de l'acide nucléique exempt de cellules correspondant à la lecture.

4. Procédé selon la revendication 2, les acides nucléiques exempts de cellules dans l'échantillon étant séquencés par séquençage à extrémité appariée et l'étape consistant à déterminer la longueur de l'acide nucléique exempt de cellules correspondant à chaque lecture mappée de manière unique dans l'ensemble de données comprenant en outre les étapes consistant à :

déterminer la position correspondant au génome de référence de l'extrémité 5' de l'acide nucléique exempt de cellules sur la base des données de séquençage pour l'extrémité 5' de chaque lecture mappée de manière unique obtenue dans le séquençage à extrémité appariée ;

déterminer la position correspondant au génome de référence de l'extrémité 3' de l'acide nucléique exempt de cellules sur la base des données de séquençage à l'autre extrémité de la même lecture mappée de manière unique obtenue dans le séquençage à extrémité appariée ; et

déterminer la longueur de l'acide nucléique exempt de cellules sur la base des positions de l'extrémité 5' et de l'extrémité 3' de l'acide nucléique exempt de cellules.

5. Procédé selon la revendication 1, l'étape consistant à déterminer la fraction d'acides nucléiques fœtaux exempts de cellules dans l'échantillon sur la base du nombre d'acides nucléiques exempts de cellules d'une longueur située dans la plage prédéterminée comprenant en outre les étapes consistant à :

déterminer le pourcentage d'acides nucléiques exempts de cellules présents dans la plage prédéterminée sur la base du nombre d'acides nucléiques exempts de cellules d'une longueur située dans la plage prédéterminée ; et

déterminer la fraction d'acides nucléiques fœtaux exempts de cellules dans l'échantillon, sur la base du pourcentage des acides nucléiques exempts de cellules présents dans la plage prédéterminée selon une fonction prédéterminée,

la fonction prédéterminée étant déterminée sur la base de la pluralité d'échantillons de contrôle.

6. Procédé selon la revendication 5, la fraction prédéterminée étant obtenue par les étapes suivantes consistant à :

(i) déterminer le pourcentage d'acides nucléiques exempts de cellules obtenus à partir de chaque échantillon de contrôle présent dans la plage prédéterminée ; et

(ii) ajuster le pourcentage d'acides nucléiques exempts de cellules obtenu pour chaque échantillon de contrôle présent dans la plage prédéterminée avec la fraction connue d'acides nucléiques fœtaux exempts de cellules afin de déterminer ladite fonction ; le pourcentage d'acides nucléiques exempts de cellules obtenu à partir de chaque échantillon de contrôle présent dans la plage prédéterminée étant éventuellement ajusté avec la fraction connue d'acides nucléiques fœtaux exempts de cellules par un ajustement linéaire.

7. Procédé selon la revendication 1, la plage prédéterminée étant de 185 bp à 204 bp.

8. Procédé selon la revendication 5, la fonction prédéterminée étant $d = 0,0334*p + 1,6657$, où $d$ représente une fraction d'acides nucléiques fœtaux exempts de cellules, et $p$ représente le pourcentage d'acide nucléique exempt de cellules présent dans la plage prédéterminée.

9. Procédé selon la revendication 8, les échantillons de contrôle étant des échantillons de sang périphérique obtenus à partir de femmes enceintes portant un fœtus mâle normal, dans lesquels la fraction d'acides nucléiques exempts de cellules qui sont des acides nucléiques fœtaux exempts de cellules est connue comme étant déterminée par le chromosome Y.

10. Procédé selon la revendication 1, ladite fraction d'acides nucléiques fœtaux exempts de cellules étant déterminée dans un échantillon de sang périphérique obtenu à partir d'une femme enceinte portant des jumeaux en tant que première fraction d'ADN fœtal exempt de cellules et qui comprend en outre les étapes consistant à :

> (a) déterminer une deuxième fraction d'ADN fœtal exempt de cellules sur la base de données de séquençage dérivées du chromosome Y dans le résultat de séquençage ; et
> (b) déterminer le sexe des jumeaux sur la base de la première fraction d'ADN fœtal exempt de cellules et de ladite deuxième fraction d'ADN fœtal exempt de cellules.

11. Procédé selon la revendication 10, ladite deuxième fraction d'ADN fœtal exempt de cellules étant déterminée selon la formule suivante :

$$fra.chry = (chry.ER\% - Female.chry.ER\%) / (Man.chry.ER\% - Female.chry.ER\%) * 100\%,$$

> où *fra.chry* représente ladite deuxième fraction d'ADN fœtal exempt de cellules, *chry.ER%* représente un pourcentage des données de séquençage dérivées du chromosome Y dans le résultat de séquençage par rapport aux données de séquençage totales ;
> *Female.chry.ER%* représente un pourcentage moyen de données de séquençage d'acides nucléiques exempts de cellules s'alignant avec le chromosome Y dans un échantillon de sang périphérique obtenu à partir d'une femme enceinte prédéterminée comme portant un fœtus femelle normal par rapport aux données de séquençage totales de celui-ci ; et
> *Man.chry.ER%* représente un pourcentage moyen de données de séquençage d'acides nucléiques exempts de cellules dérivés du chromosome Y dans un échantillon de sang périphérique obtenu à partir d'un homme en bonne santé par rapport aux données de séquençage totales de celui-ci.

12. Procédé selon la revendication 10 ou la revendication 11, l'étape consistant à déterminer le sexe des jumeaux sur la base de la première fraction d'ADN fœtal exempt de cellules et de ladite deuxième fraction d'ADN fœtal exempt de cellules comprenant en outre les étapes consistant à :

> (a) déterminer un rapport de ladite deuxième fraction d'ADN fœtal exempt de cellules à la première fraction d'ADN fœtal exempt de cellules ; et
> (b) déterminer le sexe des jumeaux en comparant le rapport déterminé en (a) à un premier seuil prédéterminé et un deuxième seuil prédéterminé,
>
>> le premier seuil ayant été déterminé sur la base d'une pluralité d'échantillons de contrôle obtenus à partir de femmes enceintes connues comme portant des jumeaux femelles et le deuxième seuil ayant été déterminé sur la base d'une pluralité d'échantillons de contrôle obtenus à partir de femmes enceintes connues comme portant des jumeaux mâles,
>> et
>> les deux fœtus des jumeaux étant déterminés comme femelles si le rapport de ladite deuxième fraction d'ADN fœtal exempt de cellules à la première fraction d'ADN fœtal exempt de cellules est inférieur au premier seuil,
>> les deux fœtus des jumeaux étant déterminés comme mâles si le rapport de ladite deuxième fraction d'ADN fœtal exempt de cellules à la première fraction d'ADN fœtal exempt de cellules est supérieur au deuxième seuil, et
>> les jumeaux étant déterminés comme incluant un fœtus mâle et un fœtus femelle si le rapport de ladite deuxième fraction d'ADN fœtal exempt de cellules à la première fraction d'ADN fœtal exempt de cellules est égal au premier seuil ou au deuxième seuil, ou compris entre le premier seuil et le deuxième seuil.

13. Procédé selon la revendication 12, le premier seuil étant de 0,35 et le deuxième seuil étant de 0,7.

14. Procédé selon la revendication 1, ladite fraction d'acides nucléiques fœtaux exempts de cellules étant déterminée dans un échantillon de sang périphérique obtenu à partir d'une femme enceinte portant des jumeaux en tant que première fraction d'ADN fœtal exempt de cellules et qui comprend en outre les étapes consistant à :

(a) déterminer une troisième fraction d'ADN fœtal exempt de cellules, sur la base de données de séquençage dérivées d'un chromosome prédéterminé dans le résultat de séquençage ; et

(b) déterminer si les jumeaux sous détection ont une aneuploïdie au regard du chromosome prédéterminé sur la base de la première fraction d'ADN fœtal exempt de cellules et de ladite troisième fraction d'ADN fœtal exempt de cellules.

15. Procédé selon la revendication 14, ladite troisième fraction d'ADN fœtal exempt de cellules étant déterminée selon la formule suivante :

*fra.chri = 2 \* (chri.ER% / adjust.chri.ER% - 1) \* 100* %, où *fra.chri* représente ladite troisième fraction d'ADN fœtal exempt de cellules, i représente un numéro de série du chromosome prédéterminé, et i est un entier quelconque dans la plage allant de 1 à 22 ; *chri.ER%* représente un pourcentage des données de séquençage dérivées du chromosome prédéterminé dans le résultat de séquençage par rapport aux données de séquençage totales ; *adjust.chri.ER%* représente un pourcentage moyen de données de séquençage d'acides nucléiques exempts de cellules dérivées du chromosome prédéterminé dans un échantillon de sang périphérique obtenu à partir d'une femme enceinte prédéterminée comme portant des jumeaux normaux par rapport aux données de séquençage totales de celui-ci.

16. Procédé selon la revendication 14 ou la revendication 15, l'étape consistant à déterminer si les jumeaux sous détection ont une aneuploïdie au regard du chromosome prédéterminé sur la base de la première fraction d'ADN fœtal exempt de cellules et de ladite troisième fraction d'ADN fœtal exempt de cellules comprenant en outre les étapes consistant à :

(a) déterminer un rapport de ladite troisième fraction d'ADN fœtal exempt de cellules à la première fraction d'ADN fœtal exempt de cellules ; et

(b) déterminer si les jumeaux sous détection ont une aneuploïdie au regard du chromosome prédéterminé en comparant le rapport déterminé en (a) à un troisième seuil prédéterminé et un quatrième seuil prédéterminé,

et le troisième seuil ayant été déterminé sur la base d'une pluralité d'échantillons de contrôle obtenus à partir de femmes enceintes portant des jumeaux connus comme n'ayant pas d'aneuploïdie au regard du chromosome prédéterminé et le quatrième seuil ayant été déterminé sur la base d'une pluralité d'échantillons de contrôle obtenus à partir de femmes enceintes portant des jumeaux connus comme ayant une aneuploïdie au regard du chromosome prédéterminé,

et

les deux fœtus des jumeaux étant déterminés comme n'ayant aucune aneuploïdie au regard du chromosome prédéterminé si le rapport de ladite troisième fraction d'ADN fœtal exempt de cellules à la première fraction d'ADN fœtal exempt de cellules est inférieur au troisième seuil,

les deux fœtus des jumeaux étant déterminés comme ayant une aneuploïdie au regard du chromosome prédéterminé si le rapport de ladite troisième fraction d'ADN foetal exempt de cellules à la première fraction d'ADN fœtal exempt de cellules est supérieur au quatrième seuil, et

un fœtus des jumeaux étant déterminé comme ayant une aneuploïdie au regard du chromosome prédéterminé, tandis que l'autre fœtus des jumeaux est déterminé comme n'ayant aucune aneuploïdie au regard du chromosome prédéterminé si le rapport de ladite troisième fraction d'ADN fœtal exempt de cellules à la première fraction d'ADN fœtal exempt de cellules est égal au troisième seuil ou au quatrième seuil, ou compris entre le troisième seuil et le quatrième seuil.

17. Procédé selon la revendication 1, le troisième seuil étant de 0,35 et le quatrième seuil étant de 0,7.

18. Procédé selon la revendication 14, le chromosome prédéterminé étant au moins un sélectionné parmi les chromosomes 18, 21 et 23.

19. Procédé selon la revendication 1, ladite fraction d'acides nucléiques fœtaux exempts de cellules étant déterminée dans un échantillon de sang périphérique obtenu à partir d'une femme enceinte portant des jumeaux en tant que première fraction d'ADN fœtal exempt de cellules et qui comprend en outre les étapes consistant à :

(a) déterminer une fraction $x_i$ du nombre de données de séquençage dérivées du chromosome i dans le résultat de séquençage par rapport aux données de séquençage totales, où i représente un numéro de série du chromosome, et i est un entier quelconque dans la plage allant de 1 à 22 ;

(b) déterminer un score T du chromosome i selon $T_i = (x_i - \mu_i) / \sigma_i$, où i représente le numéro de série du chromosome et i est un entier quelconque dans la plage allant de 1 à 22, $\mu_i$ représente un pourcentage moyen de données de séquençage du chromosome i sélectionné en tant que système de référence dans une base de données de référence par rapport aux données de séquençage totales de celui-ci, $\sigma_i$ représente un écart type de pourcentages des données de séquençage du chromosome i sélectionné en tant que système de référence dans la base de données de référence par rapport aux données de séquençage totales de celui-ci,
(c) déterminer un score L du chromosome i selon $L_i = log(d(T_i, a)) / log(d(T2_i, a))$, où i représente le numéro de série du chromosome et i est un entier quelconque dans la plage allant de 1 à 22, $T2_i = (x_i - \mu_i * (1 + fra / 2)) / \sigma_i$ ; $d(T_i, a)$ et $d(T2_i, a)$ représentent une fonction de densité de probabilité de distribution t, où a représente le degré de liberté, fra représente ladite première fraction d'ADN fœtal exempt de cellules,
(d) représenter un diagramme à quatre quadrants avec T en tant que coordonnée verticale et L en tant que coordonnée horizontale en zonant avec une première ligne droite où T = cinquième seuil prédéterminé et une deuxième ligne droite où L = sixième seuil prédéterminé,

les deux fœtus des jumeaux étant déterminés comme ayant un trisome si un échantillon sous détection est déterminé comme étant d'un score T et d'un score L situés dans un premier quadrant ;
un fœtus des jumeaux étant déterminé comme ayant un trisome et l'autre fœtus des jumeaux étant déterminé comme étant normal si un échantillon sous détection est déterminé comme étant d'un score T et d'un score L situés dans un deuxième quadrant ;
les deux fœtus des jumeaux étant déterminés comme étant normaux si un échantillon sous détection est déterminé comme étant d'un score T et d'un score L situés dans un troisième quadrant, et
les jumeaux étant déterminés comme ayant donné une faible fraction fœtale si un échantillon sous détection est déterminé comme étant d'un score T et d'un score L situés dans un quatrième quadrant de telle sorte que le résultat n'est pas adopté.

**20.** Procédé selon la revendication 1, ladite fraction d'acides nucléiques fœtaux exempts de cellules étant déterminée dans un échantillon de sang périphérique obtenu à partir d'une femme enceinte portant un fœtus en tant que première fraction d'ADN fœtal exempt de cellules et qui comprend en outre les étapes consistant à :

(a) déterminer une troisième fraction d'ADN fœtal exempt de cellules sur la base de données de séquençage dérivées d'un chromosome prédéterminé dans le résultat de séquençage ; et
(b) déterminer si le fœtus sous détection a une chimère fœtale au regard du chromosome prédéterminé sur la base de la première fraction d'ADN fœtal exempt de cellules et de ladite troisième fraction d'ADN fœtal exempt de cellules.

**21.** Procédé selon la revendication 20, ladite troisième fraction d'ADN fœtal exempt de cellules étant déterminée par la formule suivante :
*fra.chri = 2 * (chri.ER% / adjust.chri.ER% - 1)\*100* %, où *fra.chri* représente ladite troisième fraction d'ADN fœtal exempt de cellules, i représente un numéro de série du chromosome prédéterminé et i est un entier quelconque dans la plage allant de 1 à 22 ; *chri.ER%* représente un pourcentage des données de séquençage dérivées du chromosome prédéterminé dans le résultat de séquençage par rapport aux données de séquençage totales ; *adjust.chri.ER%* représente un pourcentage moyen de données de séquençage d'acides nucléiques exempts de cellules dérivés du chromosome prédéterminé dans un échantillon de sang périphérique obtenu à partir d'une femme enceinte prédéterminée comme portant un fœtus normal par rapport aux données de séquençage totales de celui-ci.

**22.** Procédé selon la revendication 20 ou la revendication 21, l'étape consistant à déterminer si le fœtus sous détection a une chimère fœtale au regard du chromosome prédéterminé sur la base de la première fraction d'ADN fœtal exempt de cellules et de ladite troisième fraction d'ADN fœtal exempt de cellules comprenant en outre les étapes consistant à :

(a) déterminer un rapport de ladite troisième fraction d'ADN fœtal exempt de cellules à la première fraction d'ADN fœtal exempt de cellules ; et
(b) déterminer si le fœtus sous détection a une chimère au regard du chromosome prédéterminé en comparant le rapport déterminé en (a) à une pluralité de seuils prédéterminés.

**23.** Procédé selon la revendication 22, la pluralité de seuils prédéterminés comprenant au moins un sélectionné parmi :

un septième seuil déterminé sur la base d'une pluralité d'échantillons de contrôle avec le chromosome prédéterminé connu comme étant un monosome complet,

un huitième seuil déterminé sur la base d'une pluralité d'échantillons de contrôle avec le chromosome prédéterminé connu comme étant une chimère de monosome,

un neuvième seuil déterminé sur la base d'une pluralité d'échantillons de contrôle avec le chromosome prédéterminé connu comme étant normal,

un dixième seuil déterminé sur la base d'une pluralité d'échantillons de contrôle avec le chromosome prédéterminé connu comme étant un trisome complet,

et

le chromosome prédéterminé du fœtus sous détection étant déterminé comme étant un monosome complet si le rapport de ladite troisième fraction d'ADN fœtal exempt de cellules à la première fraction d'ADN fœtal exempt de cellules est inférieur au septième seuil ;

le chromosome prédéterminé du fœtus sous détection étant déterminé comme étant une chimère de monosome si le rapport de ladite troisième fraction d'ADN fœtal exempt de cellules à la première fraction d'ADN fœtal exempt de cellules n'est pas inférieur au septième seuil et pas supérieur au huitième seuil ;

le chromosome prédéterminé du fœtus sous détection étant déterminé comme étant normal si le rapport de ladite troisième fraction d'ADN fœtal exempt de cellules à la première fraction d'ADN fœtal exempt de cellules est supérieur au huitième seuil et inférieur au neuvième seuil ;

le chromosome prédéterminé du fœtus sous détection étant déterminé comme étant une chimère de trisome si le rapport de ladite troisième fraction d'ADN fœtal exempt de cellules à la première fraction d'ADN fœtal exempt de cellules n'est pas inférieur au neuvième seuil et pas supérieur au dixième seuil ; et

le chromosome prédéterminé du fœtus sous détection étant déterminé comme étant un trisome complet si le rapport de ladite troisième fraction d'ADN fœtal exempt de cellules à la première fraction d'ADN fœtal exempt de cellules est supérieur au dixième seuil.

24. Procédé selon la revendication 23,

le septième seuil étant supérieur à -1 et inférieur à 0, par exemple de -0,85 ;

le huitième seuil étant supérieur au septième seuil et inférieur à 0, par exemple de -0,3 ; le neuvième seuil étant supérieur à 0 et inférieur à 1, par exemple de 0,3 ; et

le dixième seuil étant supérieur au neuvième seuil et inférieur à 1, par exemple de 0,85.

25. Procédé selon la revendication 1, ladite fraction d'acides nucléiques fœtaux exempts de cellules étant déterminée dans un échantillon de sang périphérique obtenu à partir d'une femme enceinte portant un fœtus en tant que première fraction d'ADN fœtal exempt de cellules et qui comprend en outre les étapes consistant à :

(a) déterminer une fraction $x_i$ du nombre de données de séquençage dérivées du chromosome i dans le résultat de séquençage par rapport aux données de séquençage totales, où i représente un numéro de série du chromosome, et i est un entier quelconque dans la plage allant de 1 à 22 ;

(b) déterminer un score T du chromosome i selon $T_i = (x_i - \mu_i) / \sigma_i$, où i représente le numéro de série du chromosome et i est un entier quelconque dans la plage allant de 1 à 22, $\mu_i$ représente une valeur moyenne de pourcentages de données de séquençage du chromosome i sélectionné en tant que système de référence dans une base de données de référence par rapport aux données de séquençage totales de celui-ci, $\sigma_i$ représente un écart type de pourcentages des données de séquençage du chromosome i sélectionné en tant que système de référence dans la base de données de référence par rapport aux données de séquençage totales de celui-ci,

(c) déterminer un score L du chromosome i selon $L_i = log(d(T_i, a)) / log(d(T2_i, a))$, où i représente le numéro de série du chromosome et i est un entier quelconque dans la plage allant de 1 à 22, $T2_i = (x_i - \mu_i * (1 + fra / 2)) / \sigma_i$ ; $d(T_i, a)$ et $d(T2_i, a)$ représentent une fonction de densité de probabilité de distribution t, où a représente le degré de liberté, *fra* représente ladite première fraction d'ADN fœtal exempt de cellules,

(d) représenter un diagramme à quatre quadrants avec T en tant que coordonnée verticale et L en tant que coordonnée horizontale en zonant avec une troisième ligne droite où T = onzième seuil prédéterminé et une quatrième ligne droite où L = douzième seuil prédéterminé, lorsque le score T n'est pas supérieur à 0,

le fœtus étant déterminé comme ayant un monosome complet ou une chimère de monosome au regard du chromosome prédéterminé si un échantillon sous détection est déterminé comme étant d'un score T et d'un score L situés dans un premier quadrant ; le fœtus étant déterminé comme ayant une chimère de monosome au regard du chromosome prédéterminé si un échantillon sous détection est déterminé comme étant d'un score T et d'un score L situés dans un deuxième quadrant ;

le fœtus étant déterminé comme étant normal au regard du chromosome prédéterminé, si un échantillon sous détection est déterminé comme étant d'un score T et d'un score L situés dans un troisième quadrant, et le fœtus étant déterminé comme ayant donné une faible fraction fœtale si un échantillon sous détection est déterminé comme étant d'un score T et d'un score L situés dans un quatrième quadrant de telle sorte que le résultat n'est pas adopté,

(e) la représentation d'un diagramme à quatre quadrants avec T en tant que coordonnée verticale et L en tant que coordonnée horizontale en zonant avec une première ligne droite où T = treizième seuil prédéterminé et une deuxième ligne droite où L = quatorzième seuil prédéterminé, lorsque le score T est supérieur à 0,

le fœtus étant déterminé comme ayant un trisome complet ou une chimère de trisome au regard du chromosome prédéterminé si un échantillon sous détection est déterminé comme étant d'un score T et d'un score L situés dans un premier quadrant ;
le fœtus étant déterminé comme ayant une chimère de trisome au regard du chromosome prédéterminé si un échantillon sous détection est déterminé comme étant d'un score T et d'un score L situés dans un deuxième quadrant ;
le fœtus étant déterminé comme étant normal au regard du chromosome prédéterminé si un échantillon sous détection est déterminé comme étant d'un score T et d'un score L situés dans un troisième quadrant, et
le fœtus étant déterminé comme ayant donné une faible fraction fœtale si un échantillon sous détection est déterminé comme étant d'un score T et d'un score L situés dans un quatrième quadrant de telle sorte que le résultat n'est pas adopté,

26. Procédé selon la revendication 25, le onzième seuil et le treizième seuil étant chacun indépendamment de 3 et le douzième seuil et le quatorzième seuil étant chacun indépendamment de 1.

S100

Nucleic acid sequencing

S200

Determining the number of the cell-free nucleic acids in a length falling into a predetermined range

S300

Determining a fraction of cell-free nucleic acids

Fig. 1

S210

aligning the sequencing result to a reference genome

S220

determining a length of the cell-free nucleic acid

S230

determining the number of the cell-free nucleic acids in the length falling into the predetermined range

Fig. 2

S2210

determining a position, of 5'-end of the cell-free nucleic acid

S2220

determining a position, of 3'-end of the cell-free nucleic acid

S2230

determining a length of the cell-free nucleic acid

Fig. 3

Fig. 4

Fig. 5

Fig. 6

100

| Sequencing apparatus |

200

| Counting apparatus |

300

| Apparatus for determining a fraction of cell-free nucleic acids |

Fig. 7

210

| Aligning unit |

220

| First length determining unit |

230

| Number determining unit |

Fig. 8

5'-end position
determining module — 2210

3'-end position
determining module — 2220

Length calculating module — 2230

Fig. 9

Second length determining
unit — 410

First percentage
determining unit — 420

Correlation coefficient
determining unit — 430

Predetermined range
determining unit — 440

Fig. 10

310

Second percentage
determining unit

320

Unit for calculating a
fraction of cell-free nucleic
acids

Fig. 11

510

Third percentage
determining unit

520

Fitting unit

Fig. 12

## 185~204bp

R = −0.8754

Fetal fraction(by chrY)

Percentage(%)

Fig. 13

**S1**

## chr13

## chr18

chr21

S2

chr13

## chr18

## chr21

**S3**

chr13

chr18

chr21

S4

chr13

chr18

chr21

**S5**

## chr13

## chr18

## chr21

## S6

## chr13

## chr18

## chr21

**S7**

chr13

chr18

chr21

S8    chr13

## chr18

## chr21

**S9**

## chr13

## chr18

## chr21

## S10

## chr13

## chr18

## chr21

**S11**                    chr13

chr18

chr21

**EP 3 178 941 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013132305 A

- WO 2014068075 A

### Non-patent literature cited in the description

- **YU et al.** *Proc. Natl.Acad. Sci.,* 2014, vol. 111, 8583-8588

- **JIANG et al.** Noninvasive Fetal Trisomy (NIFTY) test: an advanced noninvasive prenatal diagnosis methodology for fetal autosomal and sex chromosomal aneuploidies. *BMC Med Genomics,* 01 December 2012, vol. 5, 57